# NOUVEAU FASCICULE DE BREVET EUROPEEN

(11) **EP 1 047 376 B2**
(45) Date de publication et mention de la décision concernant l'opposition: **18.03.2009**
(45) Mention de la délivrance du brevet: 23.05.2001
(21) Numéro de dépôt: 98962481.2
(22) Date de dépôt: 16.12.1998
(51) Int. Cl.: A61K 8/99, A61Q 5/10

(54) **COMPOSITION DE TEINTURE D'OXYDATION DES FIBRES KERATINIQUES CONTENANT UNE LACCASE ET PROCEDE DE TEINTURE METTANT EN OEUVRE CETTE COMPOSITION**
OXIDATIONSFÄRBEMITTEL FÜR KERATINFASERN, DAS EINE LACCASE ENTHÄLT, UND VERFAHREN ZUR FÄRBUNG MIT DIESEM MITTEL
KERATINOUS FIBRE DYEING COMPOSITION CONTAINING A LACCASE AND DYEING METHOD USING SAME

(30) Priorité: 13.01.1998 FR 9800248
(43) Date de publication de la demande: 02.11.2000
(73) Titulaire: L'ORÉAL, 75008 Paris (FR)
(72) Inventeur: LANG, Gérard, F-95390 Saint Prix (FR); COTTERET, Jean, F-78480 Verneuil/Seine (FR)
(74) Mandataire: Dossmann, Gérard
(86) Numéro de dépôt international: PCT/FR1998/002752
(87) Numéro de publication internationale: WO 1999/036034

(56) Documents cités:
- EP-A- 0 504 005
- WO-A-97/39727
- WO-A1-95/01772
- WO-A1-95/15144
- WO-A1-97/01999
- WO-A1-97/37633
- DE- - 2 155 359
- DE- - 4 440 955
- DK- - 971 513
- FR-A- 2 694 018
- US- - 3 251 742
- US-A- 5 637 115
- DATABASE WPI Week 9750 Derwent Publications Ltd., London, GB; AN 97-545467 XP002084903 "Composition for hair dyeing-contains laccaseand developing substance consisting or e.g. 3,4-diamino-benz-hydrazide" A & JP 09 263522 A (LION CORP) 7 octobre 1997

## Description

L'invention a pour objet une composition pour la teinture d'oxydation des fibres kératiniques. et en particulier des fibres kératiniques humaines telles que les cheveux comprenant, dans un milieu approprié pour la teinture, au moins un colorant d'oxydation, au moins un colorant direct cationique, et au moins une enzyme de type laccase, ainsi que le procédé de teinture mettant en oeuvre cette composition.

Il est connu de teindre les fibres kératiniques et en particulier les cheveux humains avec des compositions tinctoriales contenant des précurseurs de coloration d'oxydation, en particulier des ortho ou paraphénylènediamines, des ortho ou paraaminophénols. des bases hétérocycliques, appelés généralement bases d'oxydation. Les précurseurs de coloration d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

Il est également connu que pour faire encore varier les nuances obtenues et leur donner des reflets, on peut utiliser, en association avec les bases d'oxydation et les coupleurs, des colorants directs. c'est à dire des substances colorées qui apportent une coloration en l'absence d'agent oxydant.

Ces colorants directs appartiennent pour leur très grande majorité à la famille des composés nitrés de la série benzénique et ont l'inconvénient, lorsqu'ils sont incorporés dans des compositions tinctoriales, de conduire à des colorations présentant une ténacité insuffisante, en particulier vis-à-vis des shampooings.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

La coloration d'oxydation des fibres kératiniques est généralement réalisée en milieu alcalin, en présence de peroxyde d'hydrogène. Toutefois, l'utilisation des milieux alcalins en présence de peroxyde d'hydrogène présentent pour inconvénient d'entraîner une dégradation non négligeable des fibres, ainsi qu'une décoloration des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques. Ainsi il a déjà été proposé dans le brevet US 3251742, les demandes de brevet FR-A-2 112 549, FR-A-2 694 018, EP-A-0 504 005, WO95/07988, WO95/33836, WO95/33837, WO96/00290, WO97/19998 et WO97/19999 de teindre les fibres kératiniques avec des compositions comprenant au moins un colorant d'oxydation en association avec des enzymes du type laccase ; lesdites compositions étant mises en contact avec l'oxygène de l'air. Ces formulations de teinture, bien qu'étant mises en oeuvre dans des conditions n'entraînant pas une dégradation des fibres kératiniques comparable à celle engendrée par les teintures réalisées en présence de peroxyde d'hydrogène, conduisent à des colorations encore insuffisantes à la fois sur le plan de l'homogénéité de la couleur répartie le long de la fibre (« unisson »), sur le plan de la chromaticité (luminosité) et de la puissance tinctoriale.

WO 97/39727 divulgue une composition comprenant un colorant oxidative, un colorant directe cationique et un agent d'oxidation mais pas de laccase.

La présente invention a pour but de résoudre les problèmes évoqués ci-dessus.

La demanderesse vient maintenant de découvrir qu'il est possible d'obtenir de nouvelles teintures, capables de conduire à des colorations puissantes et chromatiques, sans engendrer de dégradation significative des fibres kératiniques, peu sélectives et résistant bien aux diverses agressions que peuvent subir les fibres, en associant au moins un colorant d'oxydation, au moins un colorant direct cationique tel que décrit ci-dessus et au moins une enzyme de type laccase, avec un pH compris entre 6 et 9.

Cette découverte est à la base de la présente invention.

L'invention a donc pour premier objet une composition prête à l'emploi, pour la teinture d'oxydation des fibres kératiniques et en particulier des fibres kératiniques humaines telles que les cheveux, caractérisée par le fait qu'elle comprend, dans un milieu approprié pour la teinture :
- au moins un colorant d'oxydation,
- au moins un colorant direct cationique choisi parmi:
   a) les composés de formule (V) suivante : 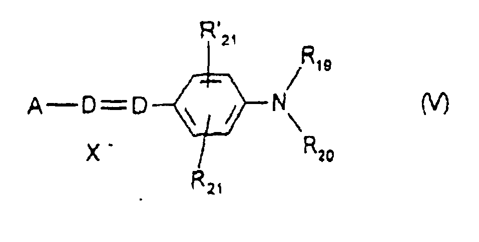 dans laquelle :
      D représente un atome d'azote ou le groupement -CH,
      R₁₉ et R₂₀, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
      R₂₁ et R'₂₁, identiques ou différents, représentent un atome d'hydrogène ou d'halogène choisi parmi le chlore, le brome, l'iode et le fluor, un radical cyano, alcoxy en C₁-C₄ ou acétyloxy,
      X représente un anion de préférence choisi parmi le chlorure, le méthyl sulfate et l'acétate,
      A représente un groupement choisi par les structures A1 à A19 suivantes : 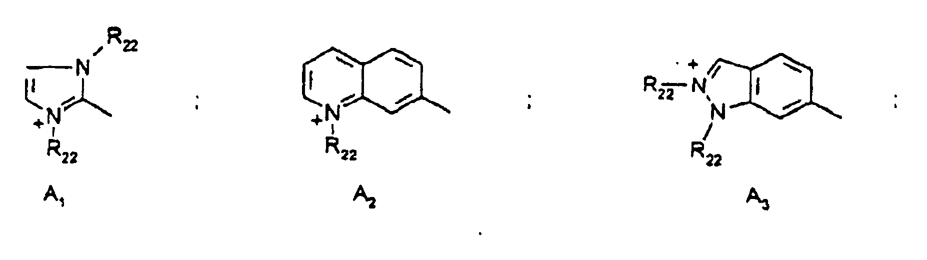 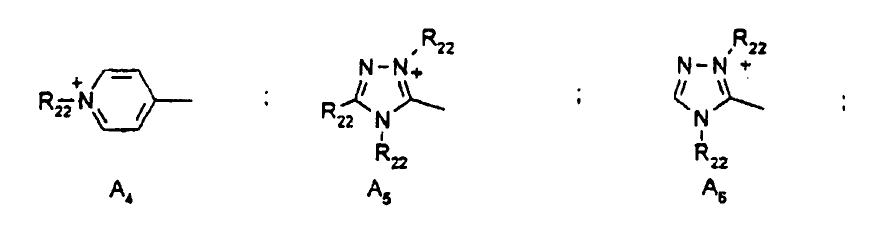 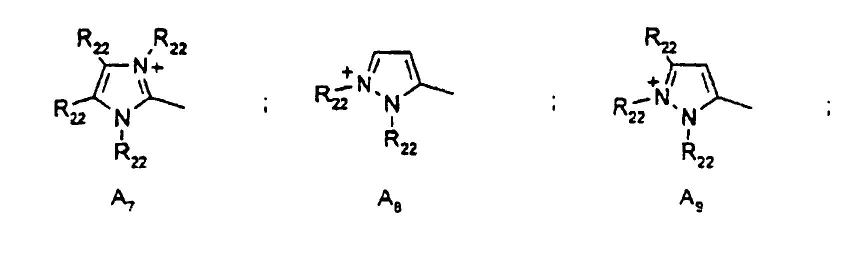 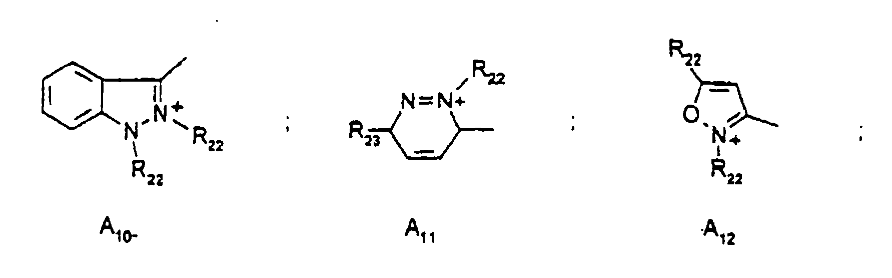 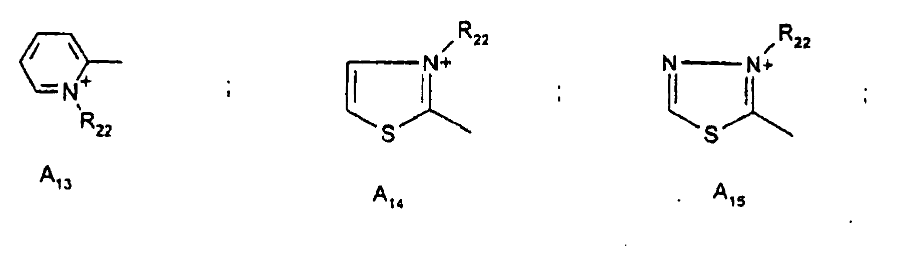 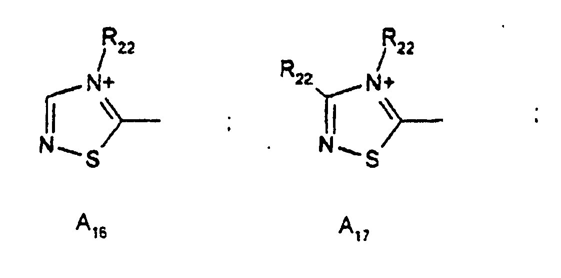 et 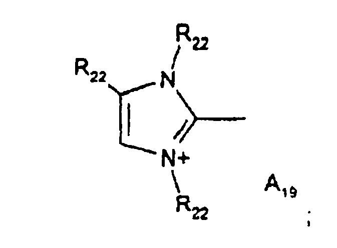
      dans lesquelles R₂₂ représente un radical alkyle en C₁-C₄ pouvant être substitué par un radical hydroxyle et R₂₃ représente un radical alcoxy en C₁-C₄;
   b) les composés de formule (VI) suivante : 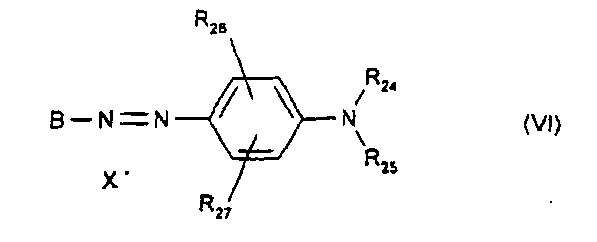 dans laquelle :
      R₂₄ représente un atome d'hydrogène ou un radical alkyle en C₁-C₄,
      R₂₅ représente un atome d'hydrogène, un radical alkyle pouvant être substitué par un radical -CN ou par un groupement amino, un radical 4'-aminophényle ou forme avec R₂₄ un hétérocycle éventuellement oxygéné et/ou azoté pouvant être substitué par un radical alkyle en C₁-C₄,
      R₂₆ et R₂₇, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
      X⁻ représente un anion de préférence choisi parmi le chlorure, le méthyl sulfate et l'acétate,
      B représente un groupement choisi par les structures B1 à B6 suivantes : 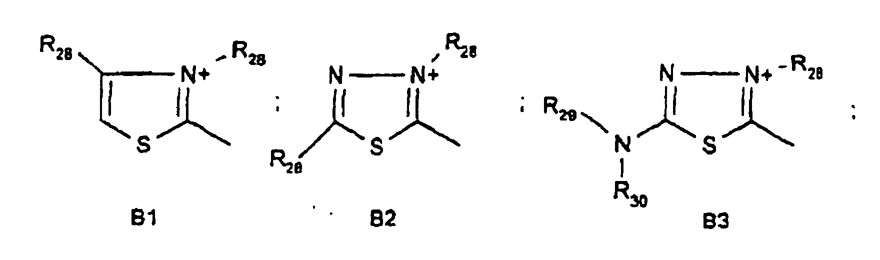 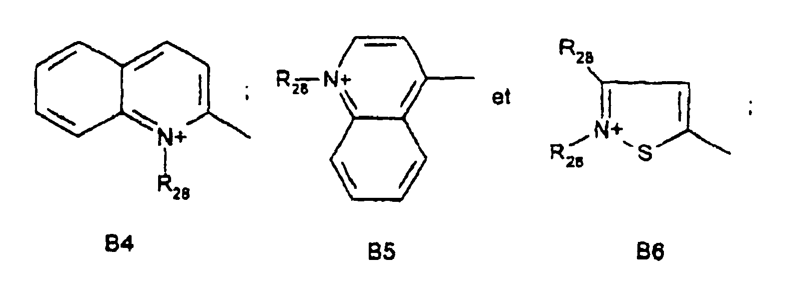
      dans lesquelles R₂₈ représente un radical alkyle en C₁-C₄, R₂₉ et R₃₀, identiques ou différents, représentent un atome d'hydrogène ou un radical alkyle en C₁-C₄ ; et
   c) les composés de formules (VII) et (VII') suivantes : 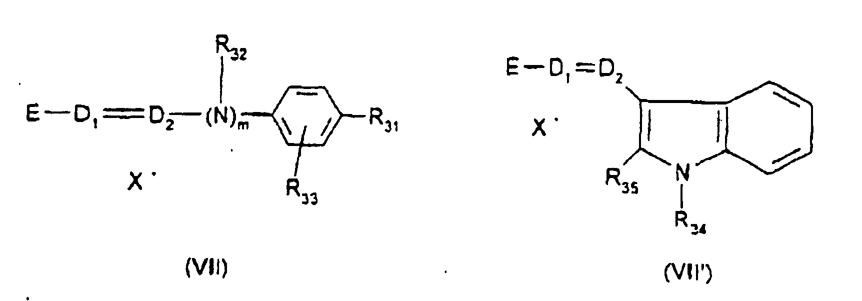 dans lesquelles :
      R₃₁ représente un atome d'hydrogène, un radical alcoxy en C₁-C₄, un atome d'halogène tel que le brome, le chlore, l'iode ou le fluor ou un radical amino,
      R₃₂ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
      R₃₃ représente un atome d'hydrogène ou d'halogène tel que le brome, le chlore, l'iode ou le fluor,
      R₃₄ et R₃₅, identiques ou différents, représentent un atome d'hydrogène ou un radical alkyle en C₁-C₄,
      D₁ et D₂, identiques ou différents, représentent un atome d'azote ou le groupement -CH,
      m = 0 ou 1,
      étant entendu que lorsque R₃₁ représente un groupement amino non substitué, alors D₁ et D₂ représentent simultanément un groupement -CH et m = 0,
      X⁻ représente un anion de préférence choisi parmi le chlorure, le méthyl sulfate et l'acétate,
      E représente un groupement choisi par les structures E1 à E8 suivantes : 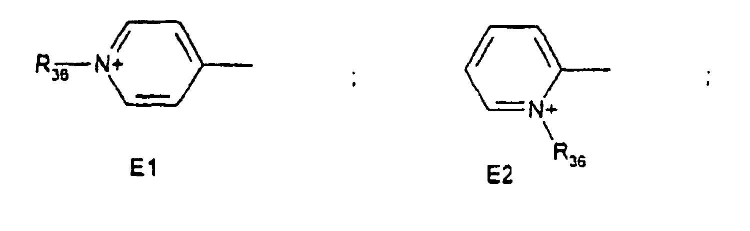 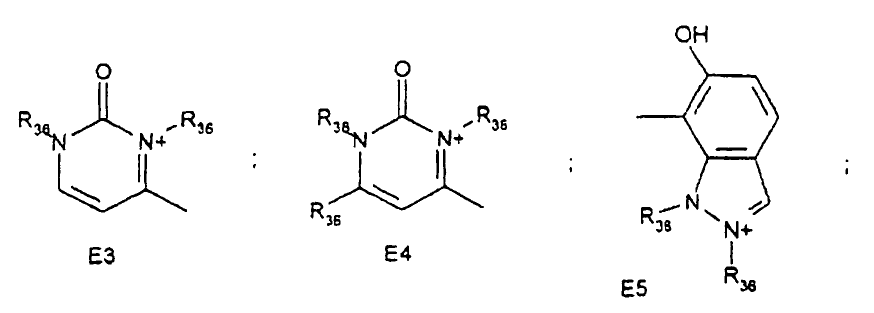 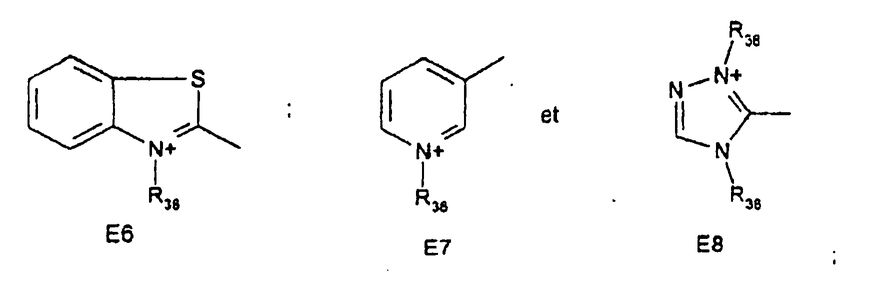 dans lesquelles R₃₆ représente un radical alkyle en C₁-C₄;
      lorsque m = 0 et que D₁ représente un atome d'azote, alors E peut également désigner un groupement de structure E9 suivante : 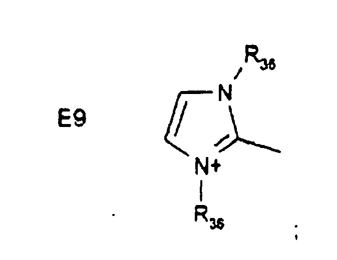 dans laquelle R₃₆ représente un radical alkyle en C₁-C₄,
- au moins une enzyme de type laccase.
et en ce qu'elle présente un pH compris entre 6 et 9.

La composition tinctoriale prête à l'emploi conforme à l'invention conduit à des colorations puissantes, chromatiques, présentant une faible sélectivité et d'excellentes propriétés de résistances à la fois vis à vis des agents atmosphériques tels que la lumière et les intempéries et vis à vis de la transpiration et des différents traitements que peuvent subir les cheveux (lavages, déformations permanentes).

L'invention a également pour objet un procédé de teinture d'oxydation des fibres kératiniques mettant en oeuvre cette composition tinctoriale prête à l'emploi.

La ou les laccases utilisées dans la composition tinctoriale prête à l'emploi conforme à l'invention peuvent notamment être choisies parmi les laccases d'origine végétale, d'origine animale, d'origine fongique (levures, moisissures, champignons) ou d'origine bactérienne, les organismes d'origine pouvant être mono ou pluricellulaires. Elles peuvent être obtenues par biotechnologie.

Parmi les laccases d'origine végétale utilisables selon l'invention , on peut citer les laccases produites par des végétaux effectuant la synthèse chlorophyllienne telles qu'indiquées dans la demande FR-A-2 694 018 comme celles que l'on retrouve dans les extraits des Anacardiacées tels que par exemple les extraits de Magnifera indica, Schinus molle ou Pleiogynium timoriense, dans les extraits des Podocarpacées , de Rosmarinus off., de Solanum tuberosum, d'Iris sp., de Coffea sp., de Daucus carrota, de Vinca minor, Persea americana, Catharenthus roseus, Musa sp., Malus pumila, Gingko biloba, Monotropa hypopithys (sucepin), Aesculus sp., Acer pseudopiatanus, Prunus persica, Pistacia palaestina.

Parmi les laccases d'origine fongique éventuellement obtenues par biotechnologie utilisables selon l'invention, on peut citer la ou les laccases issues de Polyporus versicolor, de Rhizoctonia praticola et de Rhus vernicifera comme indiquées dans les demandes FR-A-2 112 549 et EP-A-504005 ; celles décrites dans les demandes de brevet WO95/07988, WO95/33836, WO95/33837, WO96/00290, WO97/19998 et WO97/19999, dont le contenu fait partie intégrante de la présente description comme par exemple celles issues de Scytalidium, de Polyporus pinsitus, de Myceliophtora thermophila, de Rhizoctonia solani, de Pyricularia orizae, ou leurs variantes. On peut aussi citer celles issues de Tramates versicolor, de Fomes fomentarius, de Chaetomium thermophile, de Neurospora crassa, de Coriolus versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens et de leurs variantes.

On choisira plus préférentiellement les laccases d'origine fongique éventuellement obtenues par biotechnologie.

L'activité enzymatique des laccases de l'invention ayant la syringaldazine parmi leurs substrats peut être définie à partir de l'oxydation de la syringaldazine en condition aérobie. L'unité lacu correspond à la quantité d'enzyme catalysant la conversion de 1mmole de syringaldazine par minute à pH 5,5 à 30°C. L'unité u correspond à la quantité d'enzyme produisant un delta d'absorbance à 530 nm de 0,001 par minute en utilisant la syringaldazine comme substrat, à 30°C et à pH 6,5.
L'activité enzymatique des laccases de l'invention peut aussi être définie à partir de l'oxydation de la paraphénylènediamine. L'unité ulac correspond à la quantité d'enzyme produisant un delta d'absorbance à 496,5nm de 0,001 par minute en utilisant la paraphénylènediamine comme substrat (64 mM) à 30°C et à pH 5.
Selon l'invention, on préfère déterminer l'activité enzymatique en unités ulac.
Les quantités de laccase utilisées dans les compositions de l'invention varieront en fonction de la nature de la laccase choisie. De façon préférentielle, elles varieront de 0,5 à 2000 lacu, ou de 1000 à 4.10⁷ unités u, ou de 20 à 2.10⁶ unités ulac pour 100g de composition.

La nature de la ou des bases d'oxydation et/ou des coupleurs utilisés dans la composition tinctoriale prête à l'emploi n'est pas critique.

Les bases d'oxydation peuvent notamment être choisies parmi les paraphénylènediamines, les bases doubles, les para-aminophénols, les ortho aminophénols et les bases d'oxydation hétérocycliques.

Parmi les paraphénylènediamines utilisables à titre de base d'oxydation dans les composition tinctoriales conformes à l'invention, on peut notamment citer les composés de formule (I) suivante et leurs sels d'addition avec un acide : 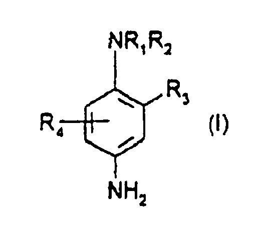 dans laquelle :
- R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₂ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₃ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₄ représente un atome d'hydrogène, d'halogène ou un radical alkyle en C₁-C₄.

Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino. imidazolinium et ammonium.

Parmi les paraphénylènediamines de formule (I) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines de formule (I) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (II) suivante, et leurs sels d'addition avec un acide : 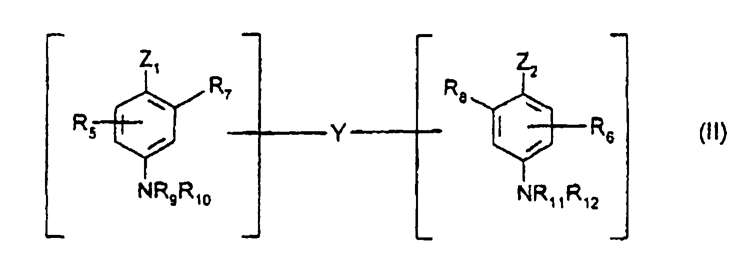 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀ R₁₁ et R₁₂, identiques ou différents, représentent un atome d'hydrogène, un bras de liaison Y ou un radical alkyle en C₁-C₄ ;
étant entendu que les composés de formule (II) ne comportent qu'un seul bras de liaison Y par molécule.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (il) ci-dessus, on peut plus particulièrement citer le N,N'-bis-(β-hydroxyèthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylénediamine, la N,N'-bis-(4-méthyl-aminophényl) tétraméthylènediamine, la N.N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (II), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols utilisables à titre de bases d'oxydation dans les compositions tinctoriales conformes à l'invention, on peut notamment citer les composés répondant à la formule (III) suivante, et leurs sels d'addition avec un acide : 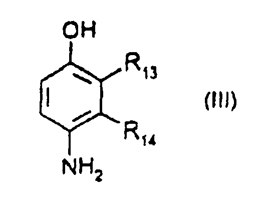 dans laquelle :
- R₁₃ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₁₄ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₃ ou R₁₄ représente un atome d'hydrogène.

Parmi les para-aminophénols de formule (III) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables à titre de bases d'oxydation dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, les dérivés pyrazolo-pyrimidiniques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-333 495 ou demandes de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxymèthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Parmi les dérivés pyrazolo-pyrimidiniques, on peut plus particulièrement citer les pyrazolo-[1,5-a]-pyrimidines de formule (IV) suivante, leurs sels d'addition avec un acide ou avec une base et leurs formes tautomères, lorsqu'il existe un équilibre tautomérique : 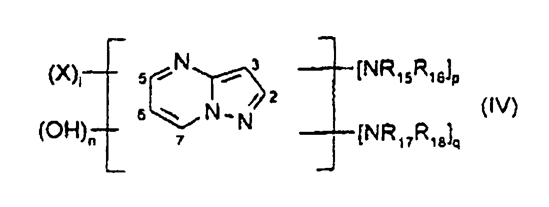 dans laquelle :
- R₁₅, R₁₆, R₁₇ et R₁₈, identiques ou différents désignent, un atome d'hydrogène, un radical alkyle en C₁-C₄, un radical aryle, un radical hydroxyalkyle en C₁-C₄, un radical polyhydroxyalkyle en C₂-C₄, un radical (C₁-C₄)alcoxy alkyle en C₁-C₄, un radical aminoalkyle en C₁-C₄ (l'amine pouvant être protégée par un radical acétyle, uréido ou sulfonyle), un radical (C₁-C₄)alkylamino alkyle en C₁-C₄, un radical di-[(C₁-C₄)alkyl] amino alkyle en C₁-C₄ (les radicaux dialkyles pouvant former un cycle carboné ou un hétérocycle à 5 ou 6 chaînons), un radical hydroxy(C₁-C₄)alkyl- ou di-[hydroxy(C₁-C₄) alkyl]-amino alkyle en C₁-C₄;
- les radicaux X désignent , identiques ou différents, un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄ (les dialkyles pouvant former un cycle carboné ou un hétérocycle à 5 ou 6 chaînons), un radical hydroxy(C₁-C₄)alkyl ou di-[hydroxy(C₁-C₄ )alkyl]amino alkyle en C₁-C₄, un radical amino, un radical (C₁-C₄)alkyl- ou di-[(C₁-C₄)alkyl]-amino ; un atome d'halogène, un groupe acide carboxylique, un groupe acide sulfonique ;
- i vaut 0, 1, 2 ou 3 ;
- p vaut 0 ou 1 ;
- q vaut 0 ou 1 ;
- n vaut 0 ou 1 ;
sous réserve que :
- la somme p + q est différente de 0 ;
- lorsque p + q est égal à 2, alors n vaut 0 et les groupes NR₁₅R₁₆ et NR₁₇R₁₈ occupent les positions (2,3) ; (5,6) ; (6,7) ; (3,5) ou (3,7) ;
- lorsque p + q est égal à 1 alors n vaut 1 et le groupe NR₁₅R₁₆ (ou NR₁₇R₁₈) et le groupe OH occupent les positions (2,3) ; (5,6) ; (6,7) ; (3,5) ou (3,7) ;

Lorsque les pyrazolo-[1,5-a]-pyrimidines de formule (IV) ci-dessus sont telles qu'elles comportent un groupe hydroxyle sur l'une des positions 2, 5 ou 7 en α d'un atome d'azote, il existe un équilibre tautomérique représenté par exemple par le schéma suivant :

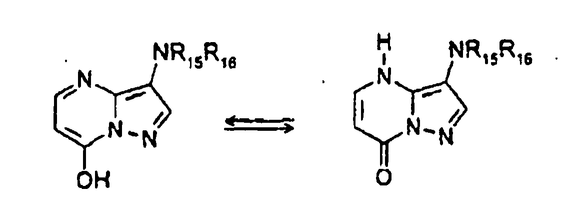

Parmi les pyrazolo-[1,5-a]-pyrimidines de formule (IV) ci-dessus on peut notamment citer :
- la pyrazolo-[1,5-a]-pyrimidine-3,7-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol
- le 3-amino pyrazolo-[1,5-a]-pyrimidin-5-ol
- le 2-(3-amino pyrazolo-[1,5-a]-pyrimidin-7-ylamino)-éthanol
- le 2-(7-amino pyrazolo-[1,5-a]-pyrimidin-3-ylamino)-éthanol
- le 2-[(3-Amino-pyrazolo[1,5-a]pyrimidin-7-yl)-(2-hydroxy-éthyl)-amino]-éthanol
- le 2-[(7-Amino-pyrazolo[1,5-a]pyrimidin-3-yl)-(2-hydroxy-éthyl)-amino]-éthanol
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N 7, N 7-tetraméthyl pyrazolo-[1,5-a]-pyrimidine-3,7-diamine ;
et leurs sels d'addition et leurs formes tautomères, lorsqu'il existe un équilibre tautomérique.

Les pyrazolo-[1,5-a]-pyrimidines de formule (IV) ci-dessus peuvent être préparées par cyclisation à partir d'un aminopyrazole selon les synthèses décrites dans les références suivantes :
- EP 628559 BEIERSDORF-LILLY
- R. Vishdu. H. Navedul, Indian J. Chem., 34b (6), 514, 1995.
- N.S. Ibrahim, K.U. Sadek, F.A. Abdel-AI, Arch. Pharm., 320, 240, 1987.
- R.H. Springer, M.B. Scholten, D.E. O'Brien, T. Novinson, J.P. Miller, R.K. Robins. J. Med. Chem., 25, 235, 1982.
- T. Novinson, R.K. Robins, T.R. Matthews, J. Med. Chem., 20, 296, 1977.
- US 3907799 ICN PHARMACEUTICALS

Les pyrazolo-[1,5-a]-pyrimidines de formule (IV) ci-dessus peuvent également être préparées par cyclisation à partir d'hydrazine selon les synthèses décrites dans les références suivantes :
- A. McKillop et R.J. Kobilecki, Heterocycles, 6(9), 1355, 1977.
- E. Alcade, J. De Mendoza, J.M. Marcia-Marquina, C. Aimera, J. Elguero, J. Heterocyclic Chem., 11(3), 423, 1974.
- K. Saito, I. Hori, M. Higarashi, H. Midorikawa, Bull. Chem. Soc. Japan, 47(2), 476, 1974.

La ou les bases d'oxydation représentent de préférence de 0,0005 à 12 % en poids environ du poids total de la composition tinctoriale conforme à l'invention, et encore plus préférentiellement de 0,005 à 6 % en poids environ de ce poids.

Le ou les coupleurs pouvant être utilisés dans la composition tinctoriale prête à l'emploi conforme à l'invention sont ceux classiquement utilisés dans les compositions de teinture d'oxydation, c'est-à-dire des méta-phénylènediamines, des méta-aminophénols, des métadiphénols, des coupleurs hétérocycliques, et leurs sels d'addition avec un acide.

Ces coupleurs peuvent notamment être choisis parmi le 2-méthyl-5-amino-phénol, le 5-N-(β-hydroxyéthyl)-amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2,4-diamino-1-(β-hydroxyéthyloxy)-benzène, le 2-amino-4-(β-hydroxyéthylamino)-1-méthoxy-benzène, le 1,3-diamino-benzène, le 1,3-bis-(2,4-diaminophénoxy)-propane, le sésamol, l'α-naphtol, le 6-hydroxy-indole, le 4-hydroxy-indole, le 4-hydroxy-N-méthyl-indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, le 2,6-diméthyl-pyrazolo-[1,5-b]-1,2,4-triazole, le 2,6-diméthyl-[3,2-c]-1,2,4-triazole, le 6-méthyl-pyrazolo-[1,5-a]-benzimidazole, et leurs sels d'addition avec un acide.

Ces coupleurs représentent de préférence de 0,0001 à 10 % en poids environ du poids total de la composition tinctoriale prête à l'emploi et encore plus préférentiellement de 0,005 à 5 % en poids environ de ce poids.

Les colorants directs cationiques de formules (V), (VI), (VII) et (VII') utilisables dans la composition tinctoriale prête à l'emploi conforme à l'invention, sont des composés connus et sont décrits par exemple dans les demandes de brevets WO 95/01772, WO 95/15144 et EP-A-0 714 954.

Parmi les colorants directs cationiques de formule (V) utilisables dans les compositions tinctoriales prêtes à l'emploi conformes à l'invention, on peut plus particulièrement citer les composés répondant aux structures (V1) à (V52) suivantes : 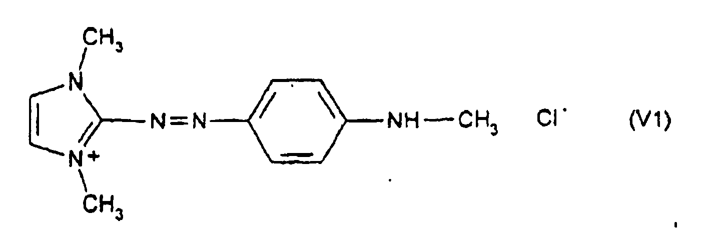 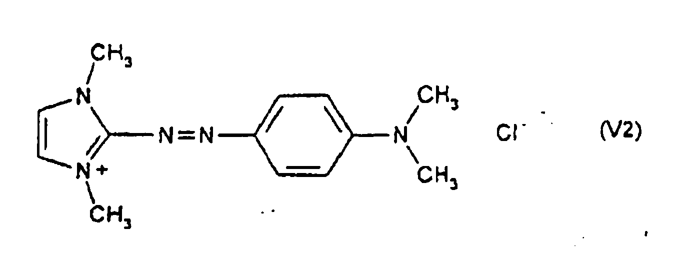 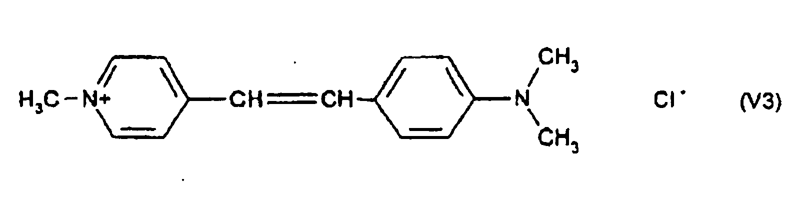 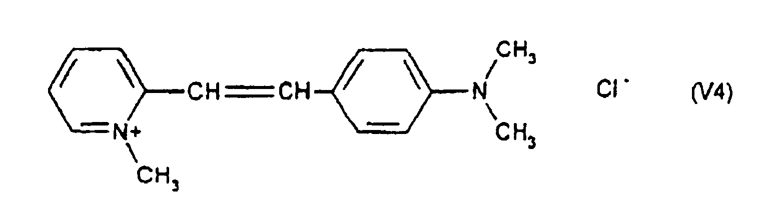 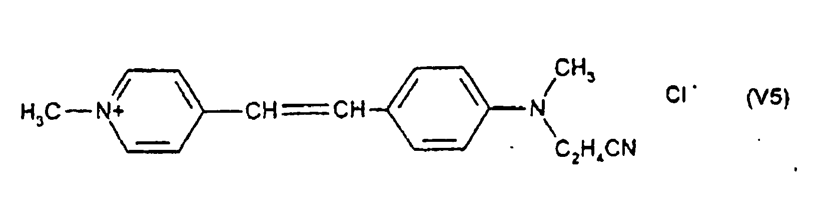 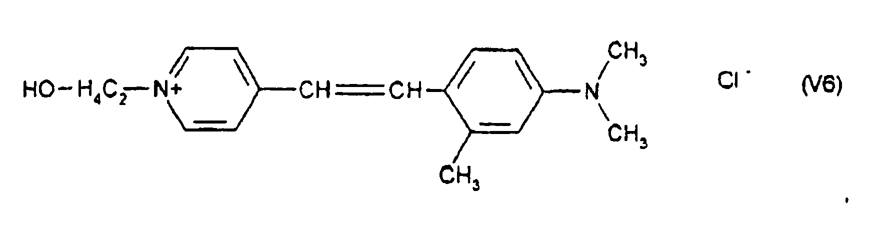 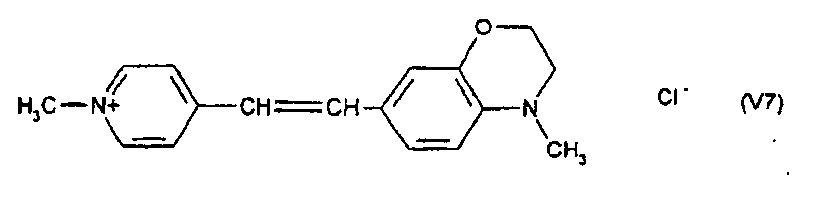 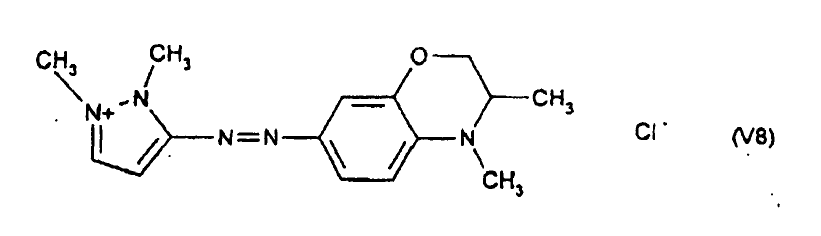 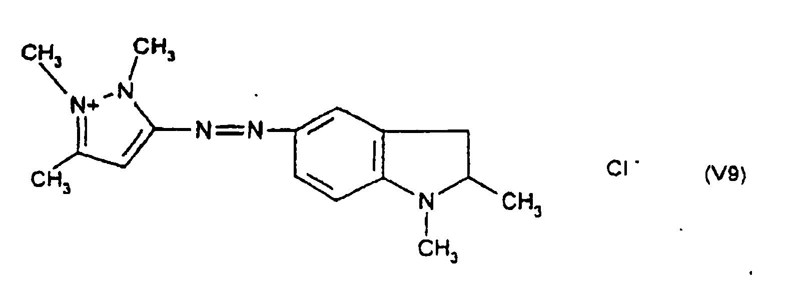 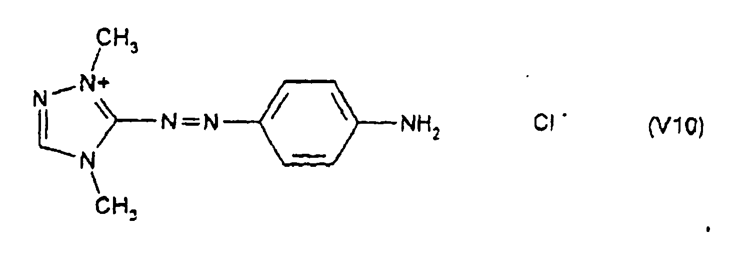 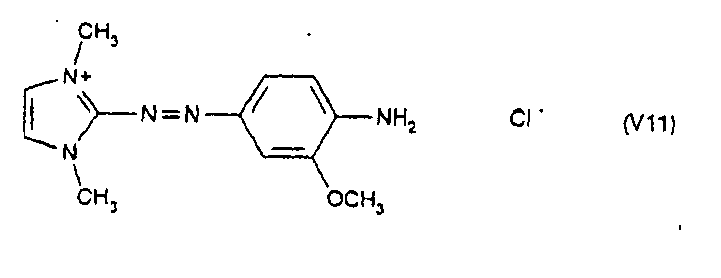 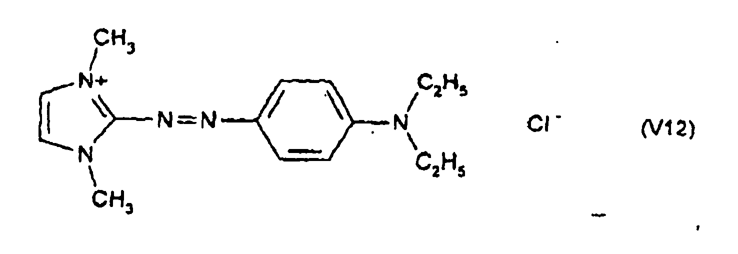 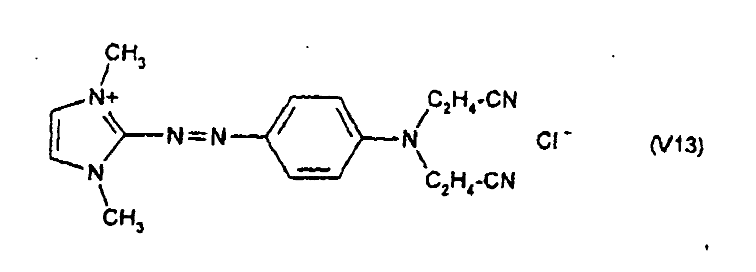 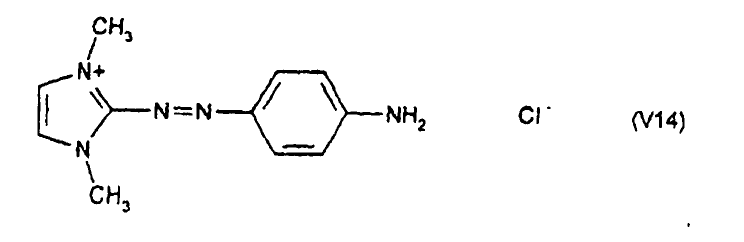 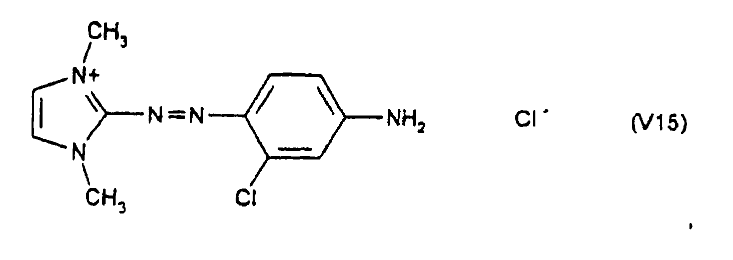 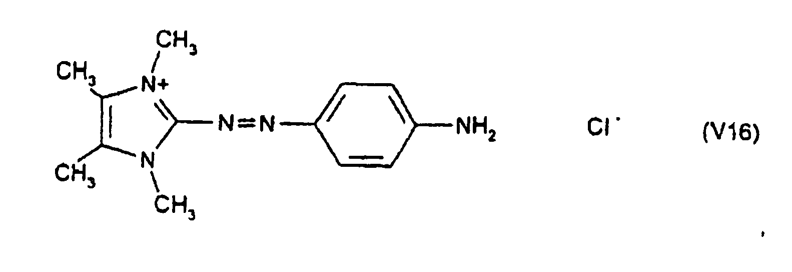 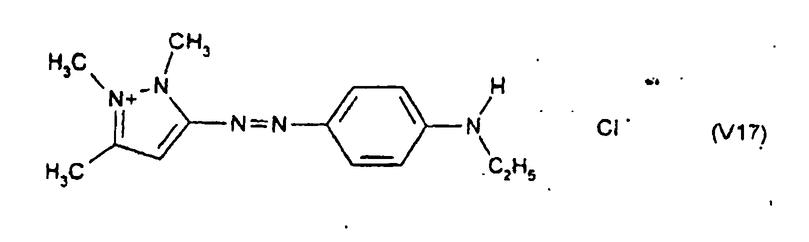 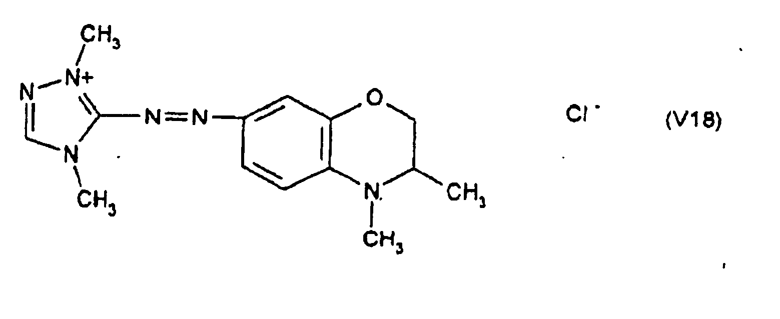 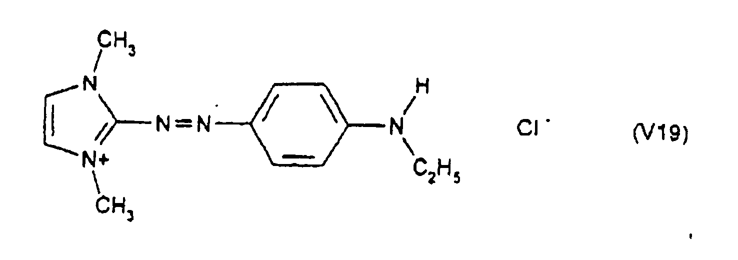 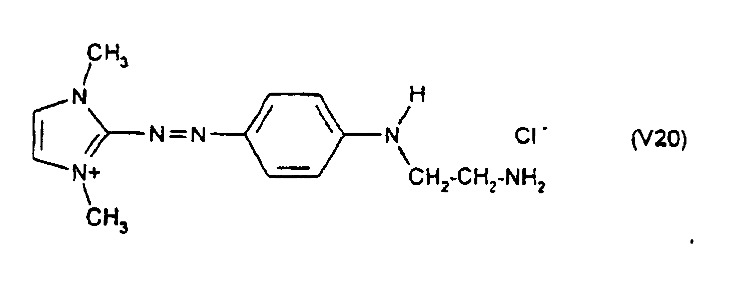 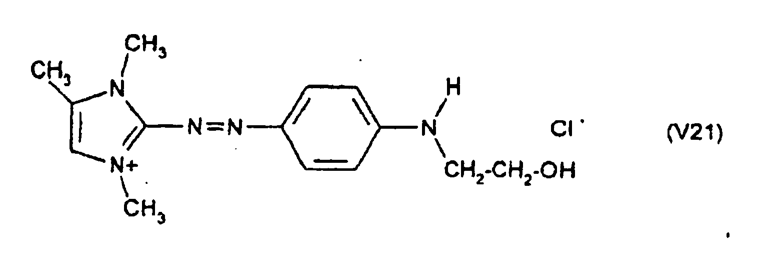 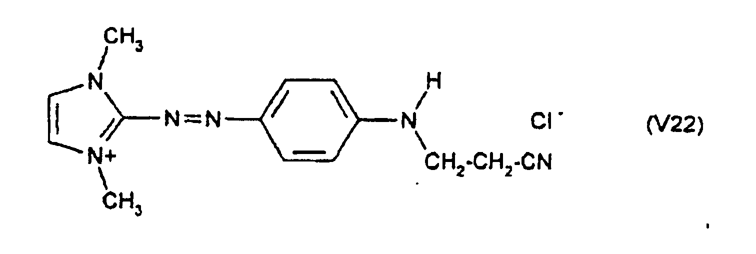 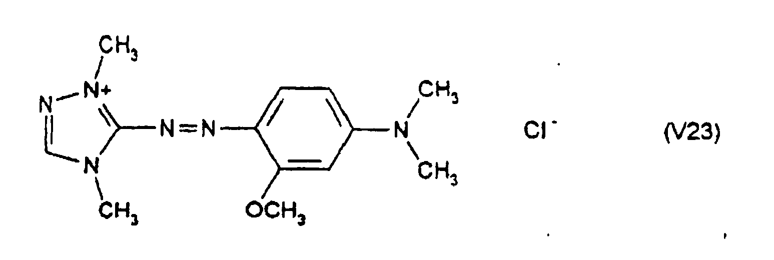 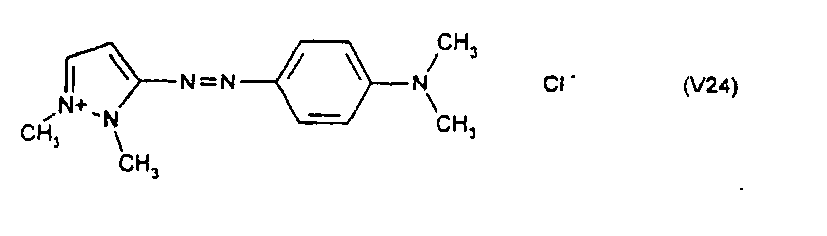 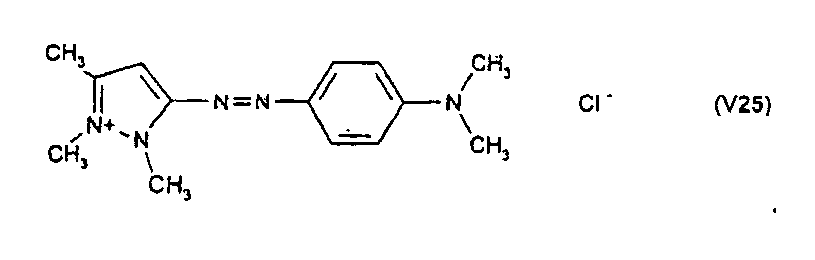 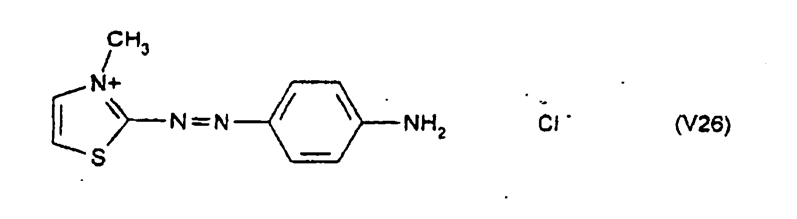 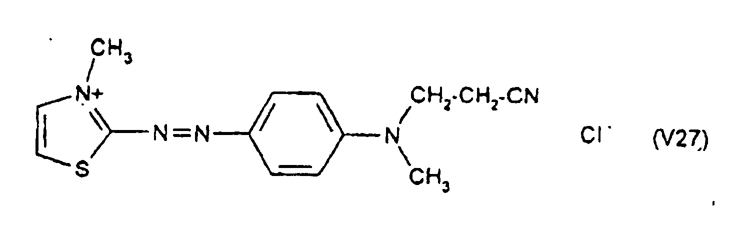 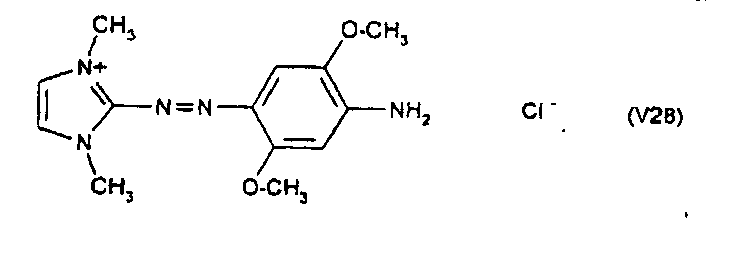 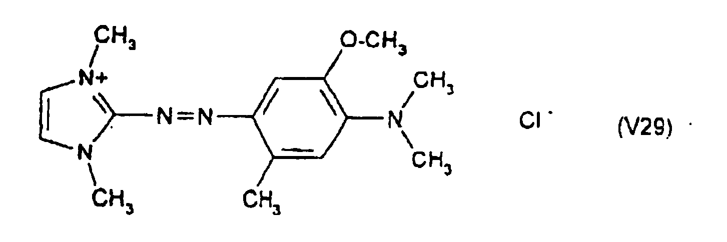 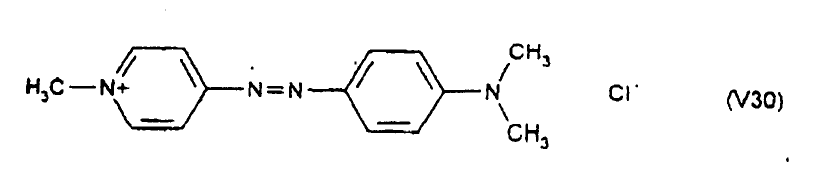 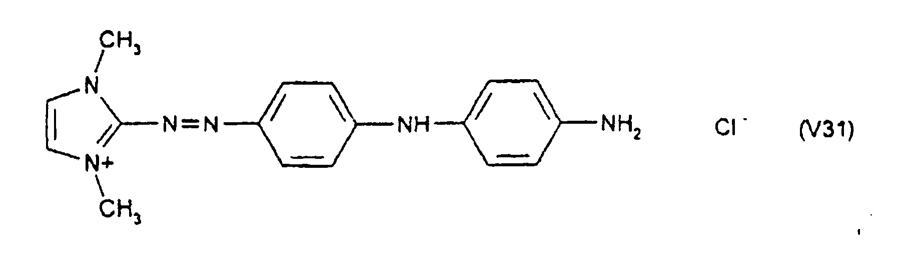 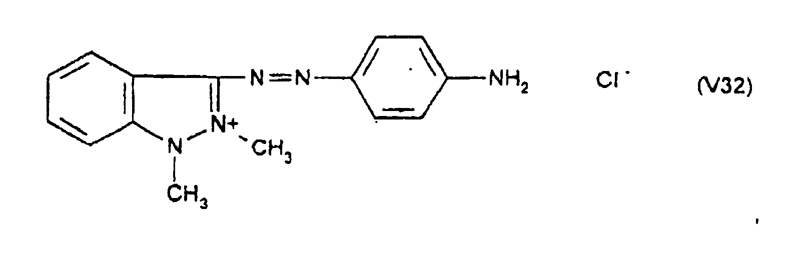 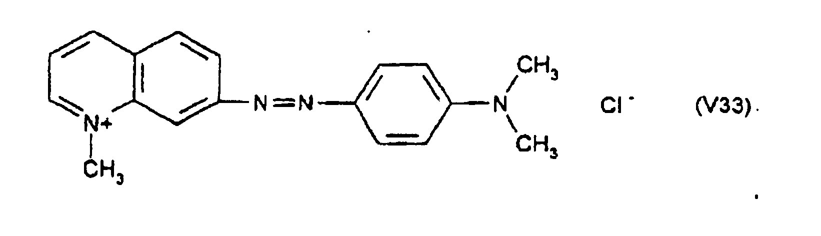 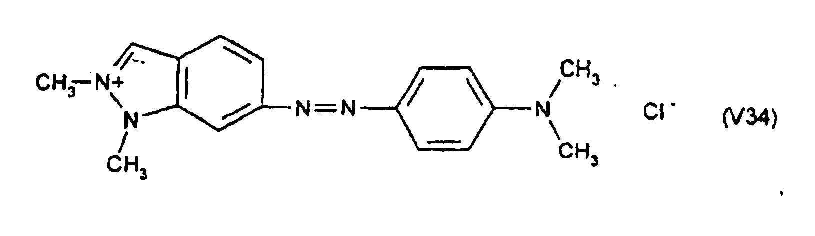 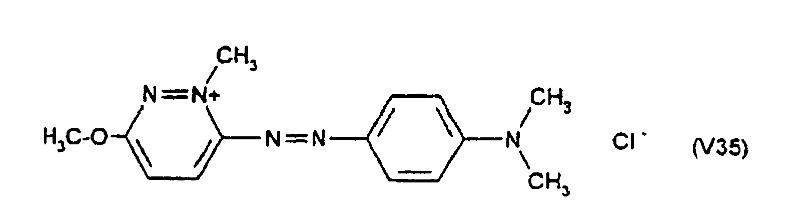 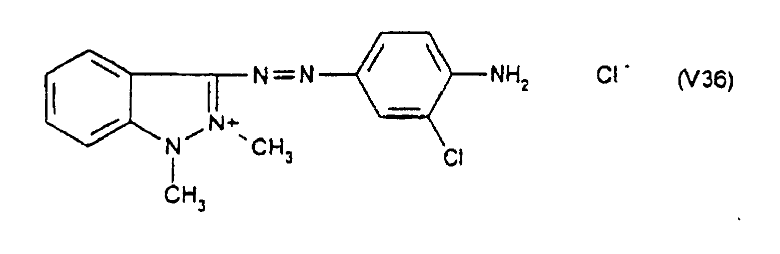 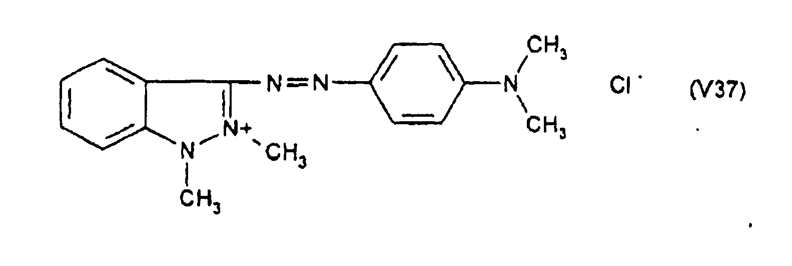 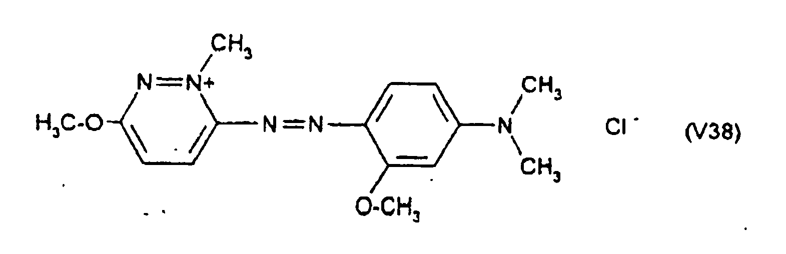 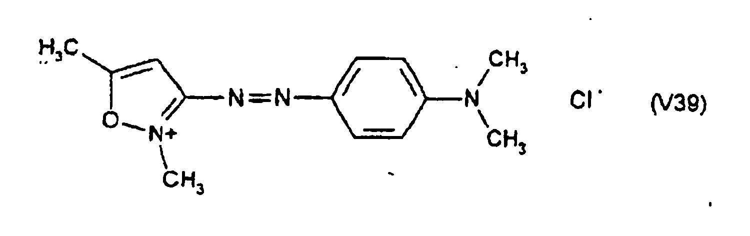 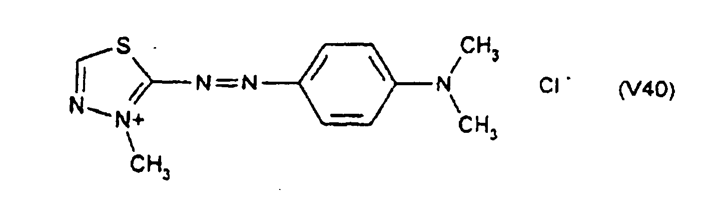 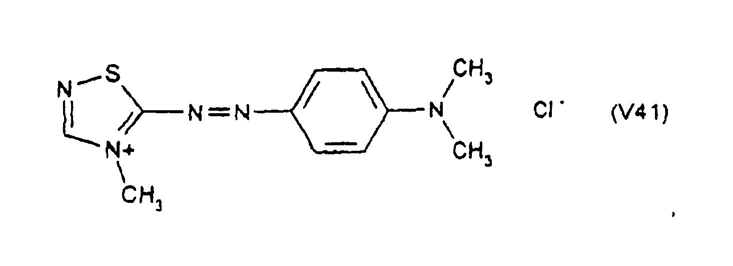 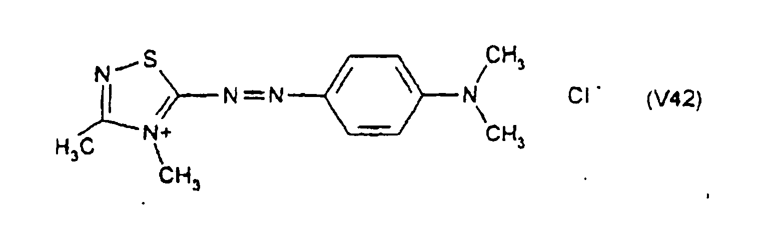 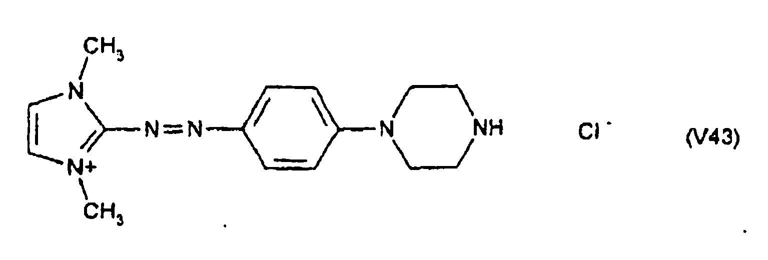 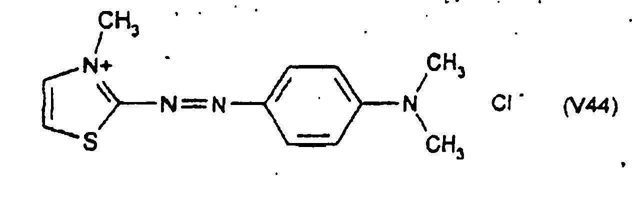 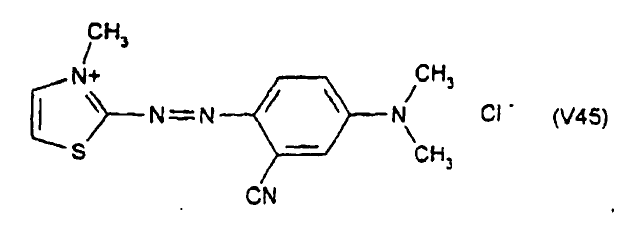 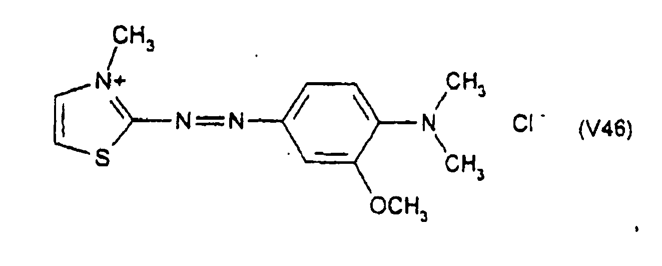 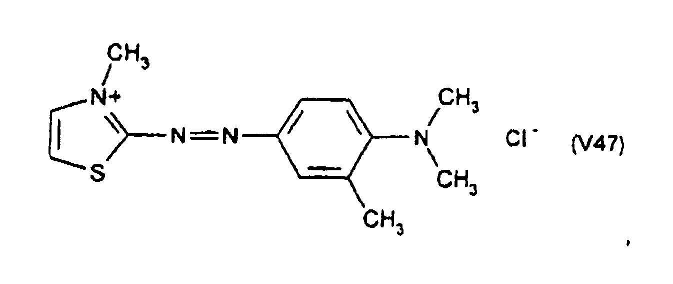 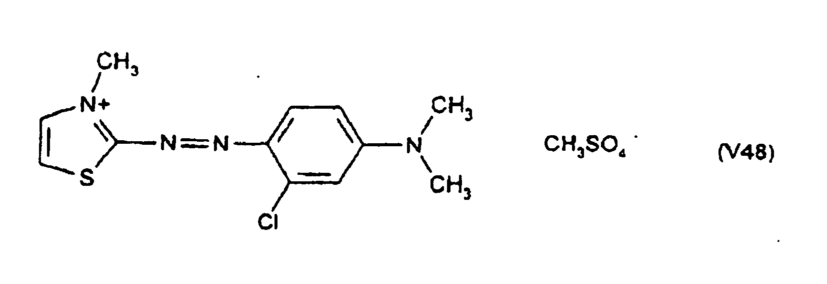 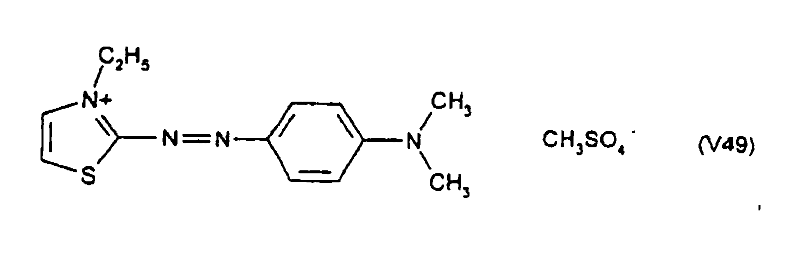 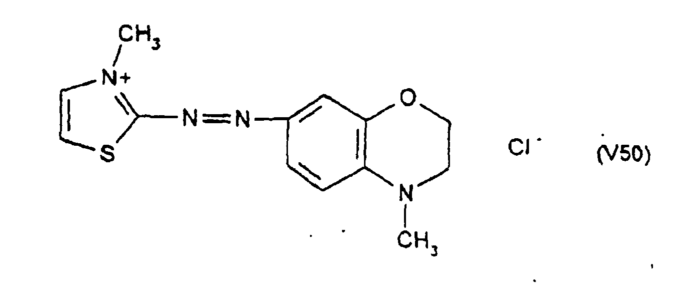 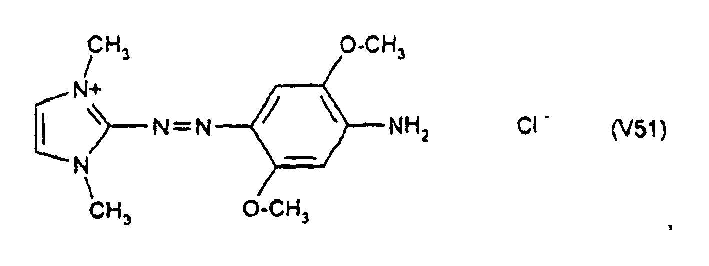 , et

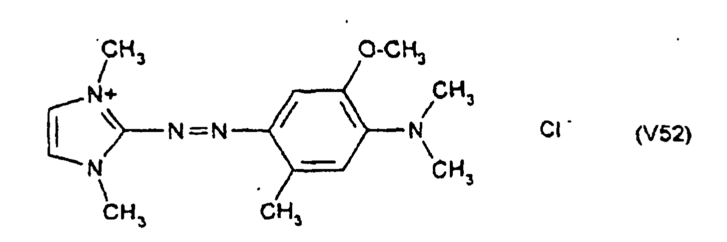

Parmi les composés de structures (V1) à (V52) décrits ci-dessus, on préfère tout particulièrement les composés répondant aux structures (V1), (V2), (V4), (V14) et (V31).

Parmi les colorants directs cationiques de formule (VI) utilisables dans les compositions tinctoriales prêtes à l'emploi conformes à l'invention, on peut plus particulièrement citer les composés répondant aux structures (VI1) à (VI12) suivantes : 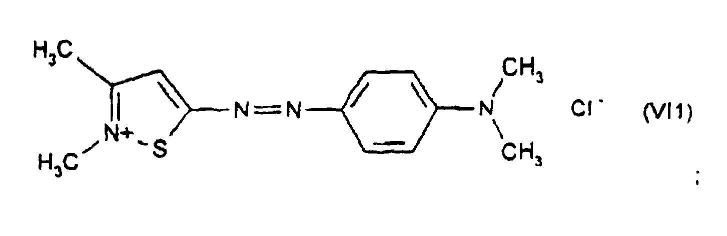 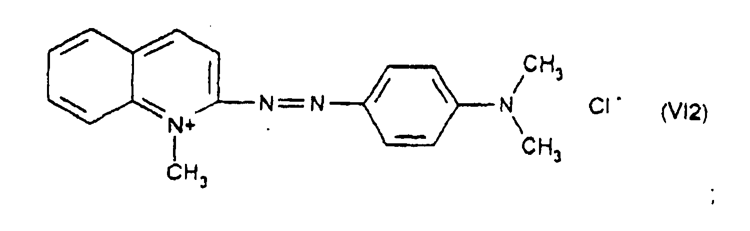 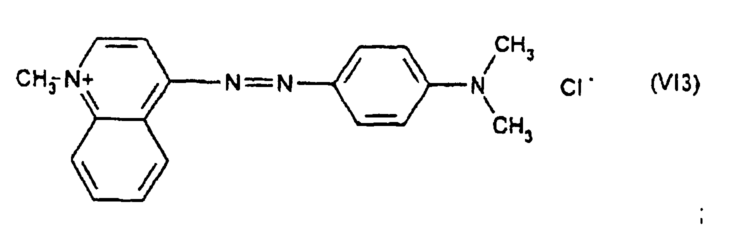 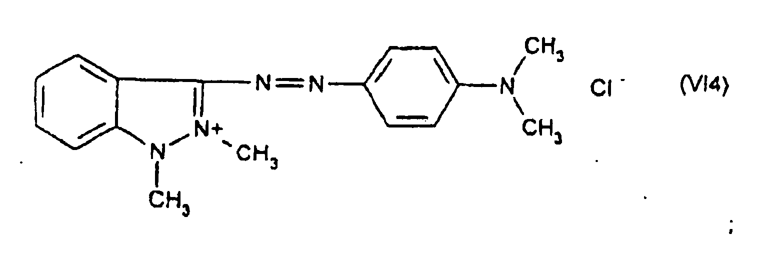 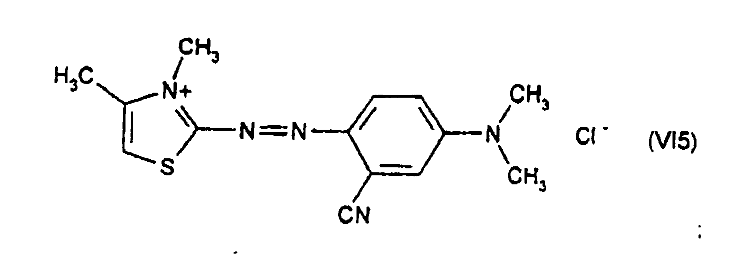 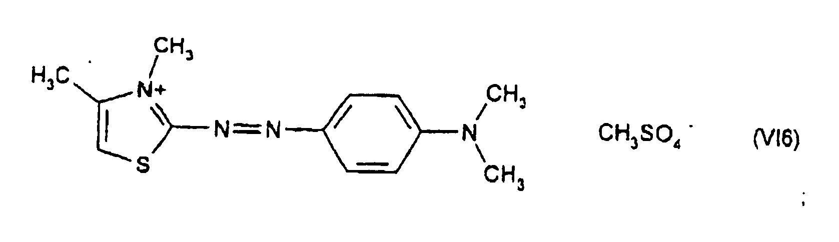 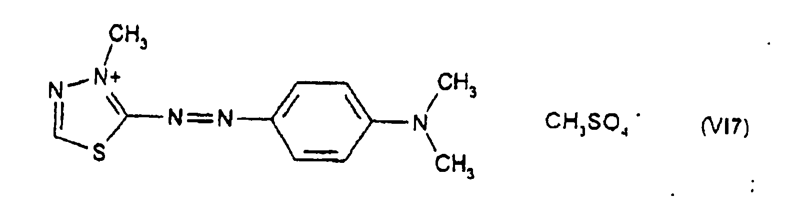 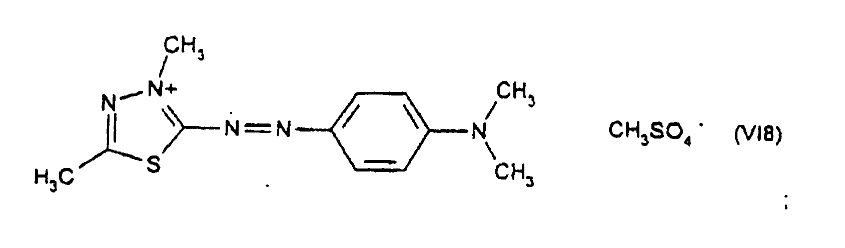 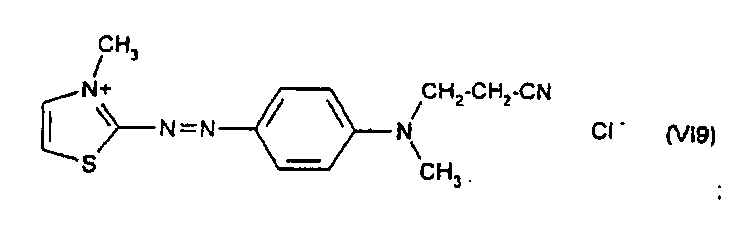 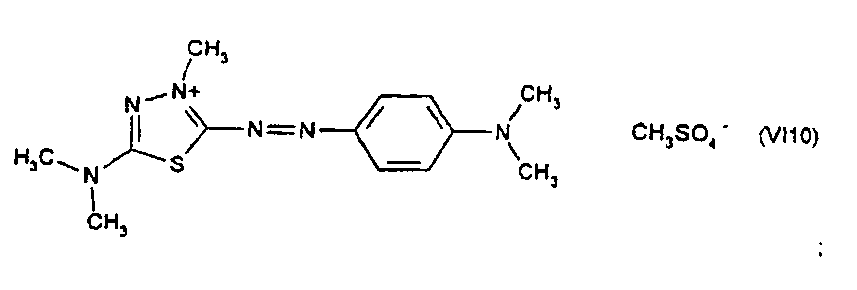 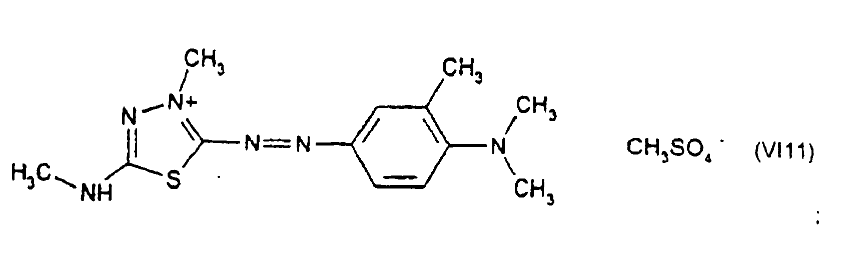 et

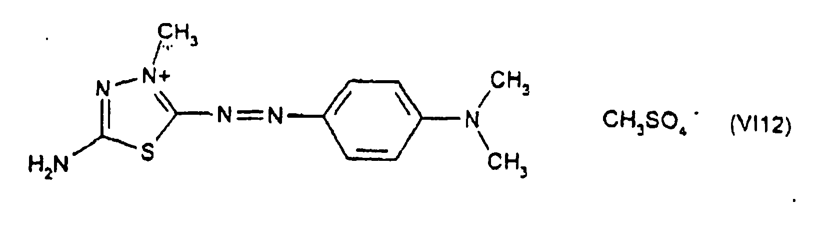

Parmi les colorants directs cationiques de formule (VII), utilisables dans les compositions tinctoriales prêtes à l'emploi conformes à l'invention, on peut plus particulièrement citer les composés répondant aux structures (VII1) à (VII18) suivantes : 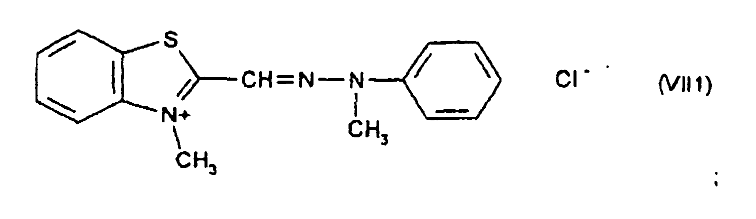 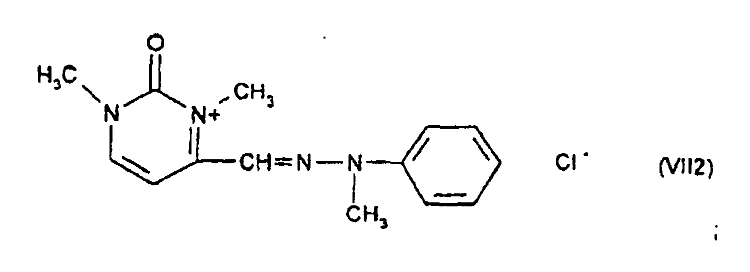 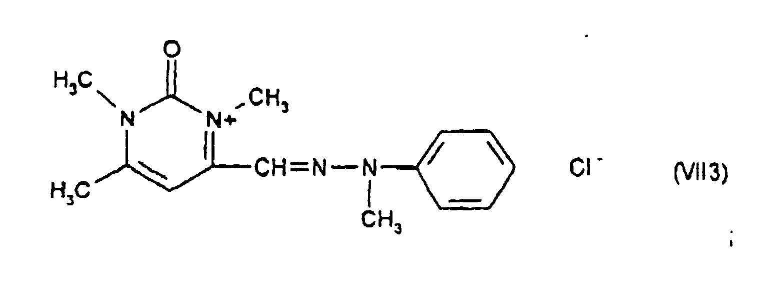 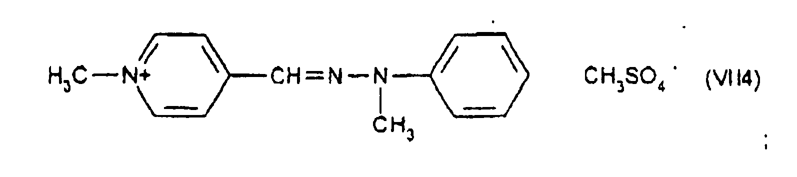 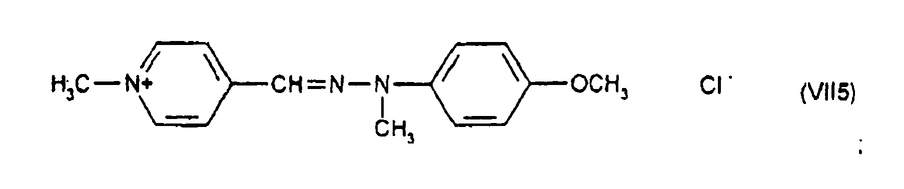 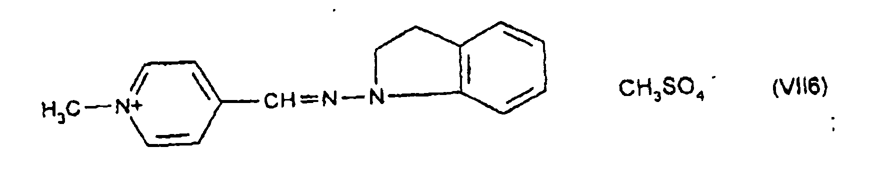 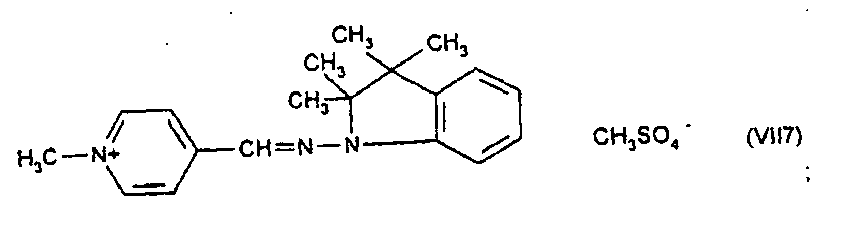 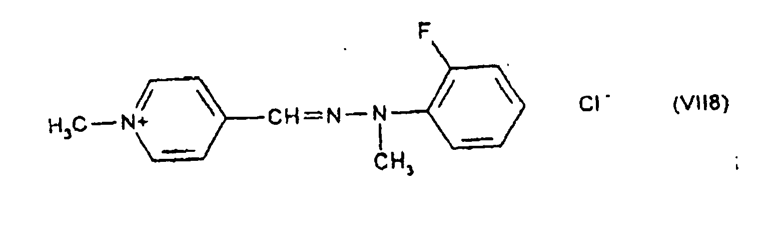 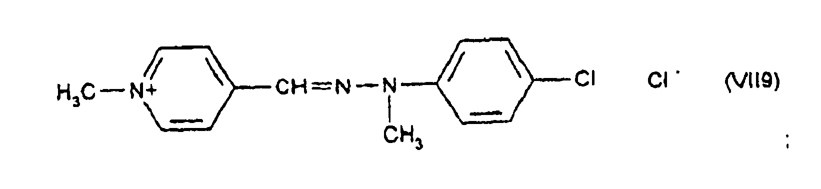 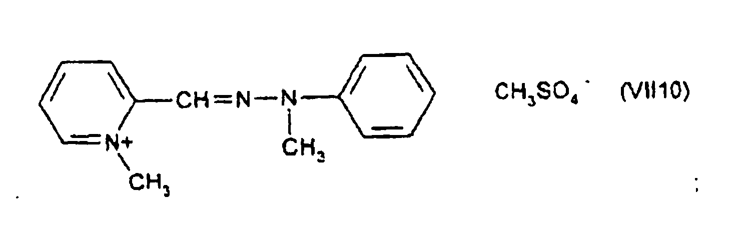 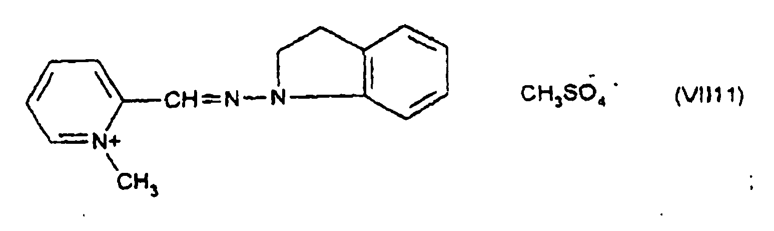 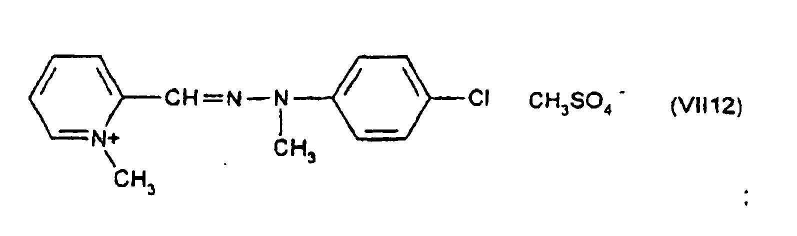 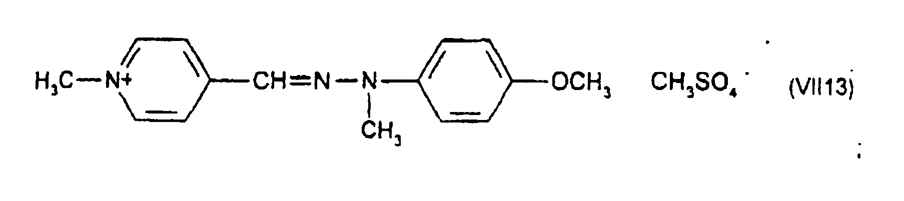 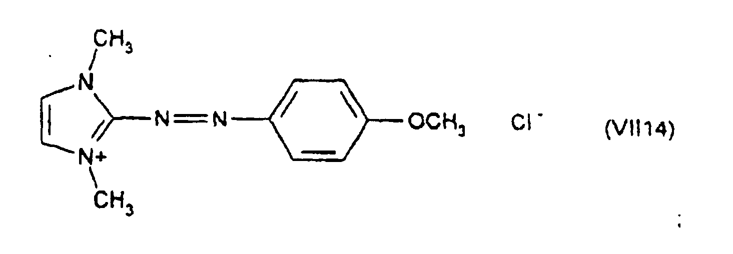 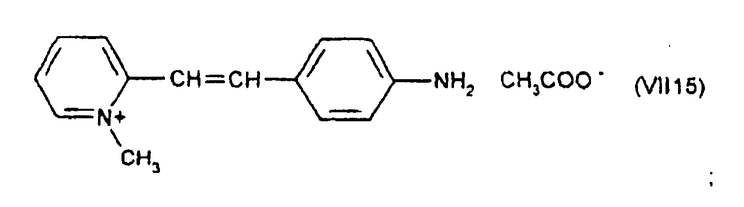 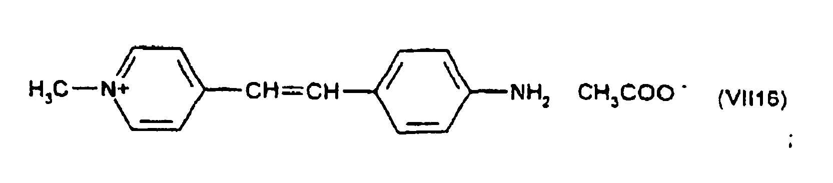 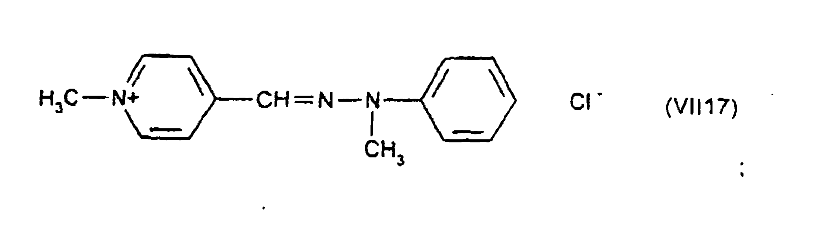 et

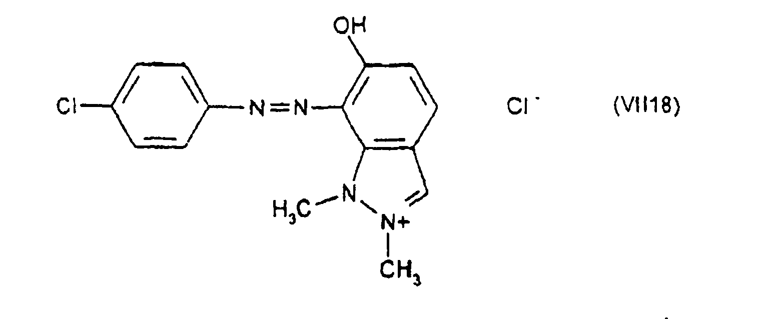

Parmi les composés particuliers de structures (VII1) à (VII18) décrits ci-dessus, on préfère tout particulièrement les composés répondant aux structures (VII4), (VII5) et (VII13).

Parmi les colorants directs cationiques de formule (VII'), utilisables dans les compositions tinctoriales prêtes à l'emploi conformes à l'invention, on peut plus particulièrement citer les composés répondant aux structures (VII'1) à (VII'3) suivantes : 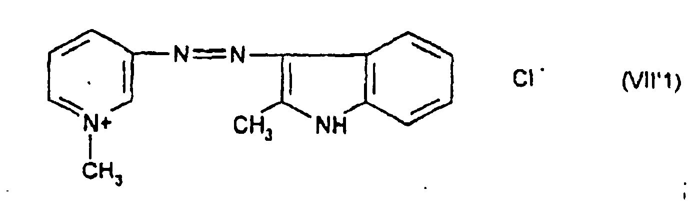 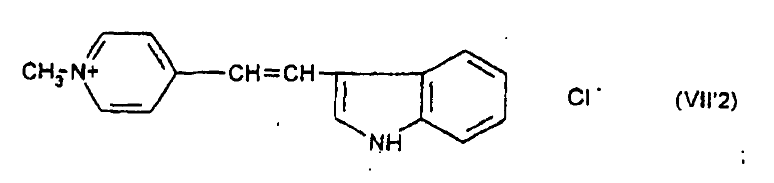 et

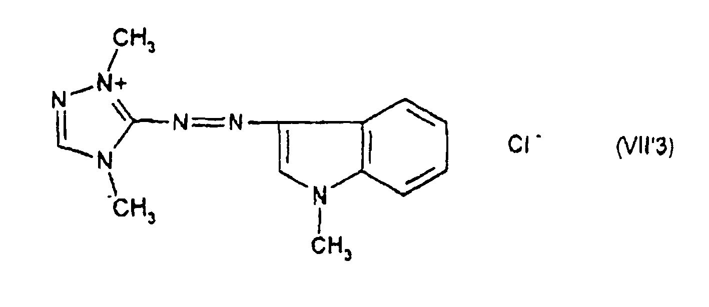

Le ou les colorants directs cationiques utilisés selon l'invention, représentent de préférence de 0,001 à 10 % en poids environ du poids total de la composition tinctoriale prête à l'emploi et encore plus préférentiellement de 0,05 à 5 % en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Le milieu approprié pour la teinture (ou support) de la composition tinctoriale prête à l'emploi conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol ainsi que les alcools aromatiques comme l'alcool benzylique, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition prête à l'emploi conforme à l'invention est choisi de telle manière que l'activité enzymatique de la laccase soit suffisante.

La composition tinctoriale prête à l'emploi conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères, des agents épaississsants, des agents antioxydants, des enzymes différentes des laccases utilisées conformément à l'invention telles que par exemple des peroxydases ou des oxydo-réductases à 2 électrons, des agents de pénétration, des agents séquestrants, des parfums, des tampons, des agents dispersants, des agents filmogènes, des agents filtrants, des vitamines, des agents conservateurs, des agents opacifiants.

Bien entendu, l'homme de l'art veillera à choisir ce ou ces éventuels composés complémentaires de manière telle que les propriétés avantageuses attachées intrinsèquement à la composition tinctoriale prête à l'emploi conforme à l'invention ne soient pas, ou substantiellement pas, altérées par la ou les adjonctions envisagées.

La composition tinctoriale prête à l'emploi conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains. Dans ce cas, le ou les colorants d'oxydation et la ou les laccases sont présents au sein de la même composition prête à l'emploi, et par conséquent ladite composition doit être exempte d'oxygène gazeux, de manière à éviter toute oxydation prématurée du ou des colorants d'oxydation.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres au moins une composition tinctoriale prête à l'emploi telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Les exemples qui suivent sont destinés à illustrer l'invention sans pour autant en limiter la portée.

### EXEMPLES 1 et 2 DE TEINTURE

On a préparé les compositions tinctoriales prêtes à l'emploi suivantes (teneurs en grammes) :

| **COMPOSITION** | **1** | **2** |
|---|---|---|
| Paraphénylènediamine (base d'oxydation) | 0,550 | - |
| Para-aminophénol (base d'oxydation) | - | 0,147 |
| 5-N-(β-hydroxyéthyl)amino 2-méthyl phénol (coupleur) | - | 0,165 |
| Colorant direct cationique rouge de structure (V1) | 0,470 | - |
| Colorant direct cationique orangé de structure (V4) | - | 0,051 |
| Laccase issue de Rhus vernicifera laccase à 180 unités/mg vendue par la société SIGMA | 1,8 | 1,8 |
| Support de teinture commun (*) | (*) | (*) |
| Eau déminéralisée q.s.p. | 100 | 100 |

| | | |
|---|---|---|
| (*) : Support de teinture commun : - Ethanol 20,0g - Alkyl (C₈-C₁₀) polyglucoside en solution aqueuse à 60 % de matière active (M.A.), vendu sous la dénomination ORAMIX CG110 ® par la société SEPPIC 4,8g (M.A) - Agent de pH: q.s. pH=6,5 | | |

Chacune des compositions tinctoriales prêtes à l'emploi décrites ci-dessus a été appliquée sur des mèches de cheveux gris naturels à 90 % de blancs pendant 40 minutes à la température de 30°C. Les cheveux ont ensuite été rincés, lavés avec un shampooing standard, puis séchés.

Les cheveux ont été teints dans les nuances figurant dans le tableau ci-après :

| **EXEMPLE** | **Nuance obtenue** |
|---|---|
| **1** | Rouge puissant |
| **2** | Blond très clair cuivré rouge |

Dans les exemples décrits ci-dessus, 1,8% de Rhus vernicifera laccase à 180 unités/mg vendue par la société SIGMA peut être remplacé par 1 % de Pyricularia Orizae à 100 unités/mg vendue par la société I.C.N.

## Revendications

1. Composition prête à l'emploi, pour la teinture d'oxydation des fibres kératiniques et en particulier des fibres kératiniques humaines telles que les cheveux, **caractérisée par le fait qu'**elle comprend, dans un milieu approprié pour la teinture :
- au moins un colorant d'oxydation,
- au moins colorant direct cationique choisi parmi :
a) les composés de formule (V) suivante : 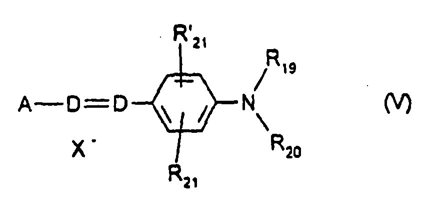 dans laquelle :
D représente un atome d'azote ou le groupement -CH,
R₁₉ et R₂₀, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
R₂₁ et R'₂₁, identiques ou différents, représentent un atome d'hydrogène ou d'halogène choisi parmi le chlore, le brome, l'iode et le fluor, un radical cyano, alcoxy en C₁-C₄ ou acétyloxy,
X ⁻ représente un anion de préférence choisi parmi le chlorure, le méthyl sulfate et l'acétate,
A représente un groupement choisi par les structures A1 à A19 suivantes : 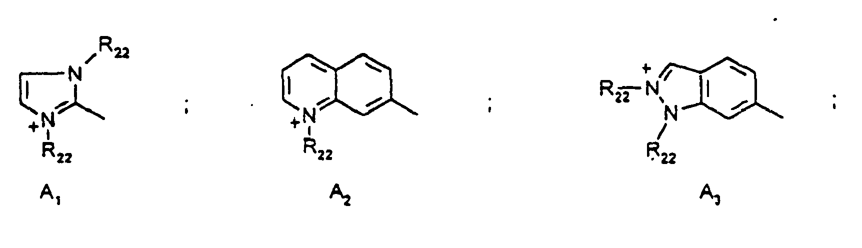 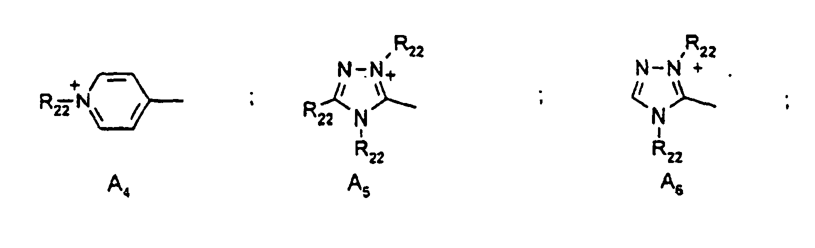 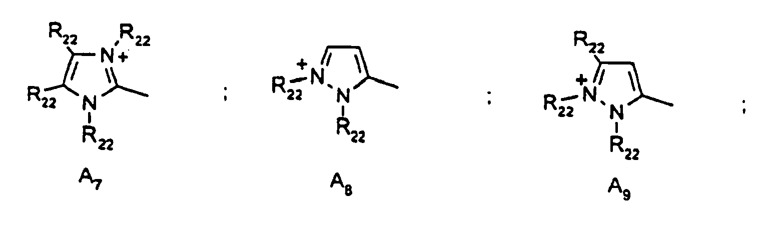 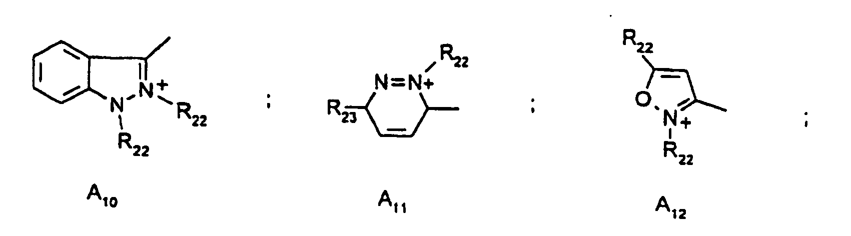 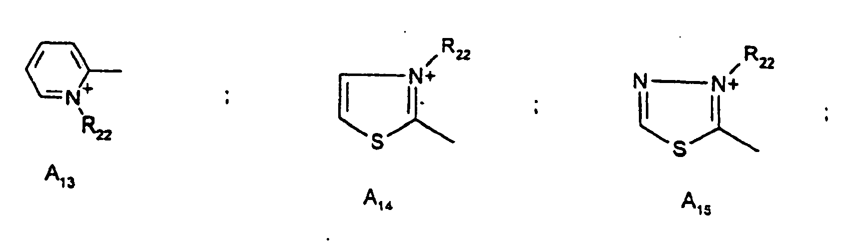 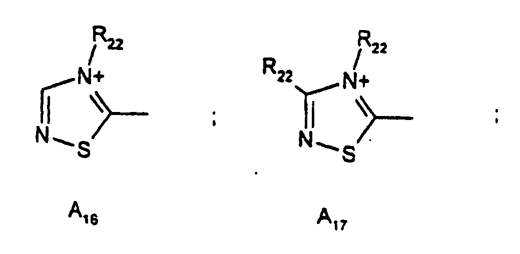 et 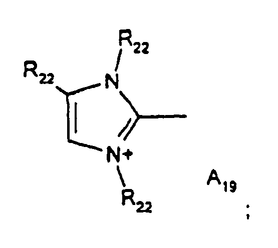 dans lesquelles R₂₂ représente un radical alkyle en C₁-C₄ pouvant être substitué par un radical hydroxyle et R₂₃ représente un radical alcoxy en C₁-C₄;
b) les composés de formule (VI) suivante : 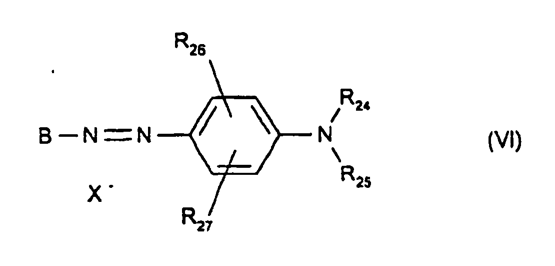 dans laquelle:
R₂₄ représente un atome d'hydrogène ou un radical alkyle en C₁-C₄,
R₂₅ représente un atome d'hydrogène, un radical alkyle pouvant être substitué par un radical -CN ou par un groupement amino, un radical 4'-aminophényle ou forme avec R₂₄ un hétérocycle éventuellement oxygéné et/ou azoté pouvant être substitué par un radical alkyle en C₁-C₄,
R₂₆ et R₂₇, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
X⁻ représente un anion de préférence choisi parmi le chlorure, le méthyl sulfate et l'acétate,
B représente un groupement choisi par les structures B1 à B6 suivantes : 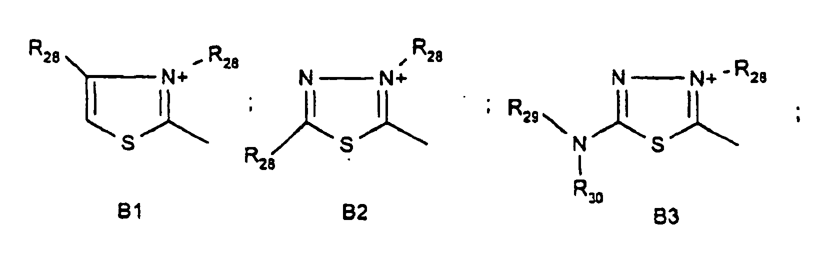 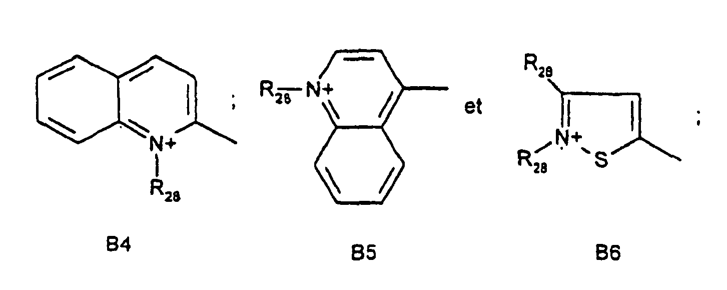 dans lesquelles R₂₈ représente un radical alkyle en C₁-C₄, R₂₉ et R₃₀, identiques ou différents, représentent un atome d'hydrogène ou un radical alkyle en C₁-C₄ ; et
c) les composés de formules (VII) et (VII') suivantes : 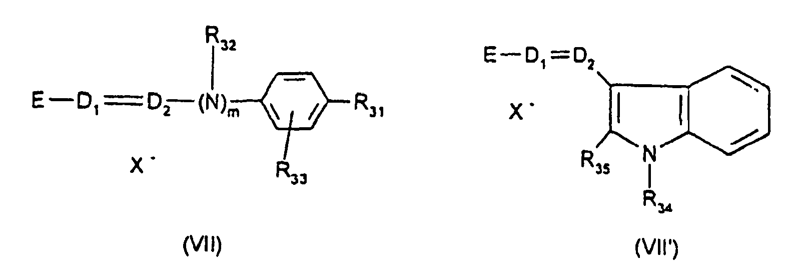 dans lesquelles :
R₃₁ représente un atome d'hydrogène, un radical alcoxy en C₁-C₄, un atome d'halogène tel que le brome, le chlore, l'iode ou le fluor ou un radical amino,
R₃₂ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
R₃₃ représente un atome d'hydrogène ou d'halogène tel que le brome, le chlore, l'iode ou le fluor,
R₃₄ et R₃₅, identiques ou différents, représentent un atome d'hydrogène ou un radical alkyle en C₁-C₄,
D₁ et D₂, identiques ou différents, représentent un atome d'azote ou le groupement -CH,
m=0 ou 1,
étant entendu que lorsque R₃₁ représente un groupement amino non substitué, alors D₁ et D₂ représentent simultanément un groupement -CH et m = 0,
X⁻ représente un anion de préférence choisi parmi le chlorure, le méthyl sulfate et l'acétate,
E représente un groupement choisi par les structures E1 à E8 suivantes 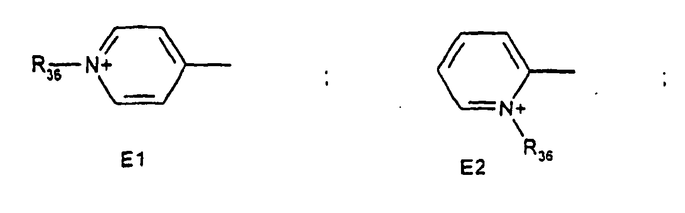 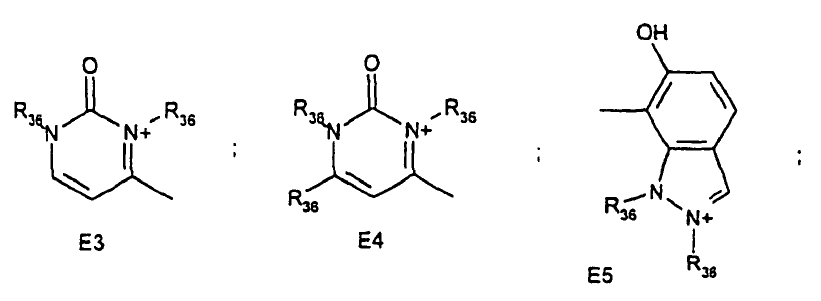 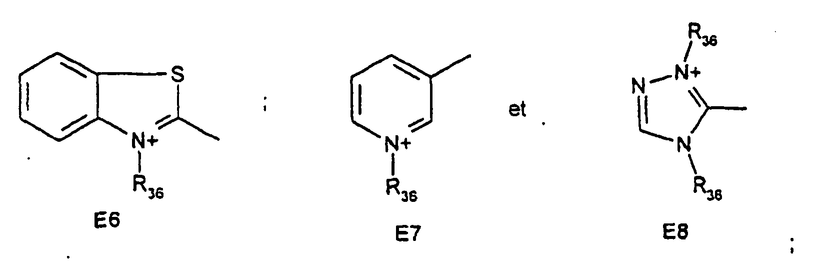 dans lesquelles R₃₆ représente un radical alkyle en C₁-C₄ ;
lorsque m = 0 et que D₁ représente un atome d'azote, alors E peut également désigner un groupement de structure E9 suivante : 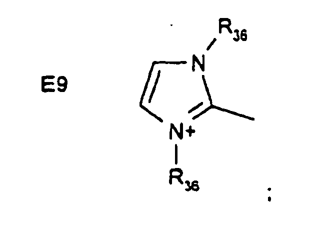 dans laquelle R₃₆ représente un radical alkyle en C₁-C₄, et
- au moins une enzyme de type laccase.

2. Composition selon la revendication 1, **caractérisée par le fait que** la ou les laccases sont choisies parmi les laccases d'origine végétale, d'origine animale, d'origine fongique, d'origine bactérienne, ou obtenues par biotechnologie.

3. Composition selon l'une quelconque des revendications 1 à 2, où les laccases sont choisies parmi celles produites par des végétaux effectuant la synthèse chlorophyllienne.

4. Composition selon la revendication 3, où les laccases sont choisies parmi celles extraites des Anacardiacées ou des Podocarpacées , de Rosmarinus off., de Solanum tuberosum, d'Iris sp., de Coffea sp., de Daucus carrota, de Vinca minor, Persea americana, Catharenthus roseus. Musa sp., Malus pumila, Gingko biloba, Monotropa hypopithys (sucepin), Aesculus sp., Acer pseudoplatanus, Prunus persica, Pistacia palaestina.

5. Composition selon la revendication 2, où les laccases sont choisies parmi celles issues de Pyricularia orizae, de Polyporus versicolor, de Rhizoctonia praticola, de Rhus vernicifera, de Scytalidium, de Polyporus pinsitus, de Myceliophtora thermophila, de Rhizoctonia solani, de Tramates versicolor, de Fomes fomentarius, de Chaetomium thermophile. de Neurospora crassa, de Coriolus versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens, ainsi que leurs variantes.

6. Composition selon l'une quelconque des revendications 1 à 5, **caractérisée par le fait que** la ou les laccases sont présentes dans des quantités allant de 0,5 à 2000 lacu, ou de 1000 à 4.10⁷, ou de 20 à 2.10⁶ unités ulac, pour 100g de composition.

7. Composition selon l'une quelconque des revendications précédentes, **caractérisée par le fait que** le ou les colorants d'oxydation sont choisis parmi les bases d'oxydation et/ou les coupleurs.

8. Composition selon la revendication 7, **caractérisée par le fait que** les bases d'oxydation sont choisies parmi les paraphénylènediamines, les bases doubles, les para-aminophénols, les ortho aminophénols et les bases d'oxydation hétérocycliques.

9. Composition selon la revendication 8, **caractérisée par le fait que** les paraphénylènediamines sont choisies parmi les composés de formule (I) suivante et leurs sels d'addition avec un acide : 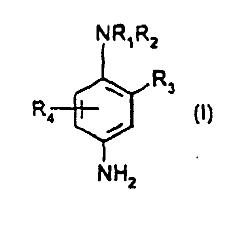 dans laquelle :
-R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
-R₂ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
-R₃ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
-R₄ représente un atome d'hydrogène, d'halogène ou un radical alkyle en C₁-C₄.

10. Composition selon la revendication 9, **caractérisée par le fait que** les paraphénylènediamines de formule (I) sont choisies parmi la paraphénylènediamine, la paratoluylènediamine, la 2-chloro paraphénylènediamine, la 2,3-diméthyl paraphénylènediamine, la 2,6-diméthyl paraphènylènediamine, la 2,6-diéthyl paraphénylènediamine, la 2,5-diméthyl paraphénylènediamine, la N,N-diméthyl paraphényiènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxy-éthyl) amino 2-chloro aniline, la 2-β-hydroxyéthyl paraphénylènediamine, la 2-fluoro paraphénylènediamine, la 2-isopropyl paraphénylènediamine, la N-(β-hydroxy-propyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β, γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine. et leurs sels d'addition avec un acide.

11. Composition selon la revendication 8, **caractérisée par le fait que** les bases doubles sont choisies parmi les composés de formule (II), et leurs sels d'addition avec un acide : 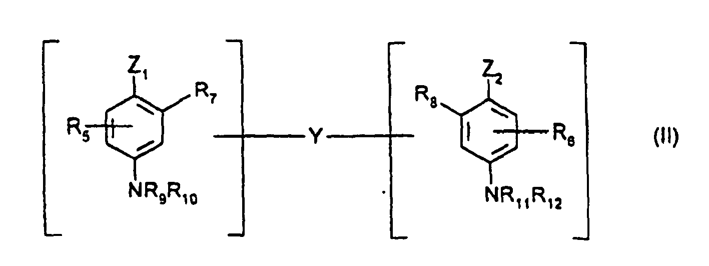 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀ R₁₁ et R₁₂, identiques ou différents, représentent un atome d'hydrogène, un bras de liaison Y ou un radical alkyle en C₁-C₄ ;
étant entendu que les composés de formule (II) ne comportent qu'un seul bras de liaison Y par molécule.

12. Composition selon la revendication 11, **caractérisée par le fait que** les bases doubles de formule (II) sont choisies parmi le N,N'-bis-(β-hydroxyéthyl) N,N'-bis-(4'-aminophényl) 1,3-diamino propanol, la N,N'-bis-(β-hydroxyéthyl) N.N'-bis-(4'-aminophényl) éthylènediamine, la N.N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylène-diamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

13. Composition selon la revendication 8, **caractérisée par le fait que** les para-aminophénols sont choisis parmi les composés de formule (III), et leurs sels d'addition avec un acide : 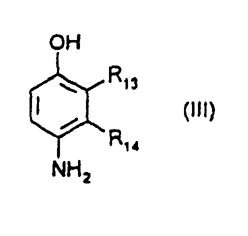 dans laquelle:
- R₁₃ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₁₄ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₃ ou R₁₄ représente un atome d'hydrogène.

14. Composition selon la revendication 13, **caractérisée par le fait que** les para-aminophénols de formule (III) sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

15. Composition selon la revendication 8, **caractérisée par le fait que** les orthoaminophénols sont choisis parmi 2-amino phénol, le 2-amino 5-méthyl phénol, le 2-amino 6-méthyl phénol, le 5-acétamido 2-amino phénol, et leurs sels d'addition avec un acide.

16. Composition selon la revendication 8, **caractérisée par le fait que** les bases d'oxydation hétérocycliques sont choisies parmi les dérivés pyridiniques, les dérivés pyrimidiniques, les dérivés pyrazoliques, les dérivés pyrazolo-pyrimidiniques, et leurs sels d'addition avec un acide.

17. Composition selon l'une quelconque des revendications précédentes, **caractérisée par le fait que** la ou les bases d'oxydation représentent de 0,0005 à 12 % en poids du poids total de la composition tinctoriale.

18. Composition selon la revendication 17, **caractérisée par le fait que** la ou les bases d'oxydation représentent de 0,005 à 6 % en poids du poids total de la composition tinctoriale.

19. Composition selon la revendication 7, **caractérisée par le fait que** le ou les coupleurs sont choisis parmi les méta-phénylènediamines, les méta-aminophénols, les métadiphénols, les coupleurs hétérocycliques, et leurs sels d'addition avec un acide.

20. Composition selon la revendication 19, **caractérisée par le fait que** les coupleurs sont choisis parmi le 2-méthyl-5-amino-phénol, le 5-N-(β-hydroxyéthyl)-amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2,4-diamino-1-(β-hydroxyéthyloxy)-benzène, le 2-amino-4-(β-hydroxyéthylamino)-1-méthoxy-benzène, le 1,3-diamino-benzène, le 1,3-bis-(2,4-diaminophénoxy)-propane, le sésamol. I'α-naphtol, le 6-hydroxy-indole, le 4-hydroxy-indole. le 4-hydroxy-N-méthyl-indole, la 6-hydroxy-indoline, la 2,6-dihydroxy-4-méthyl-pyridine. le 1-H-3-méthyl-pyrazole-5-one, le 1-phényl-3-méthyl-pyrazole-5-one, le 2,6-diméthyl-pyrazolo-[1,5-b]-1,2,4-triazole, le 2,6-diméthyl-[3,2-c]-1,2,4-triazole, le 6-méthyl-pyrazolo-[1,5-a]-benzimidazole, et leurs sels d'addition avec un acide.

21. Composition selon l'une quelconque des revendications précédentes, **caractérisée par le fait que** le ou les coupleurs représentent de 0,0001 à 10 % en poids du poids total de la composition tinctoriale.

22. Composition selon la revendication 21, **caractérisée par le fait que** le ou les coupleurs représentent de 0,005 à 5 % en poids du poids total de la composition tinctoriale.

23. Composition selon l'une quelconque des revendications précédentes, **caractérisée par le fait que** les colorants directs cationiques de formule (V) sont choisis parmi les composés répondant aux structures (V1) à (V52) suivantes : 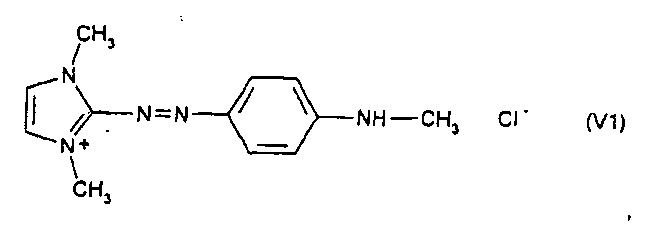 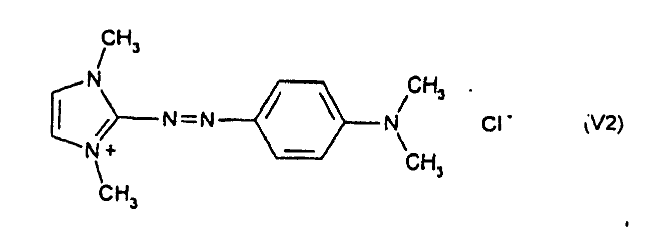 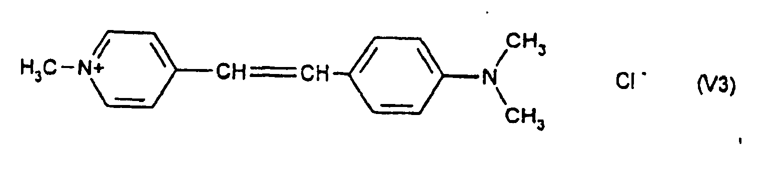 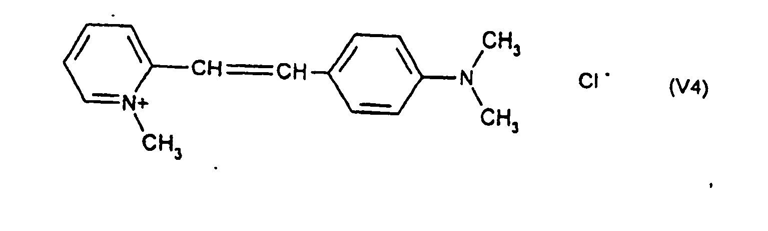 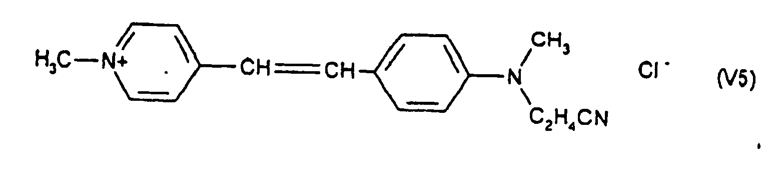 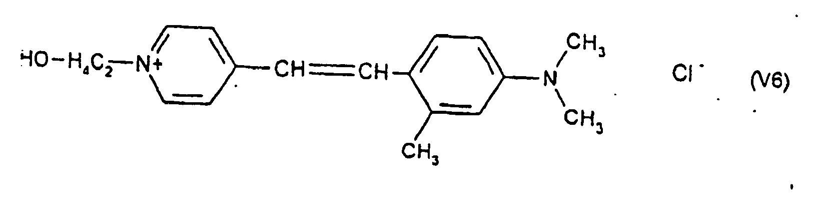 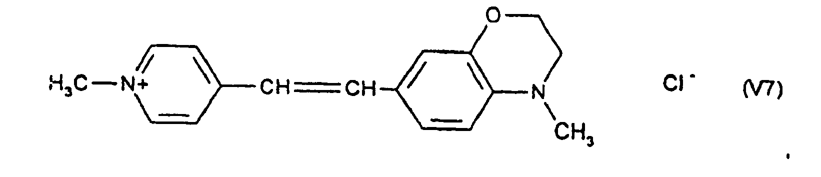 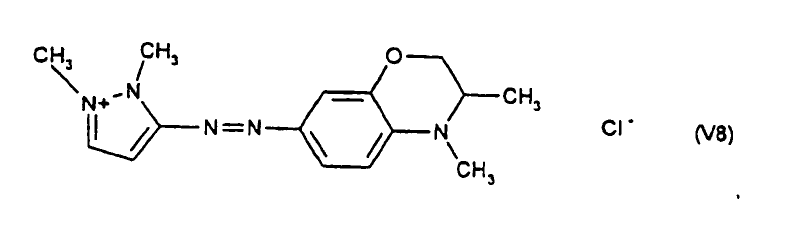 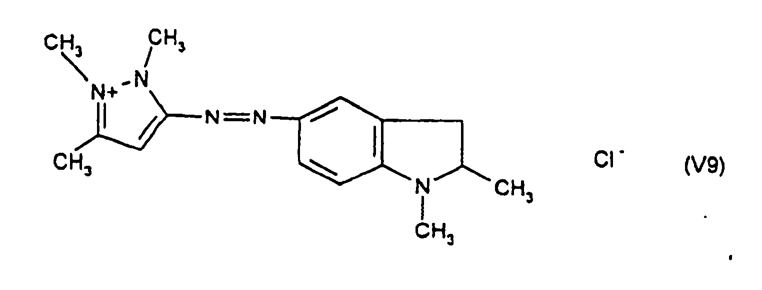 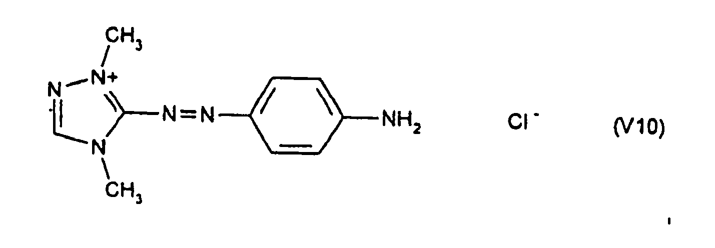 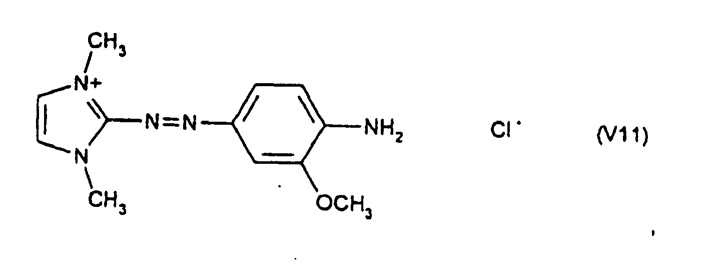 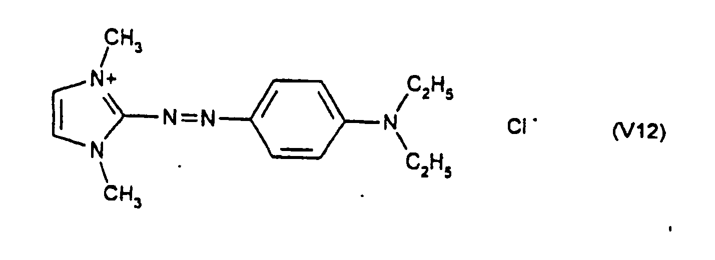 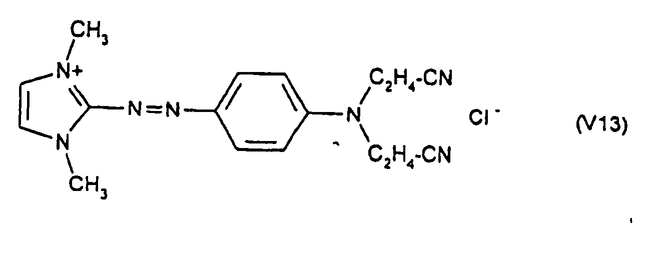 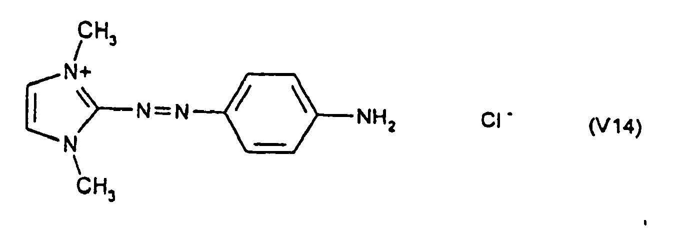 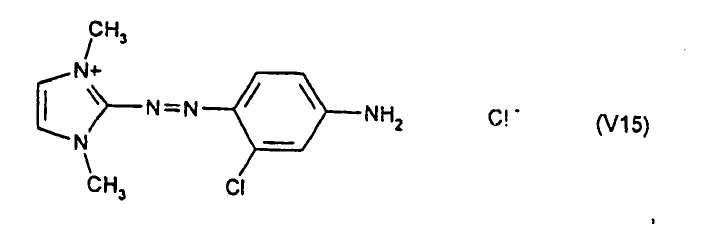 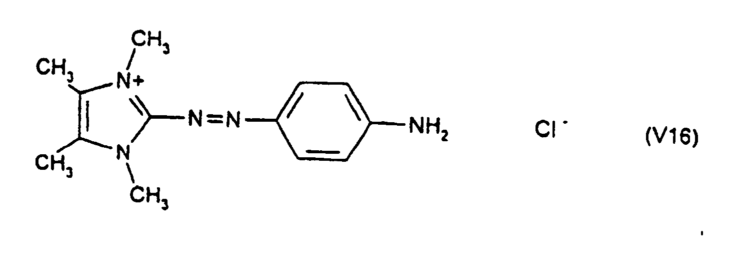 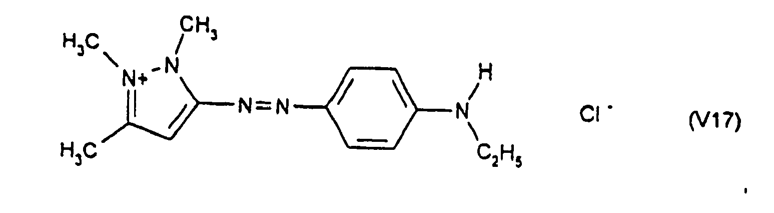 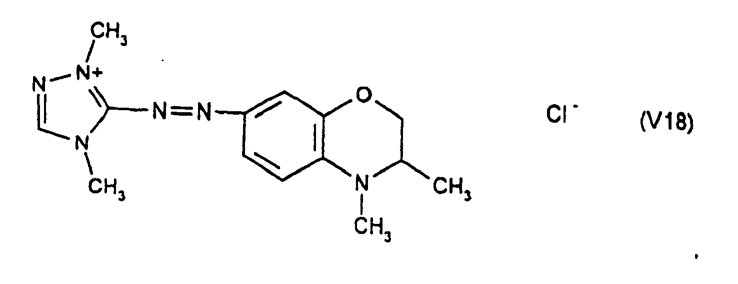 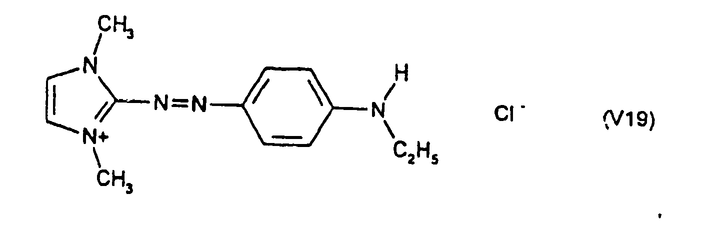 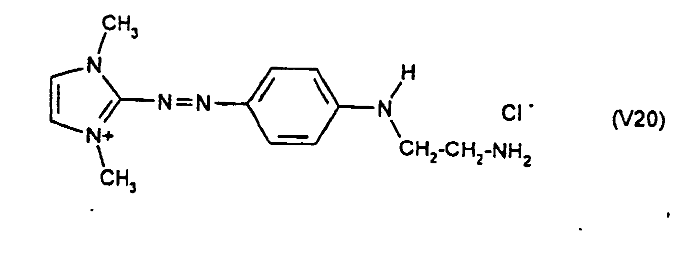 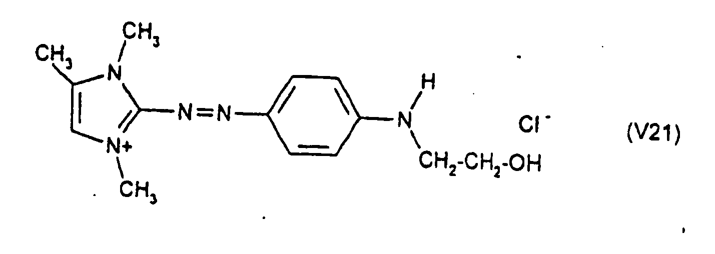 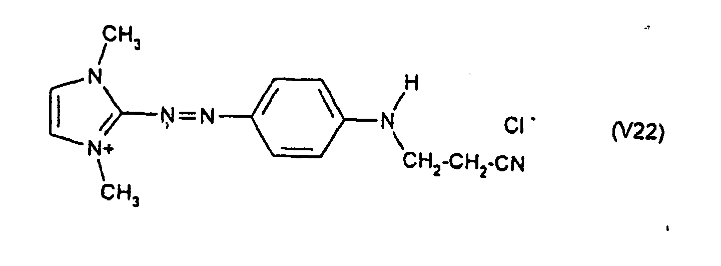 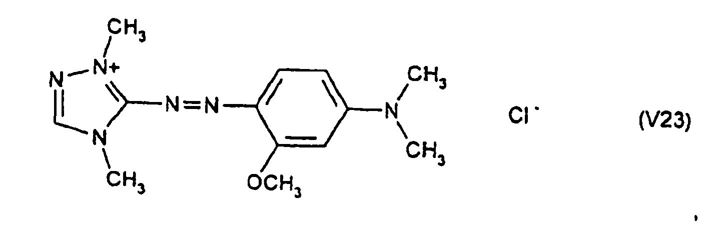 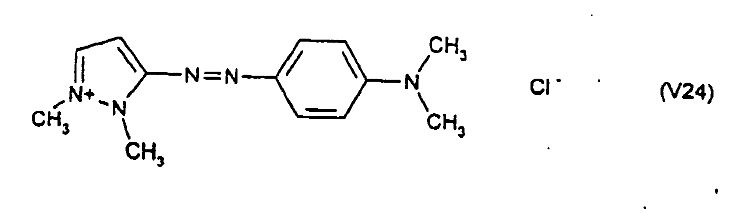 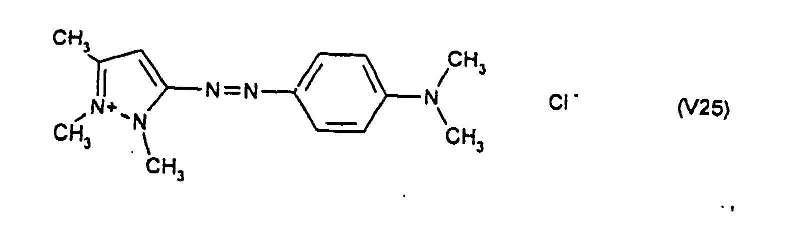 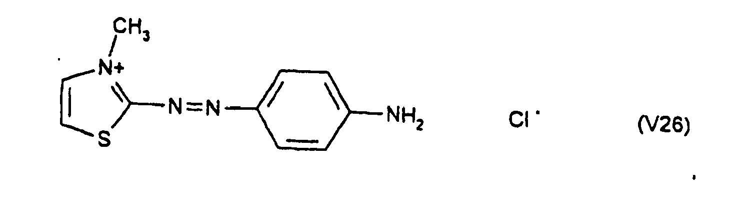 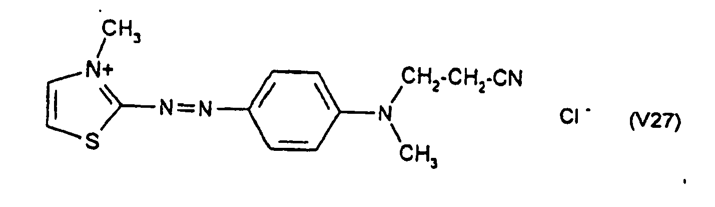 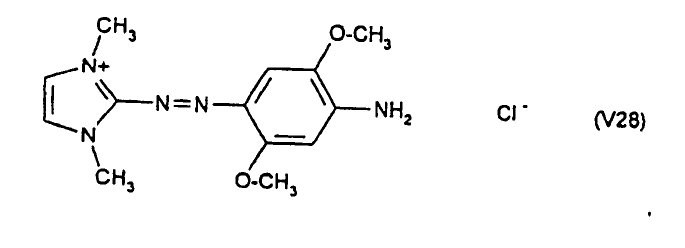 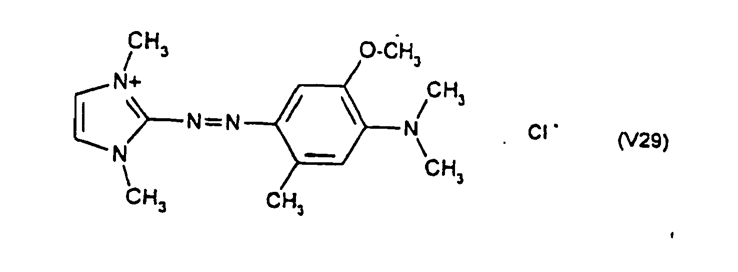 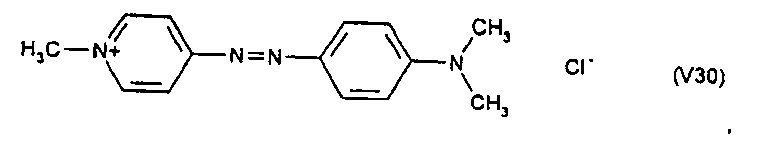 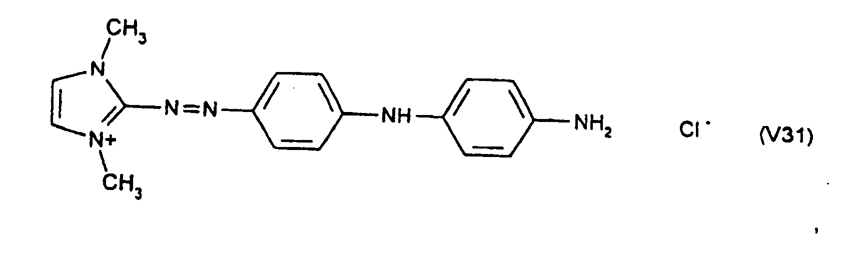 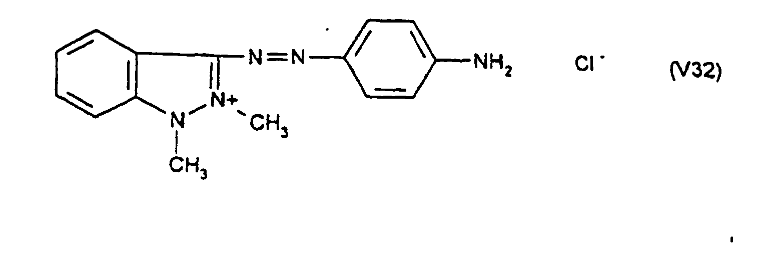 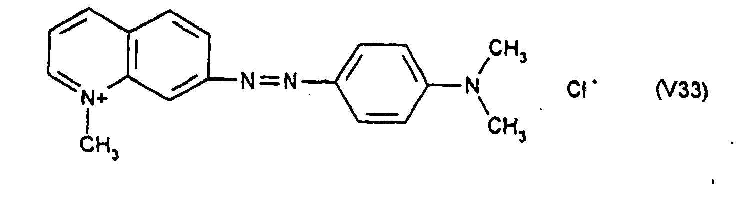 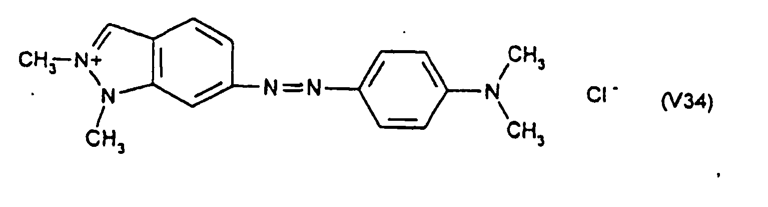 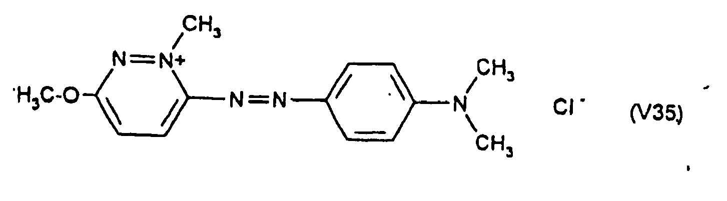 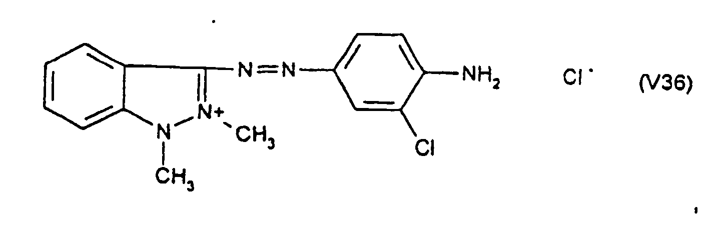 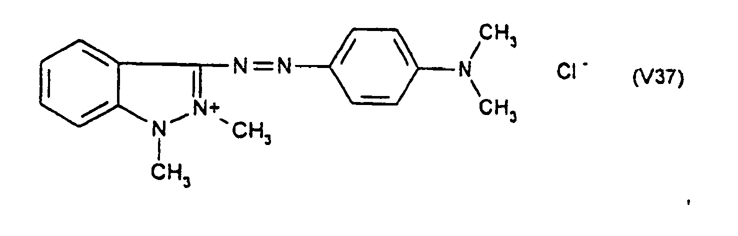 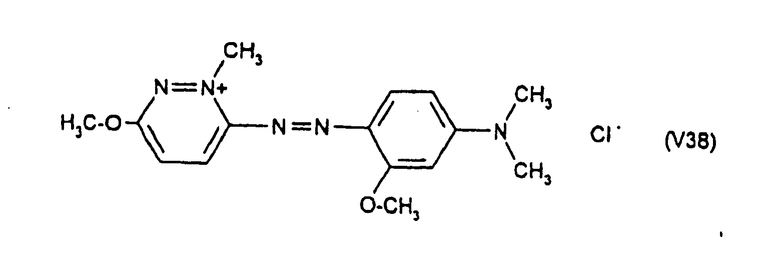 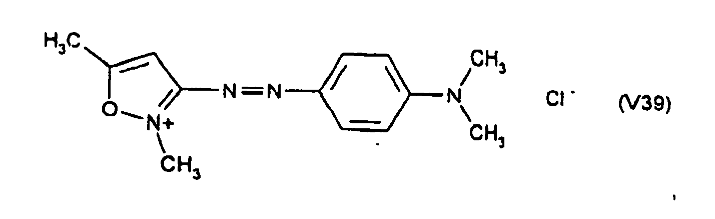 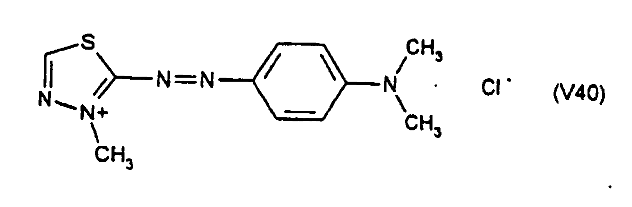 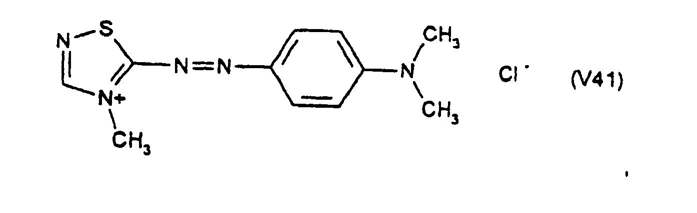 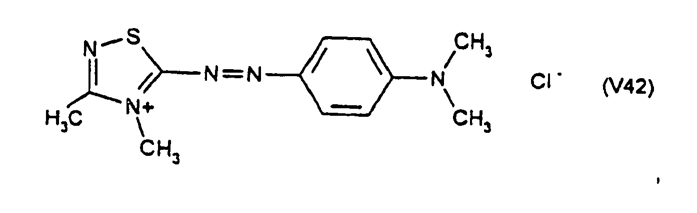 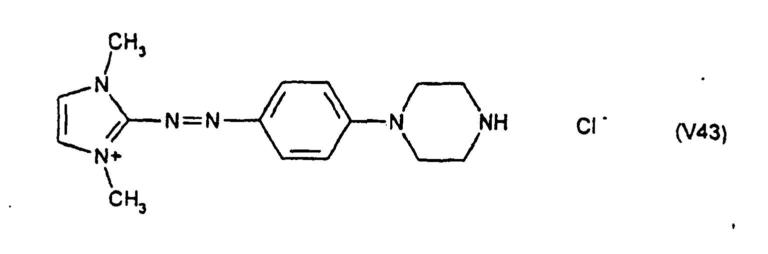 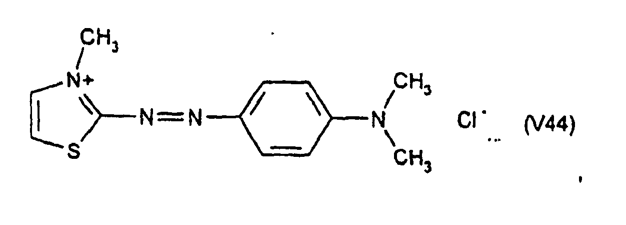 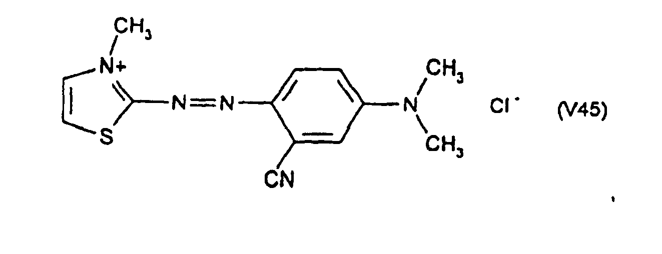 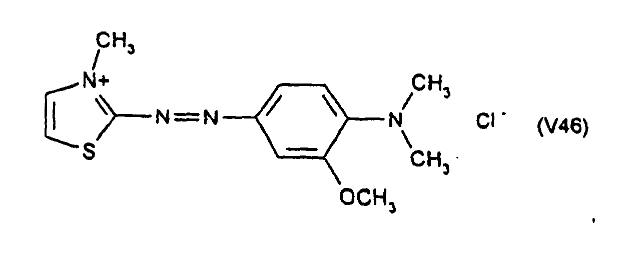 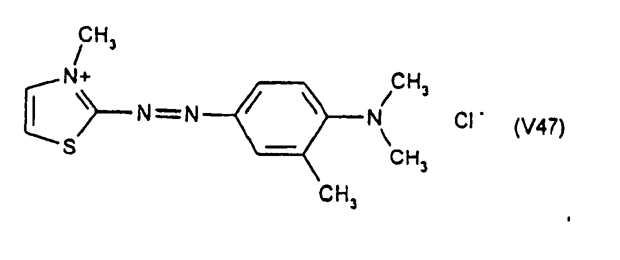 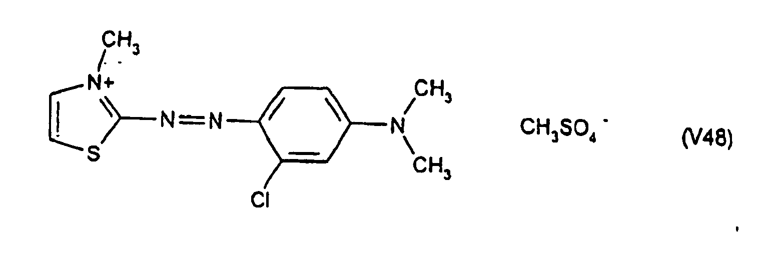 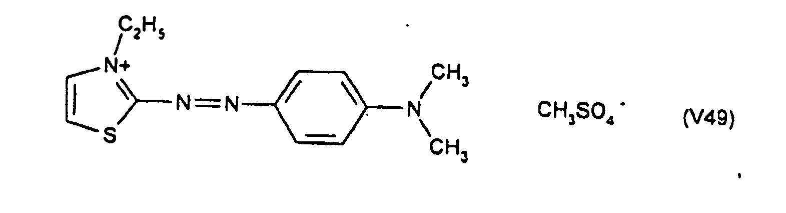 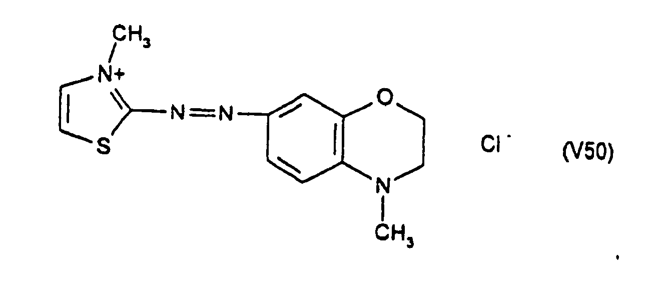 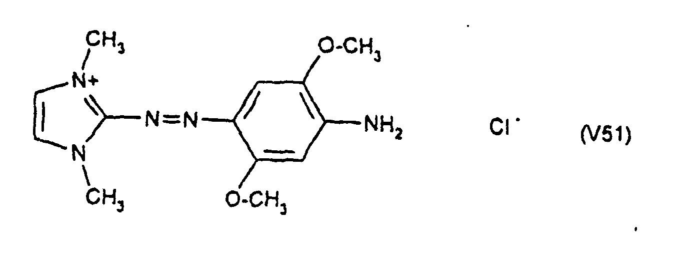 et

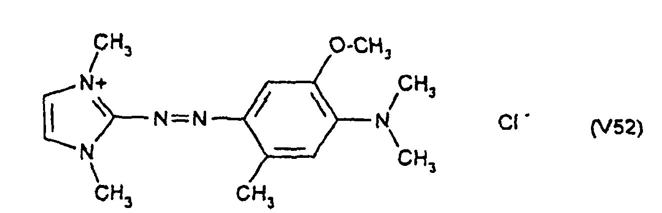

24. Composition selon l'une quelconque des revendications précédentes, **caractérisée par le fait que** les colorants directs cationiques de formule (VI) sont choisis parmi les composés répondant aux structures (VI1) à (VI12) suivantes : 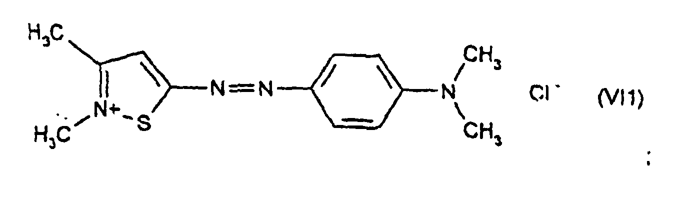 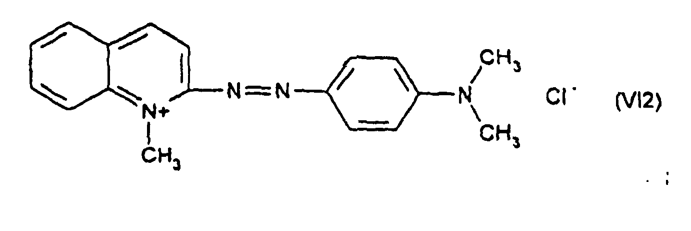 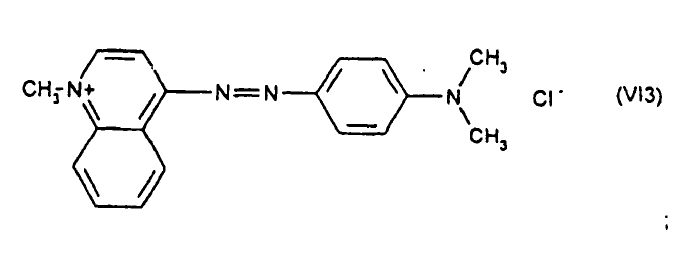 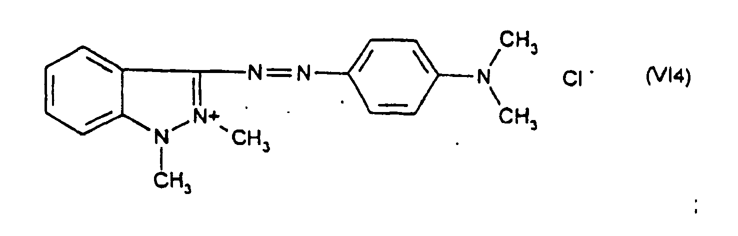 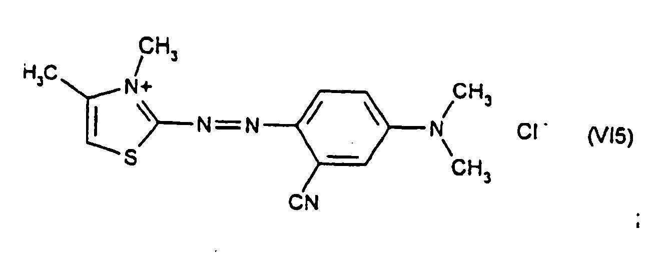 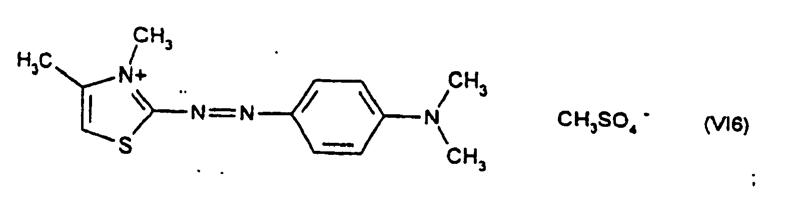 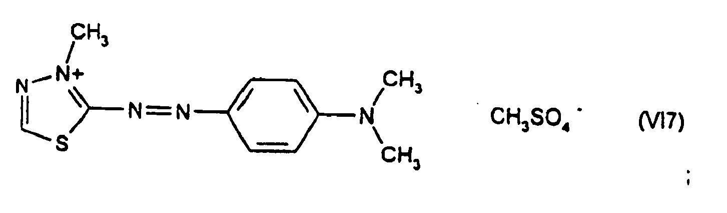 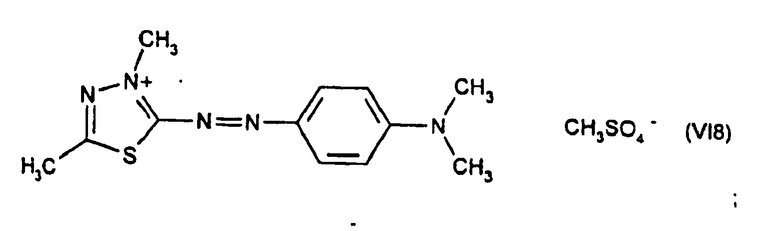 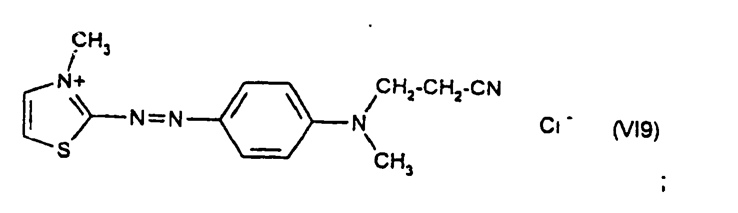 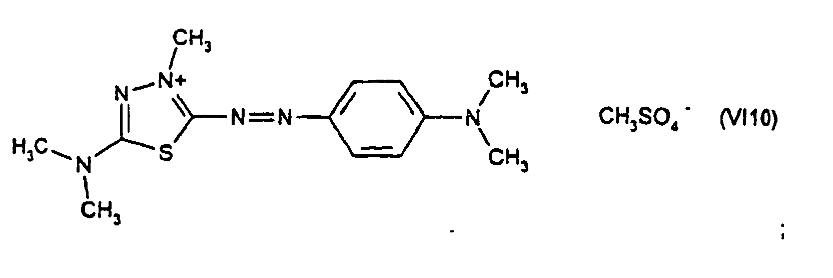 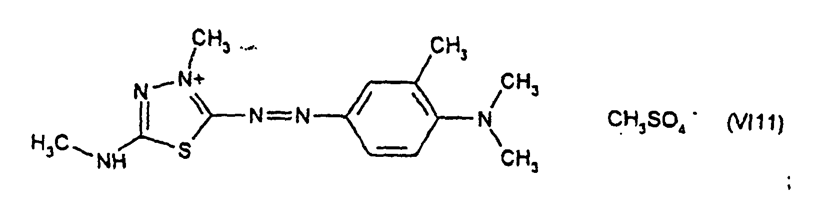 et

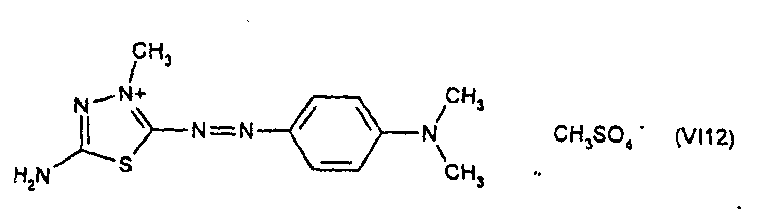

25. Composition selon l'une quelconque des revendications précédentes, **caractérisée par le fait que** les colorants directs cationiques de formule (VII) sont choisis parmi les composés répondant aux structures (VII1) à (VII18) suivantes : 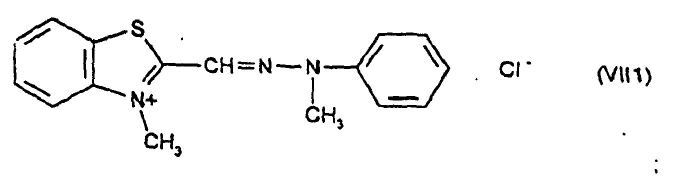 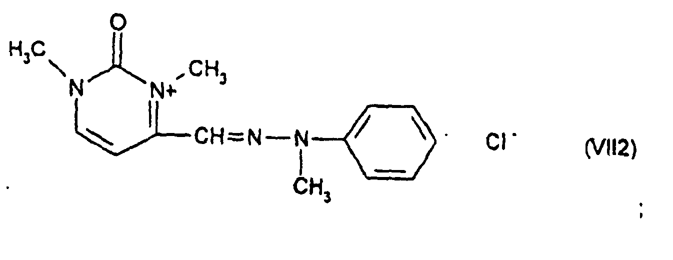 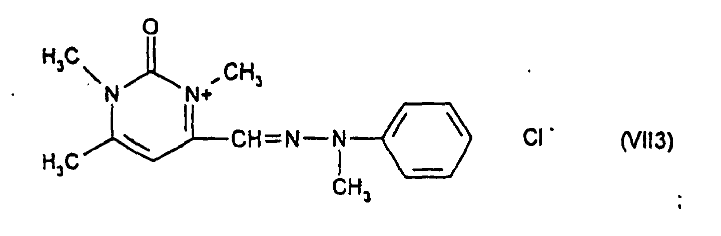 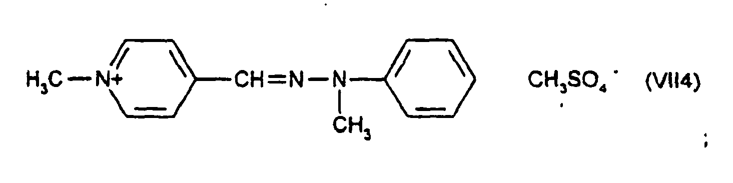 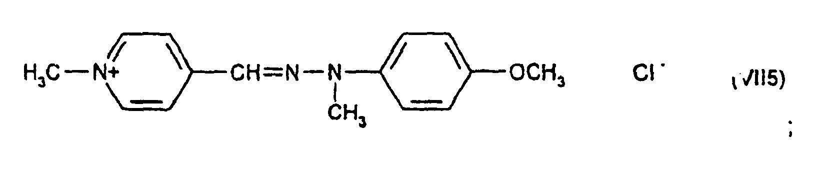 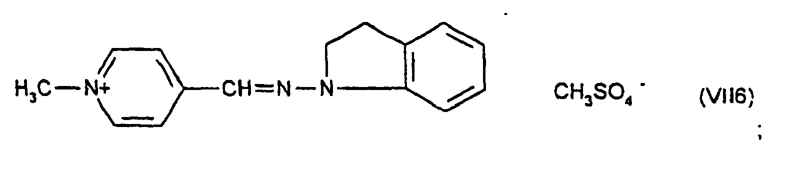 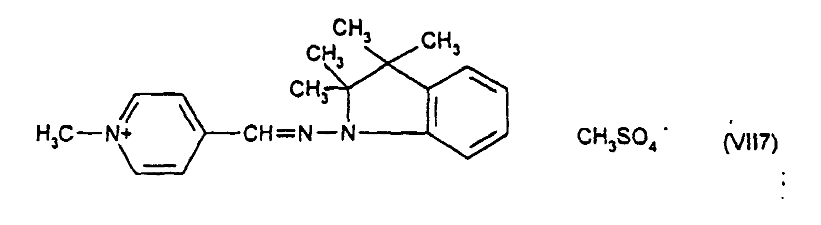 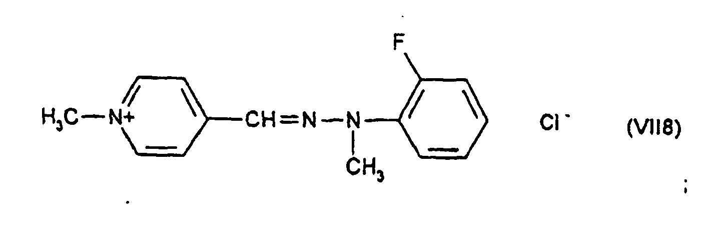 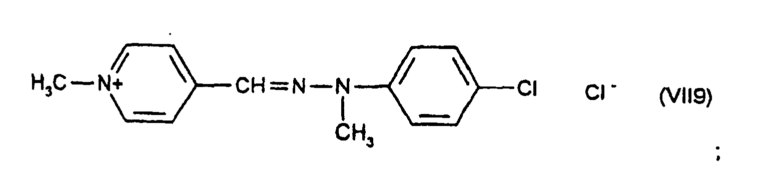 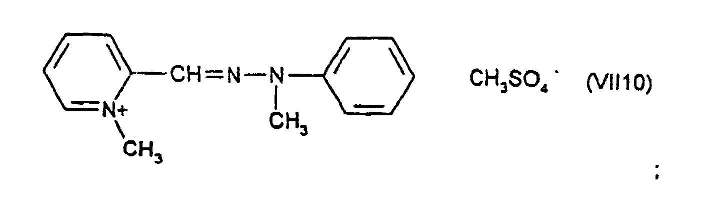 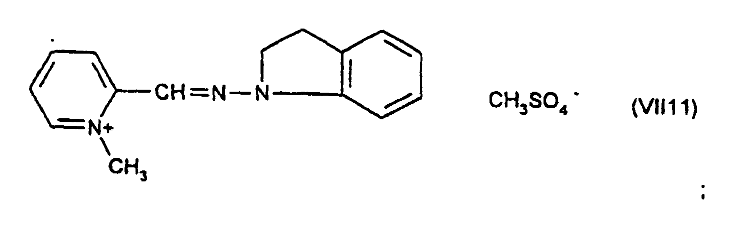 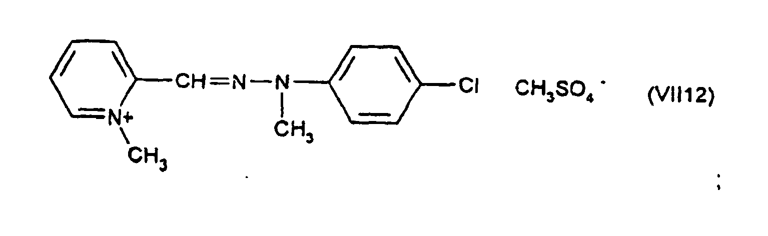 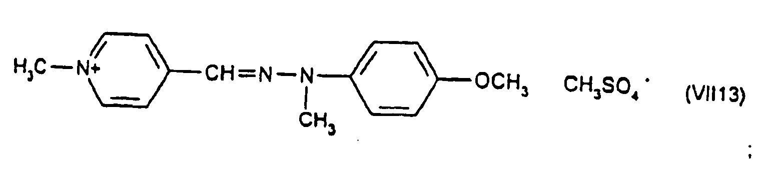 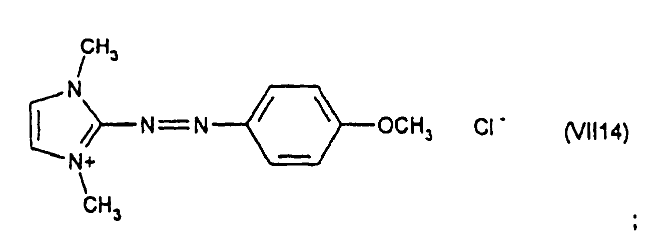 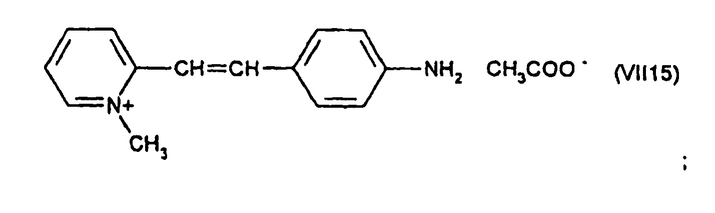 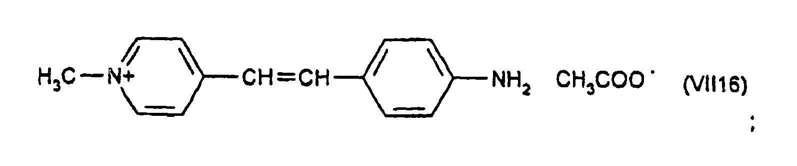 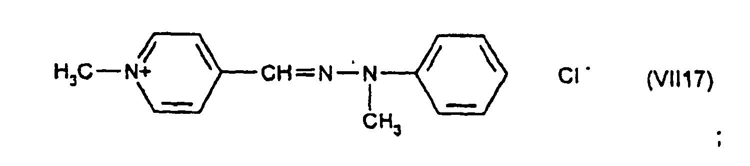 et

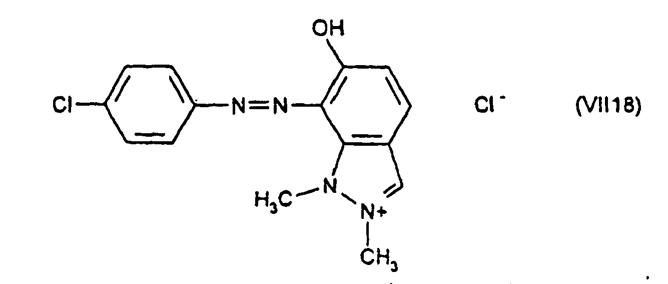

26. Composition selon l'une quelconque des revendications précédentes, **caractérisée par le fait que** les colorants directs cationiques de formule (VII') sont choisis parmi les composés répondant aux structures (VII'1) à (VII'3) suivantes : 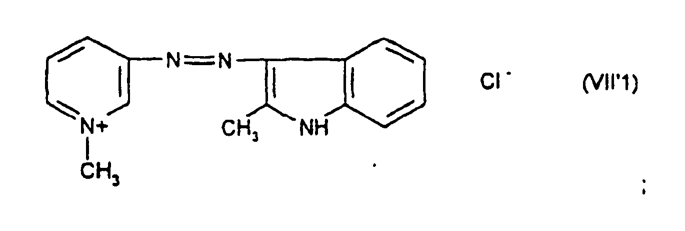 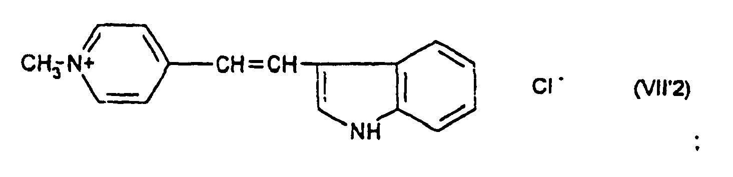 et

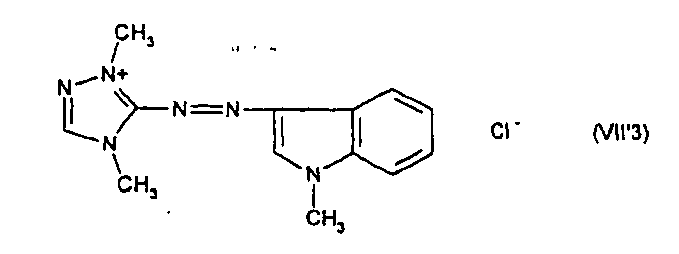

27. Composition selon l'une quelconque des revendications précédentes, **caractérisée par le fait que** le ou les colorants directs cationiques représentent de 0,001 à 10 % en poids du poids total de la composition tinctoriale prête à l'emploi.

28. Composition selon la revendication 27, **caractérisée par le fait que** le ou les colorants directs cationiques représentent de 0,05 à 5 % en poids du poids total de la composition tinctoriale prête à l'emploi.

29. Composition selon l'une quelconque des revendications précédentes, **caractérisée par le fait que** les sels d'addition avec un acide sont choisis parmi les chlorhydrates, les bromhydrates, les sulfates et les tartrates, les lactates et les acétates.

30. Composition selon l'une quelconque des revendications précédentes, **caractérisée par le fait que** le milieu approprié pour la teinture est constitué par de l'eau ou par un mélange d'eau et d'au moins un solvant organique.

31. Composition selon l'une quelconque des revendications précédentes, **caractérisée par le fait qu'**elle présente un pH compris 4 et 11.

32. Procédé de teinture des fibres kératiniques et en particulier des fibres kératiniques humaines telles que les cheveux, **caractérisé par le fait qu'**on applique sur lesdites fibres au moins une composition tinctoriale prête à l'emploi telle que définie dans l'une quelconque des revendications précédentes, pendant un temps suffisant pour développer la coloration désirée.

33. Procédé selon la revendication 32, **caractérisé par le fait qu'**il comporte une étape préliminaire consistant à stocker sous forme séparée, d'une part, une composition (A) comprenant, dans un milieu approprié pour la teinture au moins un colorant d'oxydation, au moins un colorant direct cationique et, d'autre part, une composition (B) renfermant, dans un milieu approprié pour la teinture, au moins une enzyme de type laccase, puis à procéder à leur mélange au moment de l'emploi avant d'appliquer ce mélange sur les fibres kératiniques.

34. Dispositif à plusieurs compartiments ou "kit" de teinture, **caractérisé par le fait qu'**il comporte un premier compartiment renfermant la composition (A) telle que définie dans la revendication 33 et un second compartiment renfermant la composition (B) telle que définie dans la revendication 33.

## Claims

1. Ready-to-use composition for the oxidation dyeing of keratinous fibres, and in particular human keratinous fibres such as hair, **characterized in that** it comprises, in a medium appropriate for dyeing:
- at least one oxidation dye,
- at least one cationic direct dye chosen from:
a) the compounds of the following formula (V): 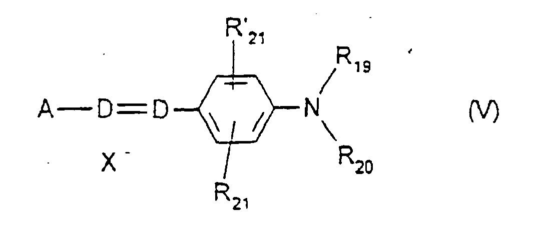 in which:
D represents a nitrogen atom or the -CH group,
R₁₉ and R₂₀, which are identical or different, represent a hydrogen atom; a C₁-C₄ alkyl radical which may be substituted with a -CN, -OH or -NH₂ radical or form with a carbon atom of the benzene ring an optionally oxygen-containing or nitrogen-containing heterocycle which may be substituted with one or more C₁-C₄ alkyl radicals; a 4'-aminophenyl radical,
R₂₁ and R'₂₁, which are identical or different, represent a hydrogen or halogen atom chosen from chlorine, bromine, iodine and fluorine, a cyano, C₁-C₄ alkoxy or acetyloxy radical,
X⁻ represents an anion preferably chosen from chloride, methyl sulphate and acetate,
A represents a group chosen from the following structures A1 to A19: 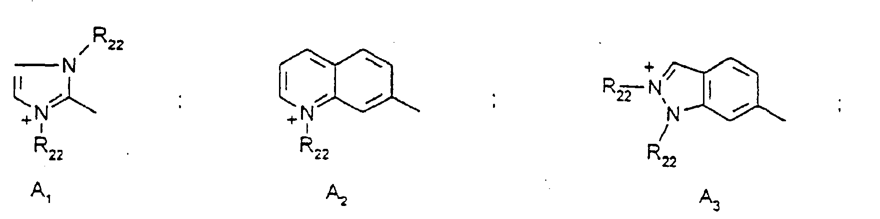 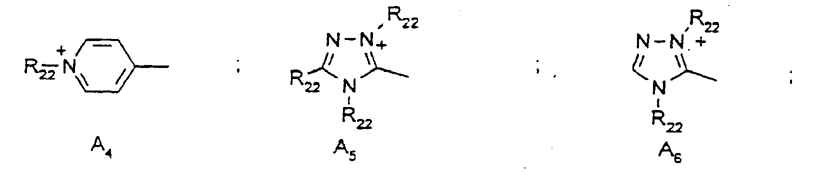 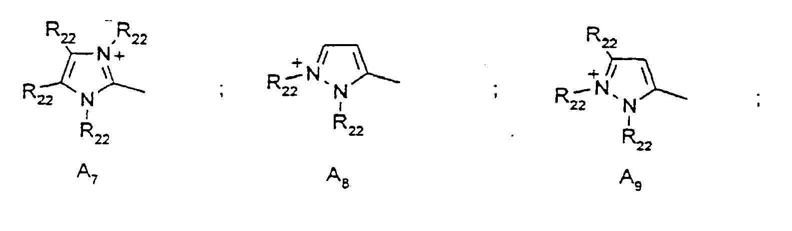 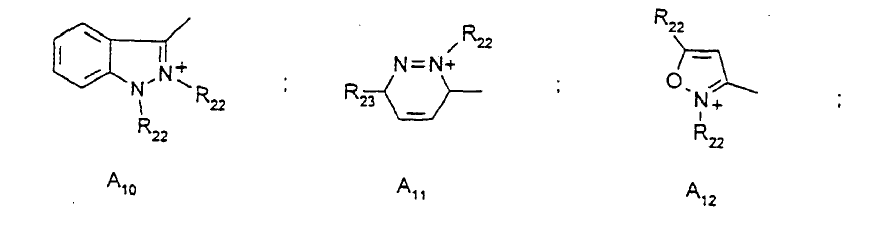 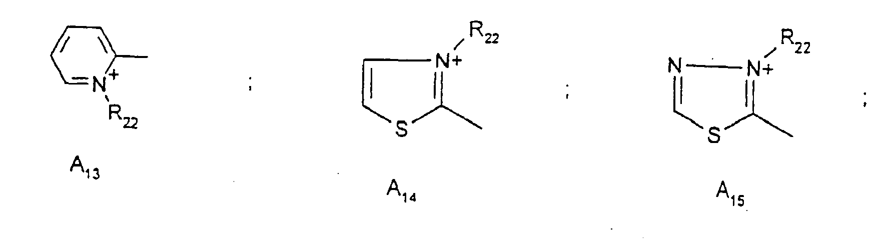 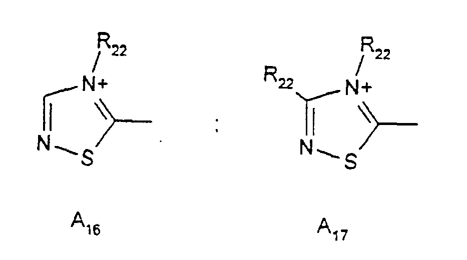 and 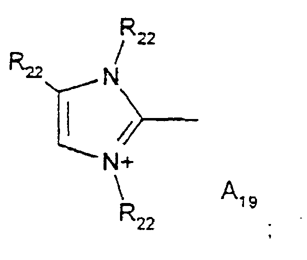
in which R₂₂ represents a C₁-C₄ alkyl radical which may be substituted with a hydroxyl radical and R₂₃ represents a C₁-C₄ alkoxy radical;
b) the compounds of the following formula (VI): 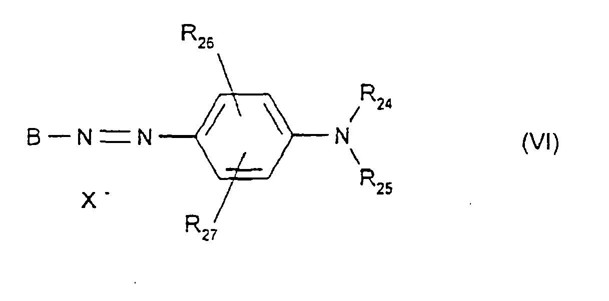 in which:
R₂₄ represents a hydrogen atom or a C₁-C₄ alkyl radical,
R₂₅ represents a hydrogen atom, an alkyl radical which may be substituted with a -CN radical or with an amino group, a 4'-aminophenyl radical or forms with R₂₄ an optionally oxygen-containing and/or nitrogen-containing heterocycle which may be substituted with a C₁-C₄ alkyl radical,
R₂₆ and R₂₇, which are identical or different, represent a hydrogen atom, a halogen atom such as bromine, chlorine, iodine or fluorine, a C₁-C₄ alkyl or C₁-C₄ alkoxy radical, a -CN radical,
X⁻ represents an anion preferably chosen from chloride, methyl sulphate and acetate,
B represents a group chosen from the following structures B1 to B6: 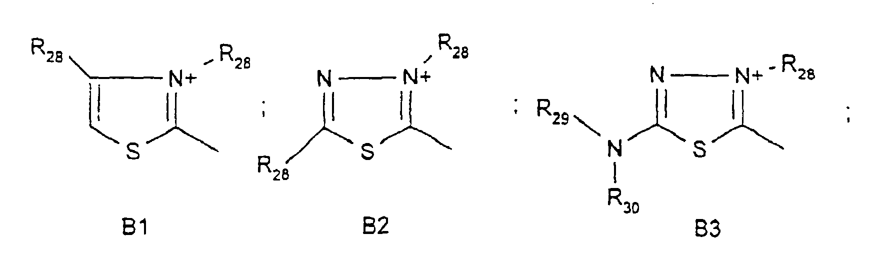 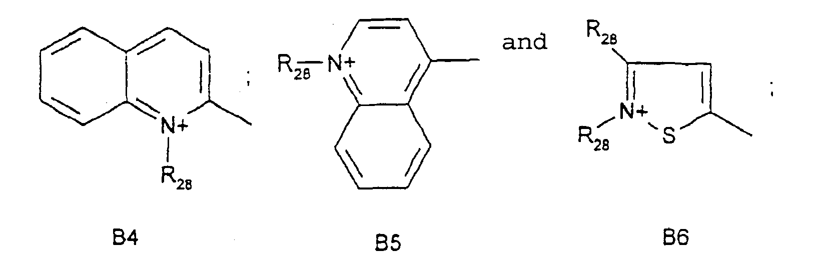
in which R₂₈ represents a C₁-C₄ alkyl radical, and R₂₉ and R₃₀, which are identical or different, represent a hydrogen atom or a C₁-C₄ alkyl radical; and
c) the compounds of the following formulae (VII) and (VII'): 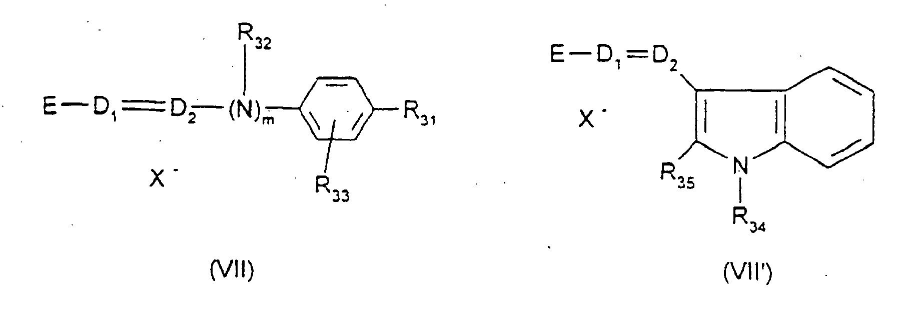 in which:
R₃₁ represents a hydrogen atom, a C₁-C₄ alkoxy radical, a halogen atom such as bromine, chlorine, iodine or fluorine or an amino radical,
R₃₂ represents a hydrogen atom, a C₁-C₄ alkyl radical or forms with a carbon atom of the benzene ring a heterocycle optionally containing oxygen and/or substituted with one or more C₁-C₄ alkyl groups,
R₃₃ represents a hydrogen atom or a halogen atom such as bromine, chlorine, iodine or fluorine,
R₃₄ and R₃₅, which are identical or different, represent a hydrogen atom or a C₁-C₄ alkyl radical,
D₁ and D₂, which are identical or different, represent a nitrogen atom or the -CH group,
m = 0 or 1,
it being understood that when R₃₁ represents an unsubstituted amino group, then D₁ and D₂ simultaneously represent a -CH group and m = 0,
X⁻ represents an anion preferably chosen from chloride, methyl sulphate and acetate,
E represents a group chosen from the following structures E1 to E8: 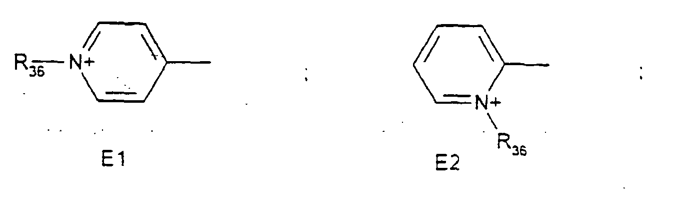 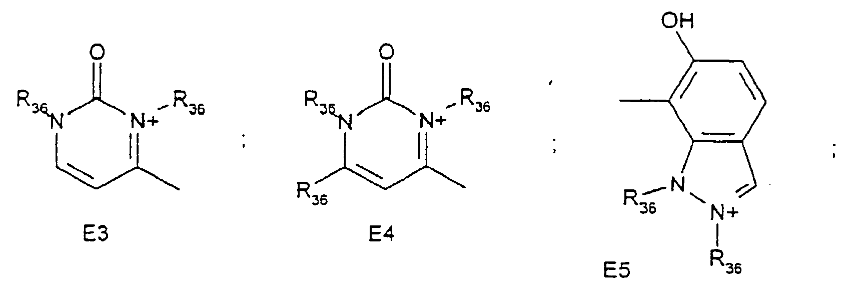 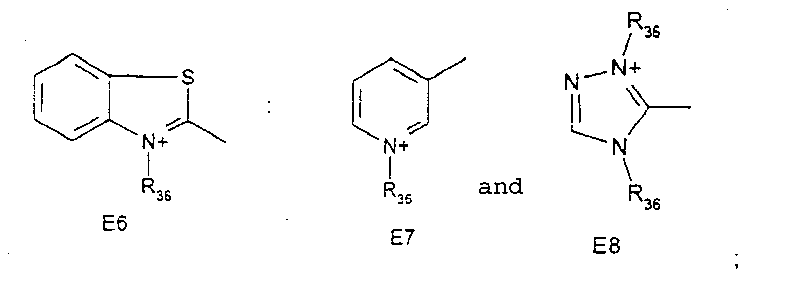
in which R₃₆ represents a C₁-C₄ alkyl radical;
when m = 0 and D₁ represents a nitrogen atom, then E may also denote a group of the following structure E9: 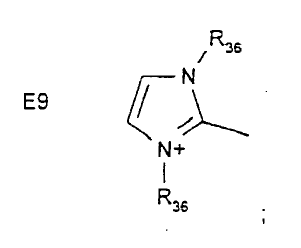 in which R₃₆ represents a C₁-C₄ alkyl radical, and
- at least one enzyme of the laccase type.

2. Composition according to Claim 1, **characterized in that** the laccase(s) are chosen from laccases of plant origin, animal origin, fungal origin, bacterial origin or are obtained by biotechnology.

3. Composition according to either of Claims 1 and 2, where the laccases are chosen from those produced by plants performing chlorophyll synthesis.

4. Composition according to Claim 3, where the laccases are chosen from those extracted from Anacardiaceae or Podocarpaceae, Rosmarinus off., Solanum tuberosum, Iris sp., Coffea sp., Daucus carrota, Vinca minor, Persea americana, Catharenthus roseus, Musa sp., Malus pumila, Gingko biloba, Monotropa hypopithys (Indian pipe), Aesculus sp., Acer pseudoplatanus, Prunus persica, Pistacia palaestina.

5. Composition according to Claim 2, where the laccases are chosen from those derived from Pyricularia orizae, Polyporus versicolor, Rhizoctonia praticola, Rhus vernicifera, Scytalidium, Polyporus pinsitus, Myceliophtora thermophila, Rhizoctonia solani, Tramates versicolor, Fomes fomentarius, Chaetomium thermophile, Neurospora crassa, Coriolus versicol, Botrytis cinerea, Rigidoporus lignosus, Phellinus noxius, Pleurotus ostreatus, Aspergillus nidulans, Podospora anserina, Agaricus bisporus, Ganoderma lucidum, Glomerella cingulata, Lactarius piperatus, Russula delica, Heterobasidion annosum, Thelephora terrestris, Cladosporium cladosporioides, Cerrena unicolor, Coriolus hirsutus, Ceriporiopsis subvermispora, Coprinus cinereus, Panaeolus papilionaceus, Panaeolus sphinctrinus, Schizophyllum commune, Dichomitius squalens and variants thereof.

6. Composition according to any one of Claims 1 to 5, **characterized in that** the laccase(s) are present in quantities ranging from 0.5 to 2000 lacu, or from 1000 to 4×10⁷, or from 20 to 2×10⁶ lacu units, per 100 g of composition.

7. Composition according to any one of the preceding claims, **characterized in that** the oxidation dye(s) are chosen from oxidation bases and/or couplers.

8. Composition according to Claim 7, **characterized in that** the oxidation bases are chosen from para-phenylenediamines, double bases, para-aminophenols, ortho-aminophenols and heterocyclic oxidation bases.

9. Composition according to Claim 8, **characterized in that** the para-phenylenediamines are chosen from the compounds of the following formula (I) and their addition salts with an acid: 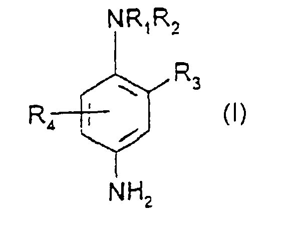 in which:
- R₁ represents a hydrogen atom, a C₁-C₄ alkyl radical, a monohydroxy(C₁-C₄ alkyl) radical, a polyhydroxy-(C₂-C₄ alkyl) radical, a (C₁-C₄) alkoxy (C₁-C₄) alkyl radical, a C₁-C₄ alkyl radical substituted with a nitrogen-containing group, a phenyl radical or a 4'-aminophenyl radical;
- R₂ represents a hydrogen atom, a C₁-C₄ alkyl radical, a monohydroxy (C₁-C₄ alkyl) radical, a polyhydroxy-(C₂-C₄ alkyl) radical, a (C₁-C₄) alkoxy (C₁-C₄) alkyl radical or a C₁-C₄ alkyl radical substituted with a nitrogen-containing group;
- R₃ represents a hydrogen atom, a halogen atom such as a chlorine, bromine, iodine or fluorine atom, a C₁-C₄ alkyl radical, a monohydroxy(C₁-C₄ alkyl) radical, a hydroxy(C₁-C₄ alkoxy) radical, an acetylamino(C₁-C₄ alkoxy) radical, a mesylamino(C₁-C₄ alkoxy) radical or a carbamoylamino(C₁-C₄ alkoxy) radical,
- R₄ represents a hydrogen or halogen atom or a C₁-C₄ alkyl radical.

10. Composition according to Claim 9, **characterized in that** the para-phenylenediamines of formula (I) are chosen from para-phenylenediamine, para-tolylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-N,N-bis(β-hydroxyethyl)amino-2-methylaniline, 4-N,N-bis(β-hydroxyethyl)amino-2-chloroaniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N,N-(ethyl-β-hydroxyethyl)-para-phenylenediamine, N-(β,γ-dihydroxypropyl)-para-phenylenediamine, N- (4' - aminophenyl)-para-phenylenediamine, N-phenyl-para-phenylenediamine, 2-β-hydroxyethyloxy-para-phenylenediamine, 2-β-acetylaminoethyloxy-para-phenylenediamine, N-(β-methoxyethyl)-para-phenylenediamine, and their addition salts with an acid.

11. Composition according to Claim 8, **characterized in that** the double bases are chosen from the compounds of formula (II), and their addition salts with an acid: 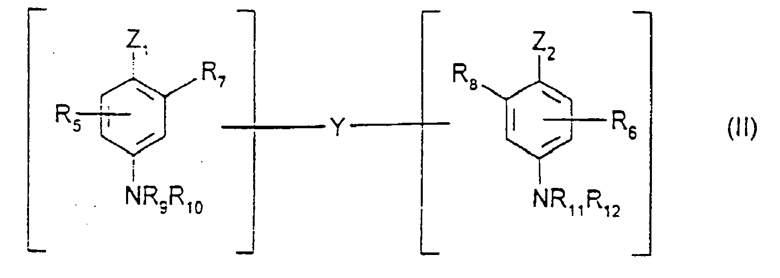 in which:
- Z₁ and Z₂, which are identical or different, represent a hydroxyl or -NH₂ radical which may be substituted with a C₁-C₄ alkyl radical or with a linking arm Y;
- the linking arm Y represents a linear or branched alkylene chain comprising from 1 to 14 carbon atoms, which may be interrupted by or which may end with one or more nitrogen-containing groups and/or one or more heteroatoms such as oxygen, sulphur or nitrogen atoms, and optionally substituted with one or more hydroxyl or C₁-C₆ alkoxy radicals;
- R₅ and R₆ represent a hydrogen or halogen atom, a C₁-C₄ alkyl radical, a monohydroxy(C₁-C₄ alkyl) radical, a polyhydroxy (C₂-C₄ alkyl) radical, an amino (C₁-C₄ alkyl) radical or a linking arm Y;
- R₇, R₈, R₉, R₁₀, R₁₁ and R₁₂, which are identical or different, represent a hydrogen atom, a linking arm Y or a C₁-C₄ alkyl radical;
it being understood that the compounds of formula (II) contain only one linking arm Y per molecule.

12. Composition according to Claim 11, **characterized in that** the double bases of formula (II) are chosen from N,N'-bis(β-hydroxyethyl)-N,N'-bis(4'-aminophenyl)-1,3-diaminopropanol, N,N'-bis(p-hydroxyethyl)-N,N'-bis(4'-aminophenyl)ethylenediamine, N, N' - bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine, N, N' -bis (ethyl) -N, N' -bis (4' -amino-3' -methylphenyl)ethylenediamine, 1,8-bis(2,5-diaminophenoxy)-3,5-dioxaoctane, and their addition salts with an acid.

13. Composition according to Claim 8, **characterized in that** the para-aminophenols are chosen from the compounds of formula (III), and their addition salts with an acid: 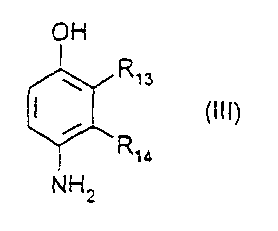 in which:
- R₁₃ represents a hydrogen or halogen atom, a C₁-C₄ alkyl, monohydroxy (C₁-C₄ alkyl), (C₁-C₄) alkoxy (C₁-C₄) - alkyl, amino (C₁-C₄ alkyl) or hydroxy (C₁-C₄) alkylamino-(C₁-C₄ alkyl) radical,
- R₁₄ represents a hydrogen or halogen atom, a C₁-C₄ alkyl, monohydroxy(C₁-C₄ alkyl), polyhydroxy(C₂-C₄ alkyl), amino (C₁-C₄ alkyl), cyano(C₁-C₄ alkyl) or (C₁-C₄) alkoxy (C₁-C₄) alkyl radical,
it being understood that at least one of the radicals R₁₃ or R₁₄ represents a hydrogen atom.

14. Composition according to Claim 13, **characterized in that** the para-aminophenols of formula (III) are chosen from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol, 4-amino-2-fluorophenol, and their addition salts with an acid.

15. Composition according to Claim 8, **characterized in that** the ortho-aminophenols are chosen from 2-aminophenol, 2-amino-5-methylphenol, 2-amino-6-methylphenol, 5-acetamido-2-aminophenol, and their addition salts with an acid.

16. Composition according to Claim 8, **characterized in that** the heterocyclic oxidation bases are chosen from pyridine derivatives, pyrimidine derivatives, pyrazole derivatives, pyrazolopyrimidine derivatives, and their addition salts with an acid.

17. Composition according to any one of the preceding claims, **characterized in that** the oxidation base(s) represent from 0.0005 to 12% by weight of the total weight of the dyeing composition.

18. Composition according to Claim 17, **characterized in that** the oxidation base(s) represent from 0.005 to 6% by weight of the total weight of the dyeing composition.

19. Composition according to Claim 7, **characterized in that** the coupler(s) are chosen from meta-phenylenediamines, meta-aminophenols, meta-diphenols, heterocyclic couplers, and their addition salts with an acid.

20. Composition according to Claim 19, **characterized in that** the couplers are chosen from 2-methyl-5-aminophenol, 5-N-(β-hydroxyethyl) amino-2-methylphenol, 3-aminophenol, 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, sesamol, α-naphthol, 6-hydroxyindole, 4-hydroxyindole, 4-hydroxy-N-methylindole, 6-hydroxyindoline, 2,6-di-hydroxy-4-methylpyridine, 1-H-3-methylpyrazol-5-one, 1-phenyl-3-methylpyrazol-5-one, 2,6-dimethyl-pyrazolo[1,5-b]-1,2,4-triazole, 2, 6-dimethyl [3, 2-c] - 1,2,4-triazole, 6-methylpyrazolo[1,5-a]benzimidazole, and their addition salts with an acid.

21. Composition according to any one of the preceding claims, **characterized in that** the coupler(s) represent from 0.0001 to 10% by weight of the total weight of the dyeing composition.

22. Composition according to Claim 21, **characterized in that** the coupler(s) represent from 0.005 to 5% by weight of the total weight of the dyeing composition.

23. Composition according to any one of the preceding claims, **characterized in that** the cationic direct dyes of formula (V) are chosen from the compounds corresponding to the following structures (VI) to (V52): 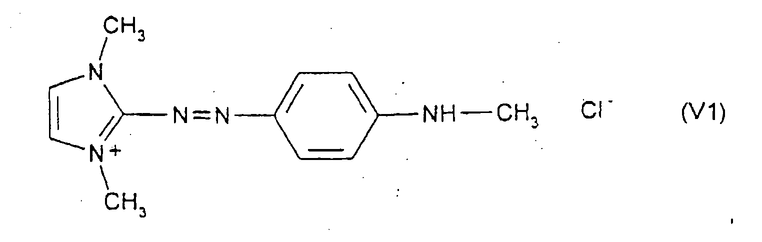 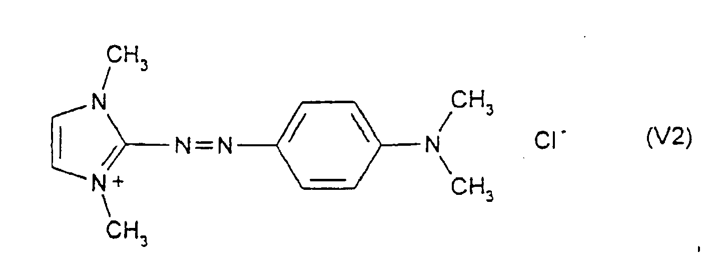 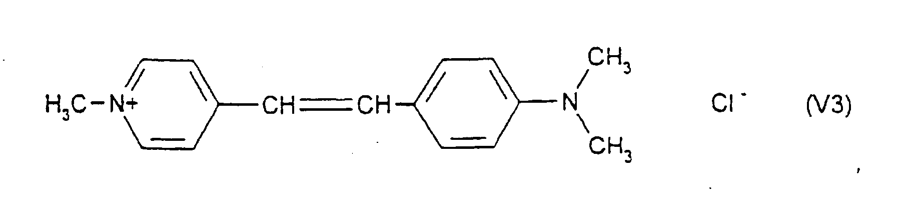 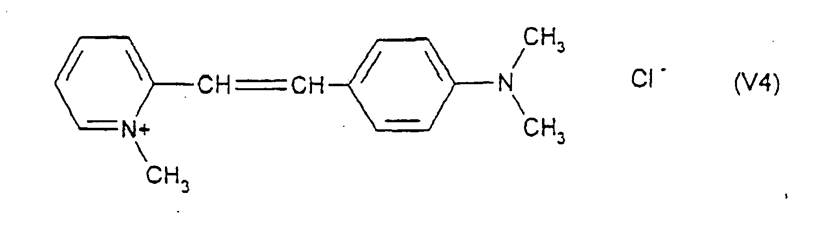 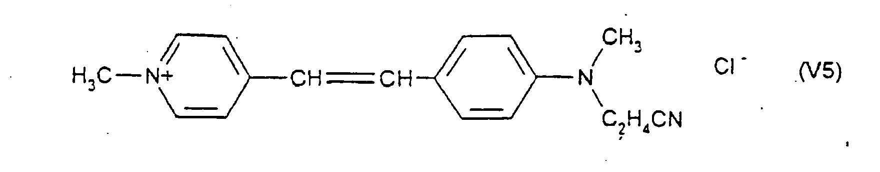 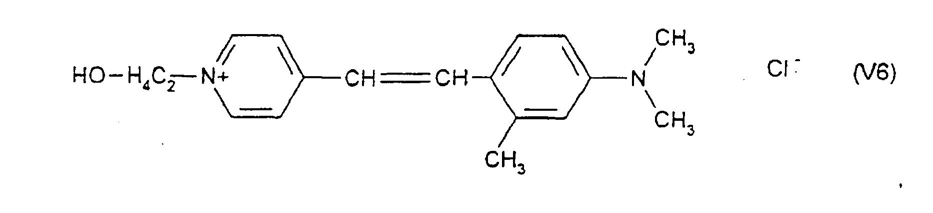 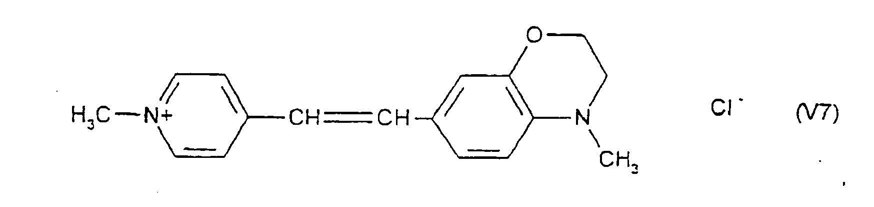 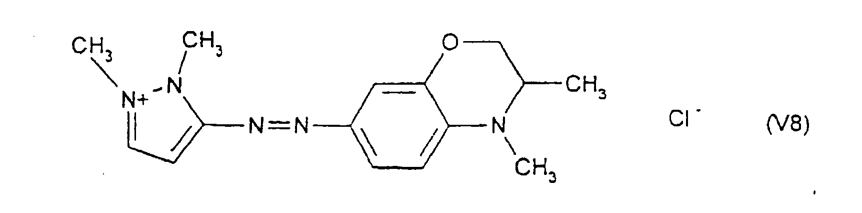 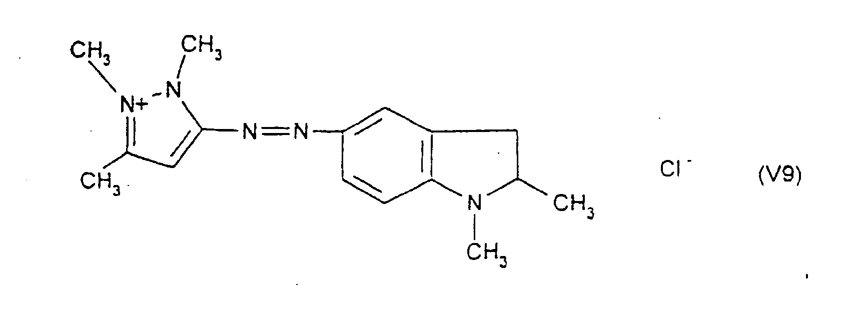 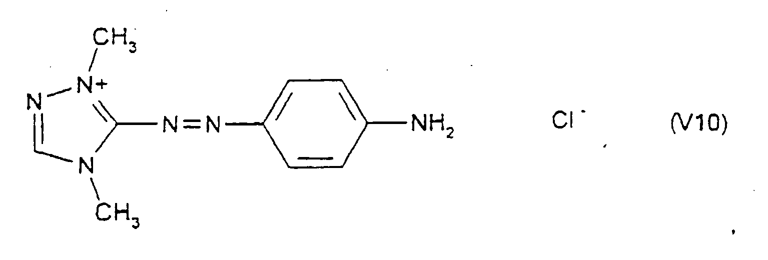 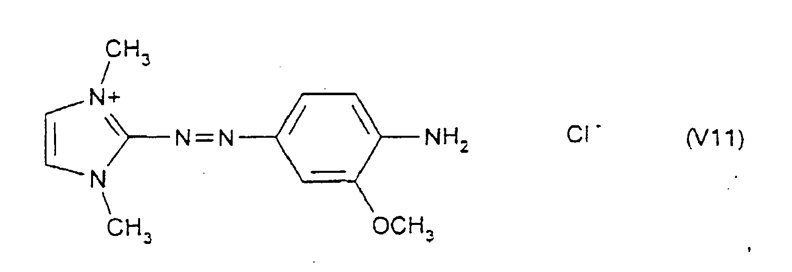 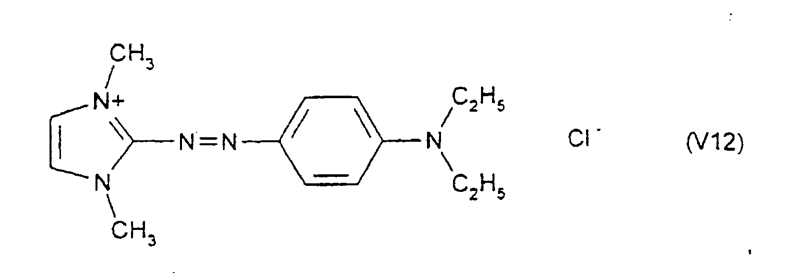 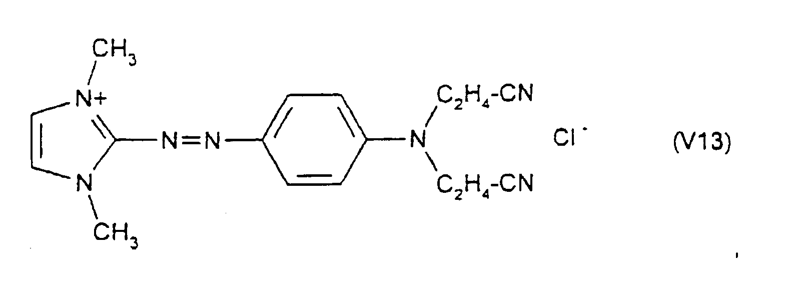 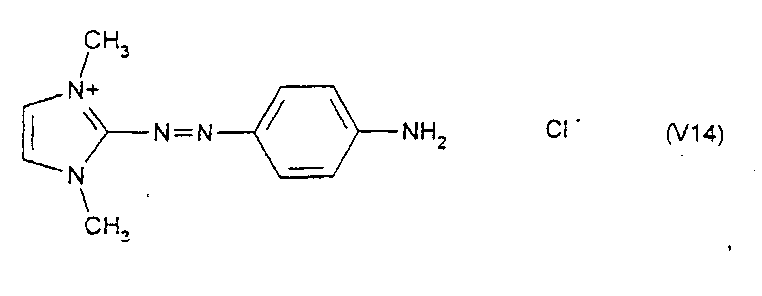 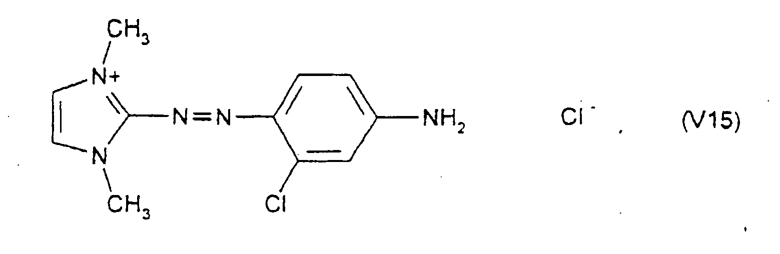 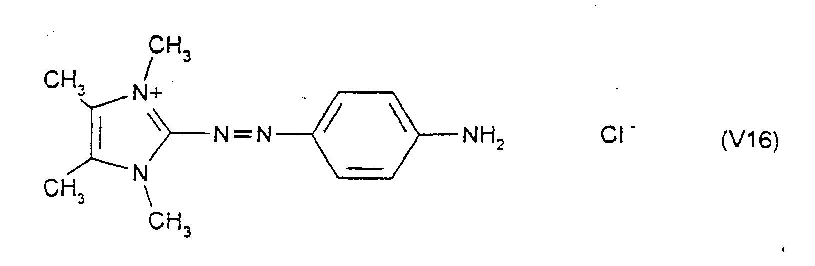 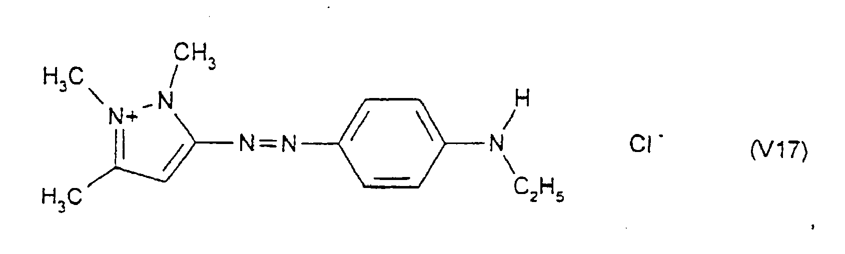 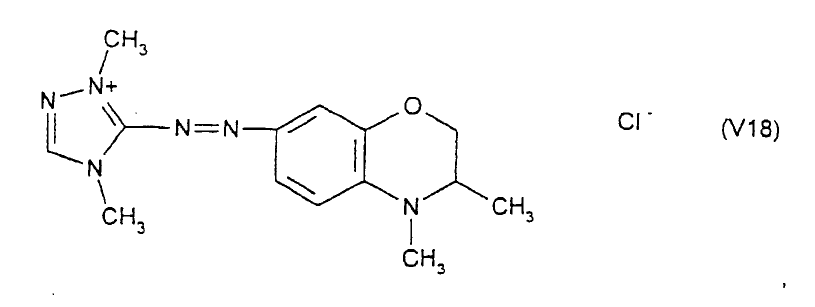 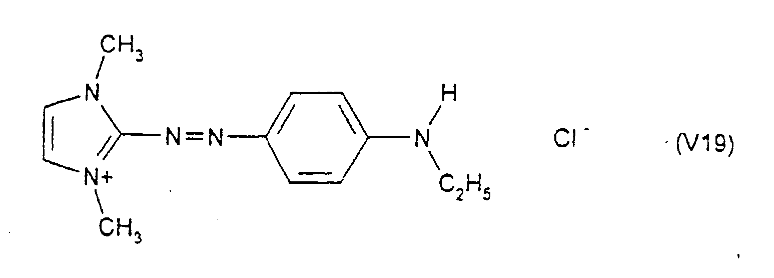 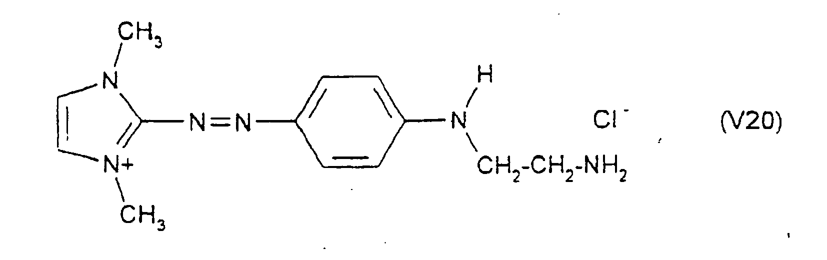 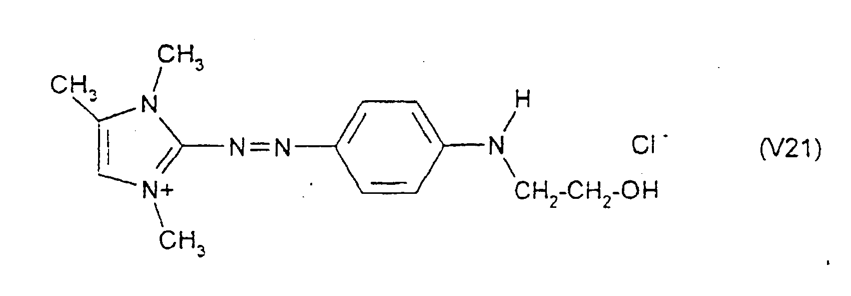 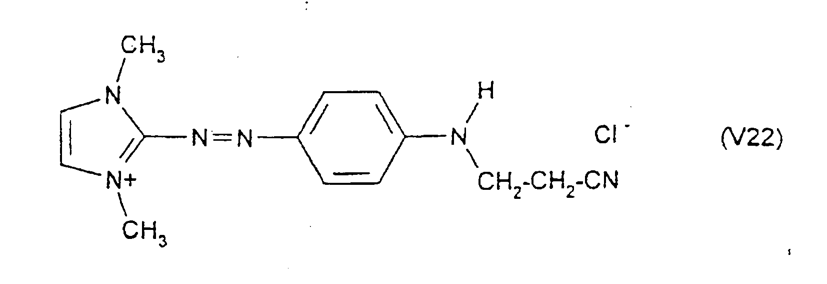 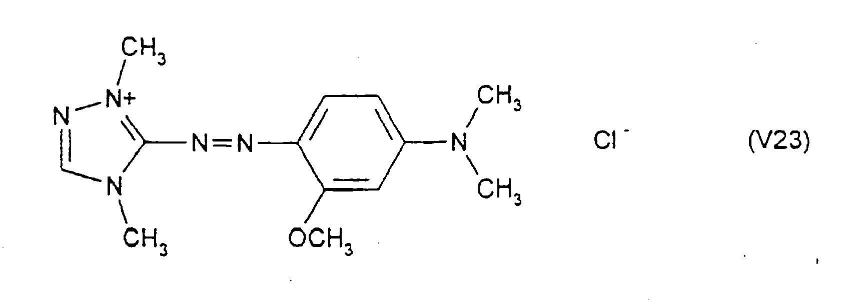 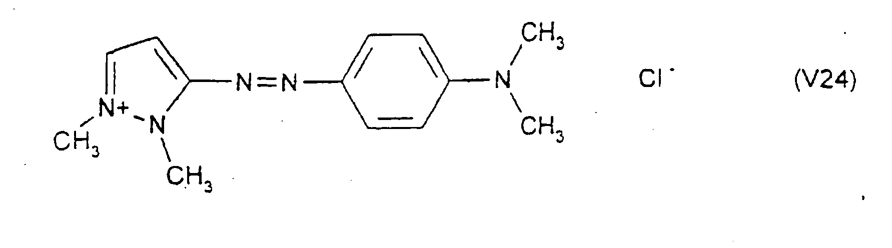 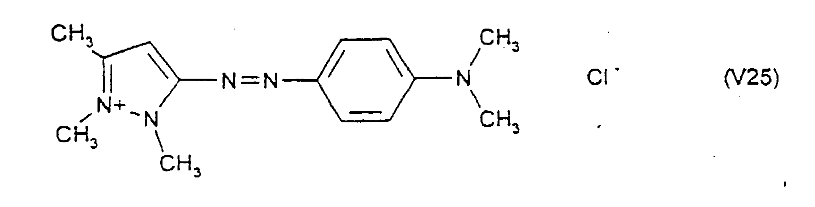 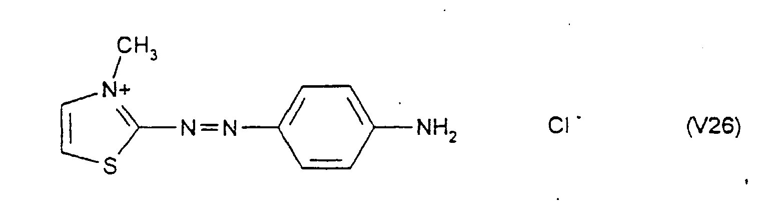 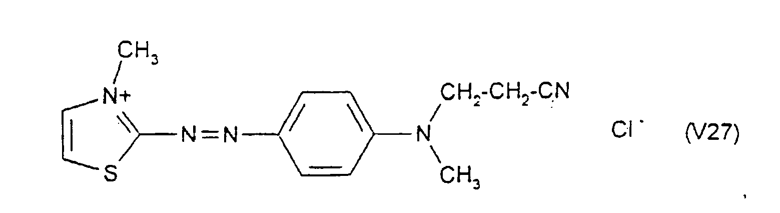 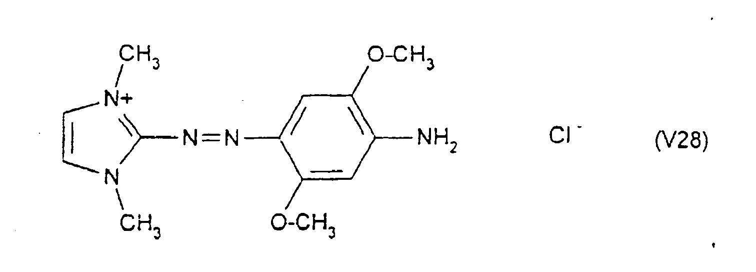 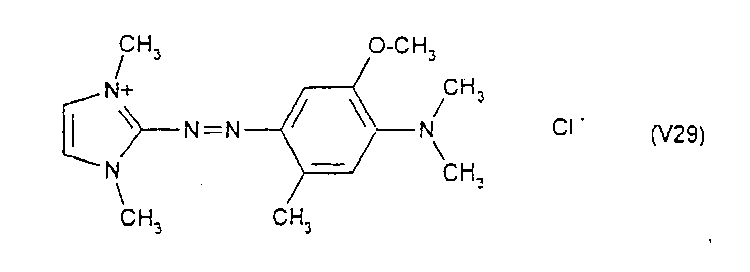 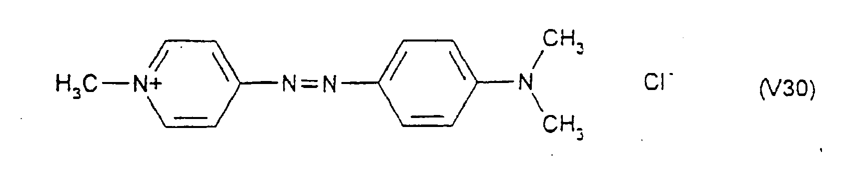 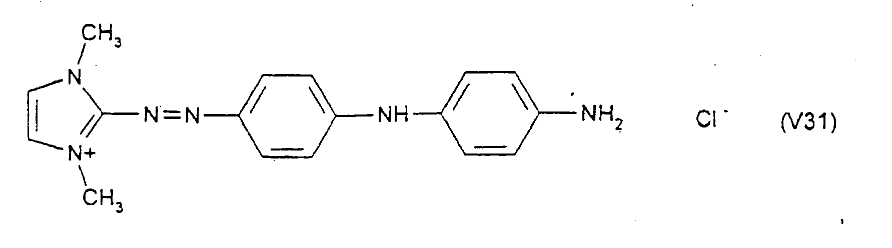 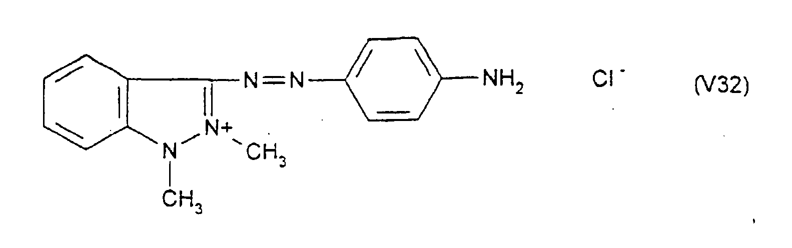 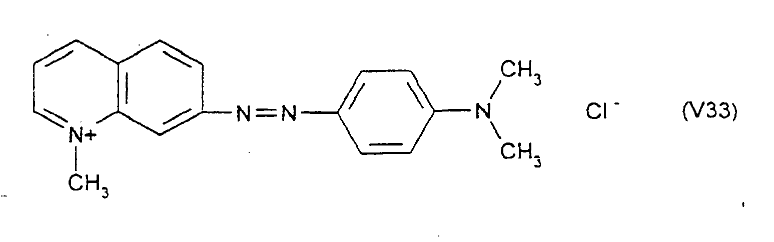 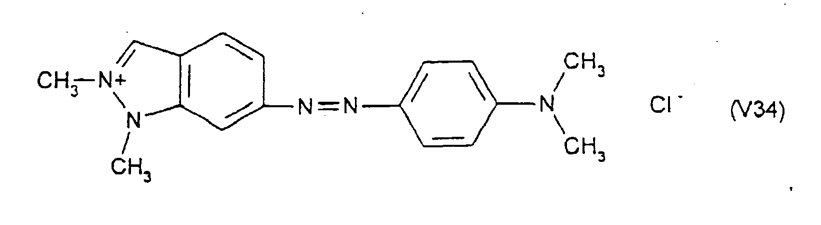 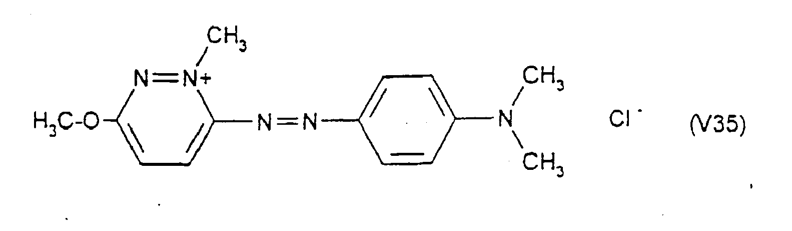 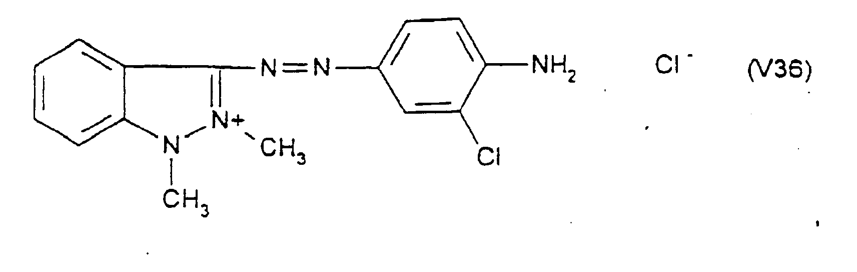 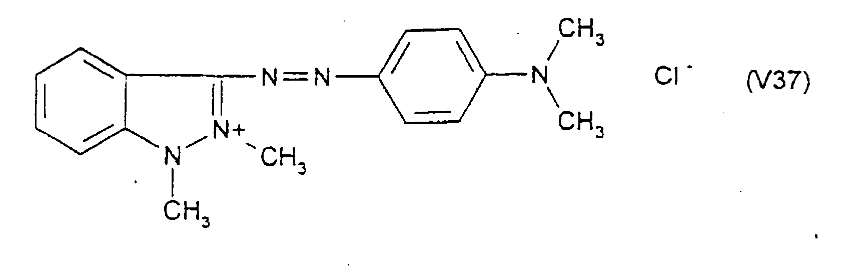 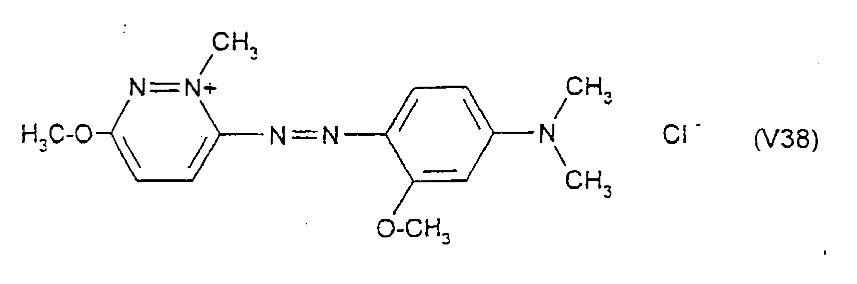 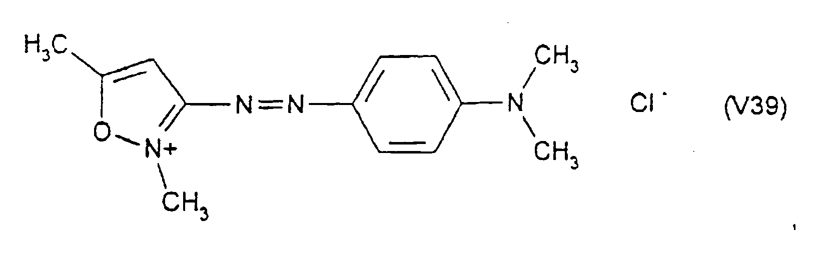 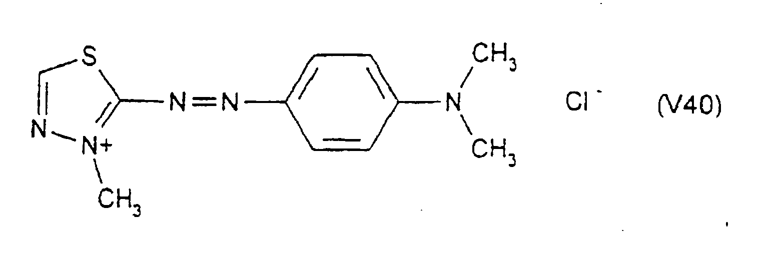 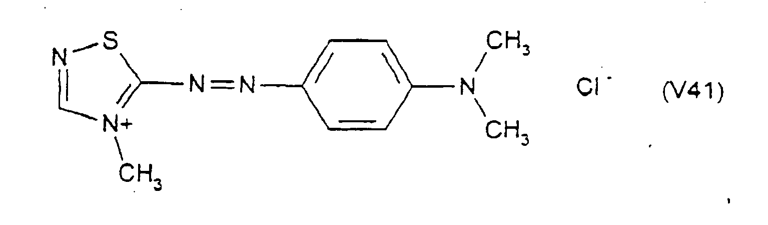 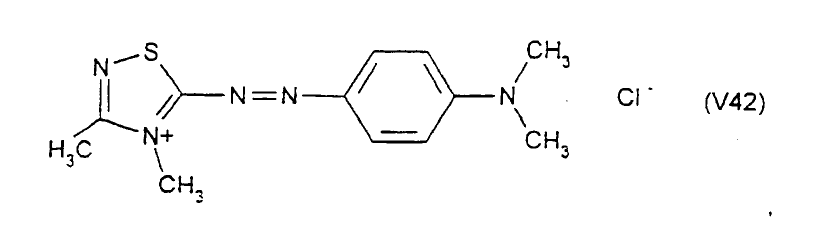 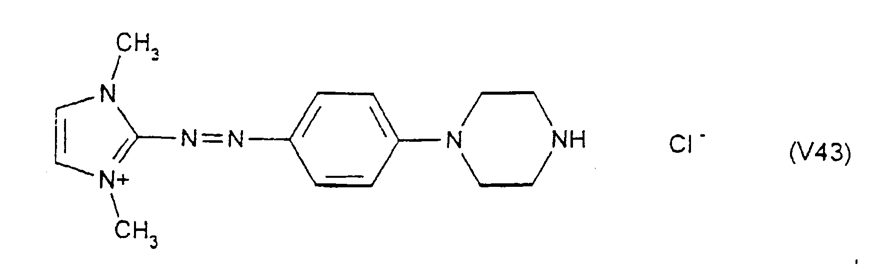 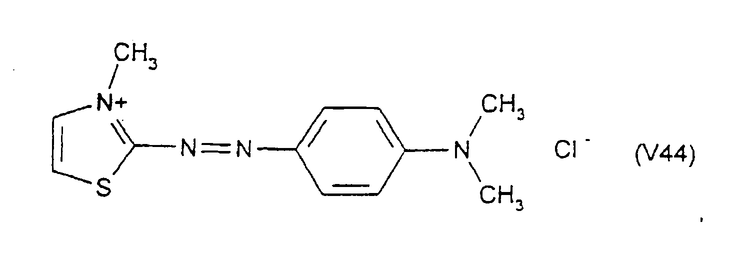 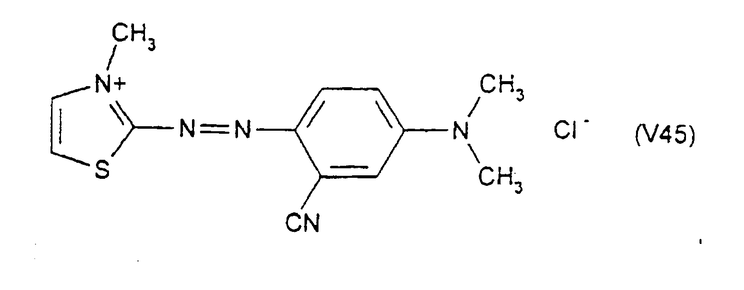 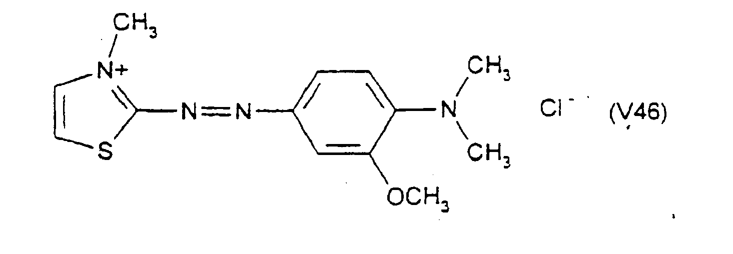 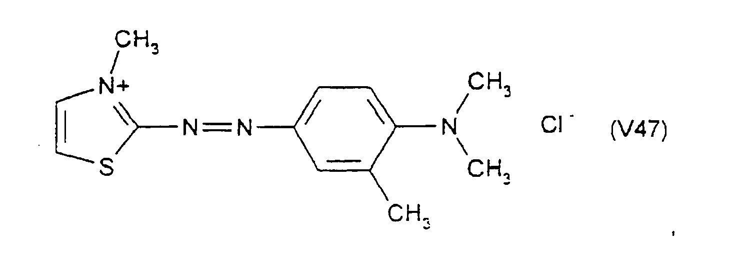 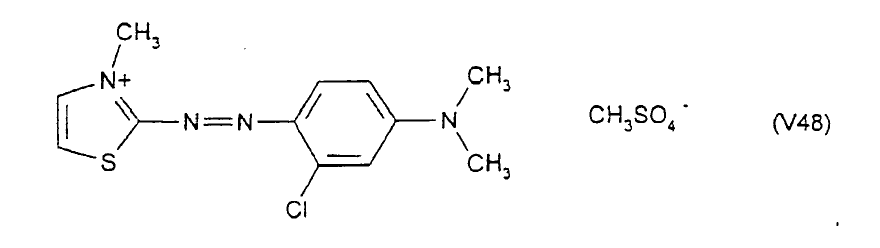 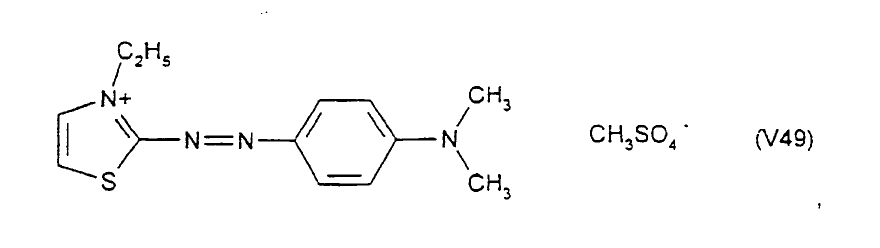 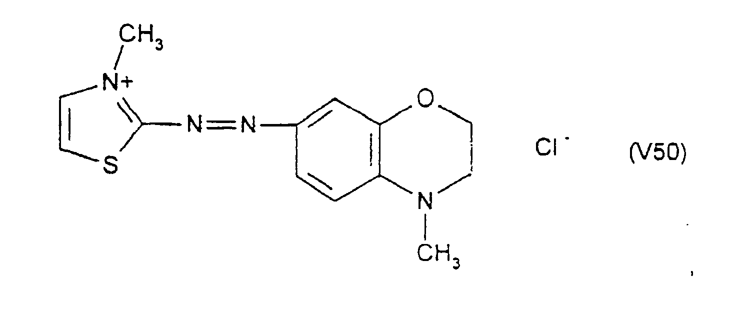 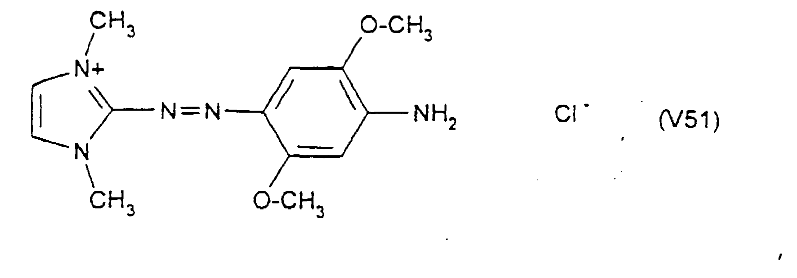 and

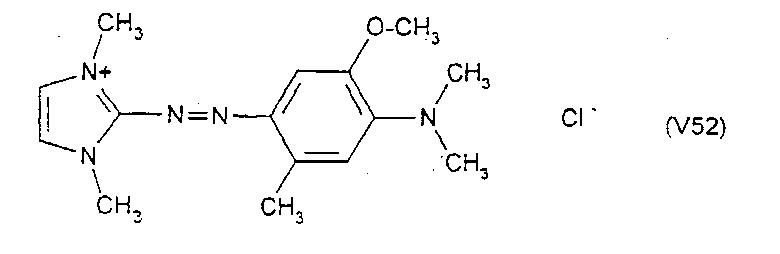

24. Composition according to any one of the preceding claims, **characterized in that** the cationic direct dyes of formula (VI) are chosen from the compounds corresponding to the following structures (VII) to (VI12) : 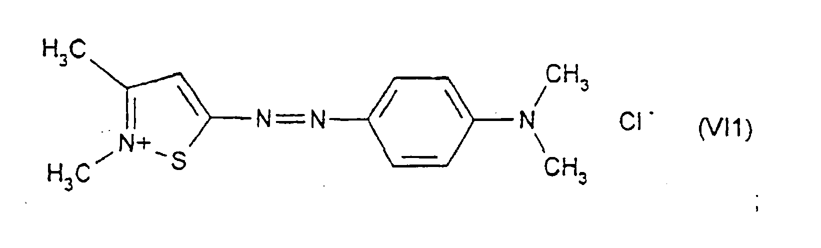 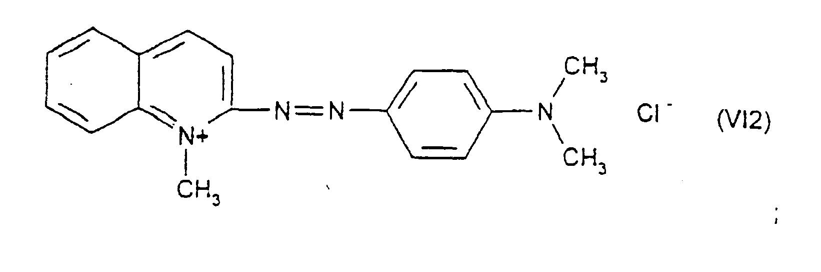 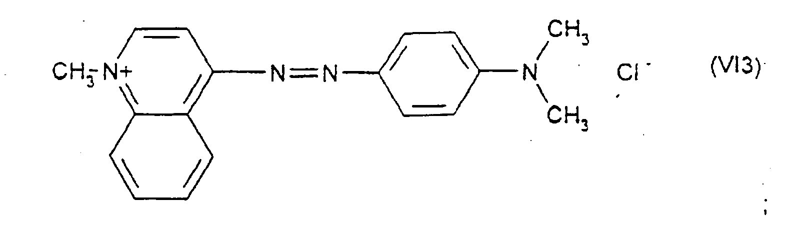 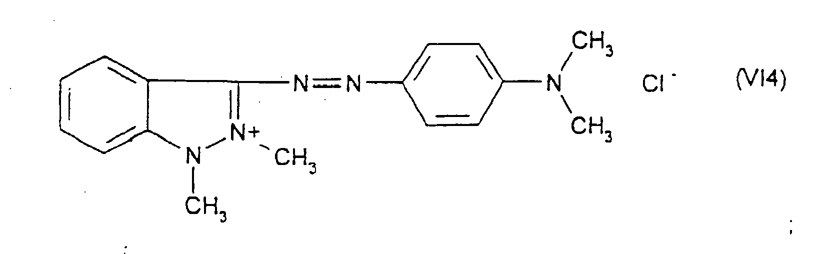 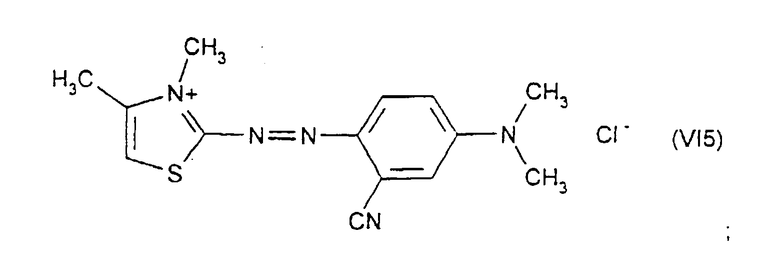 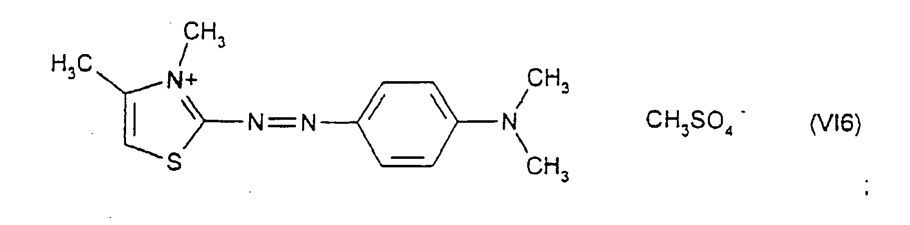 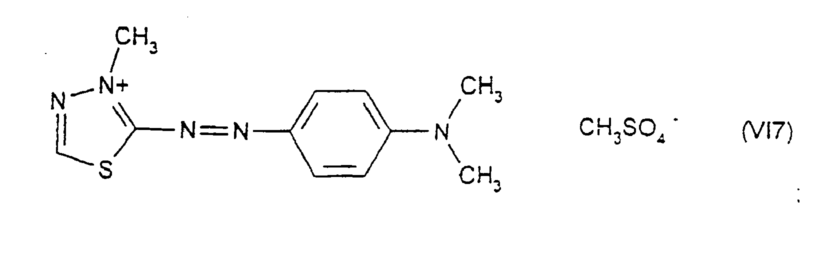 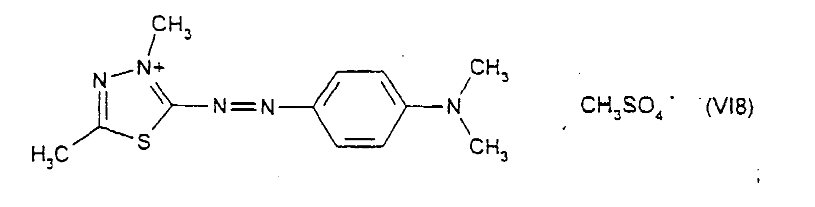 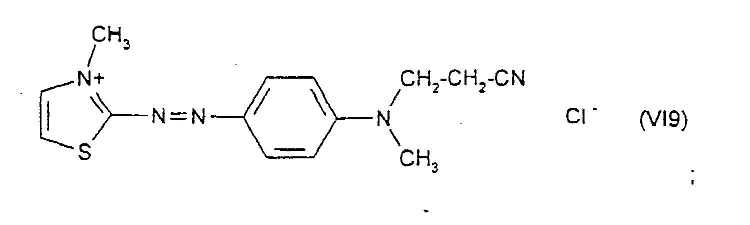 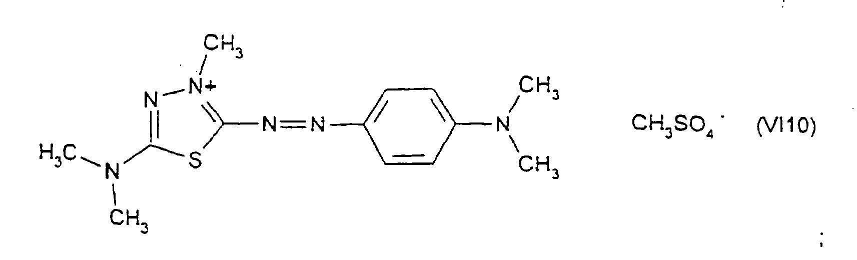 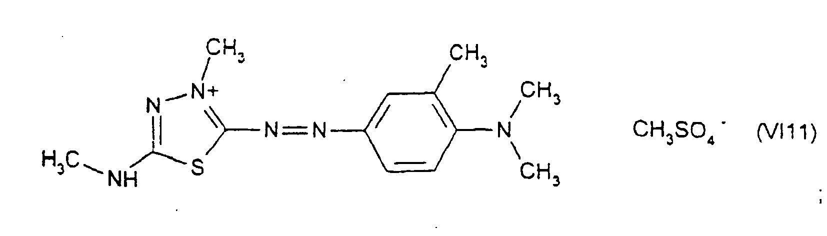 and

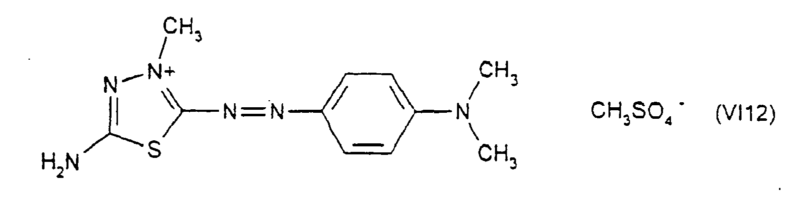

25. Composition according to any one of the preceding claims, **characterized in that** the cationic direct dyes of formula (VII) are chosen from the compounds corresponding to the following structures (VII1) to (VII18): 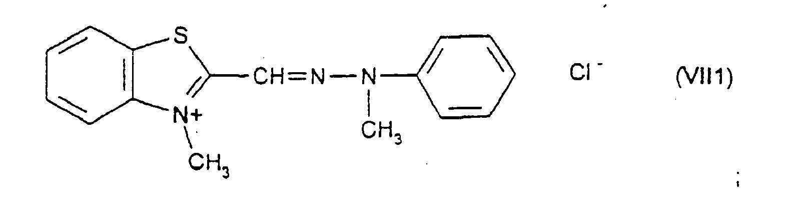 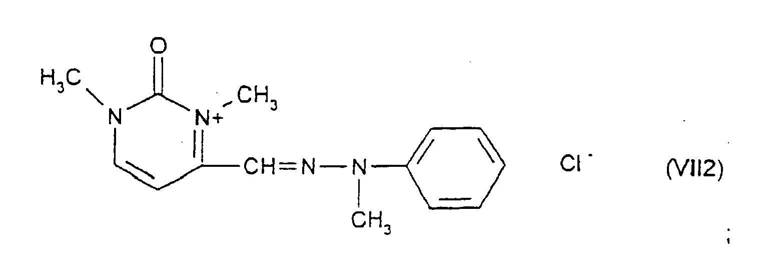 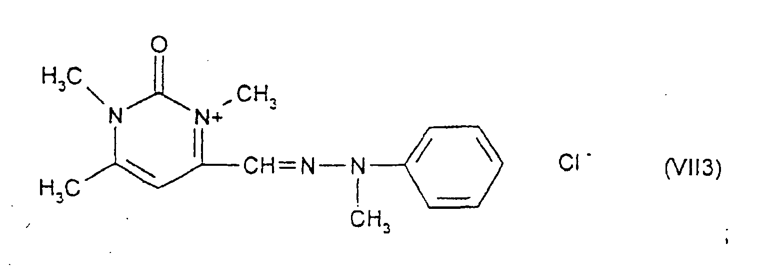 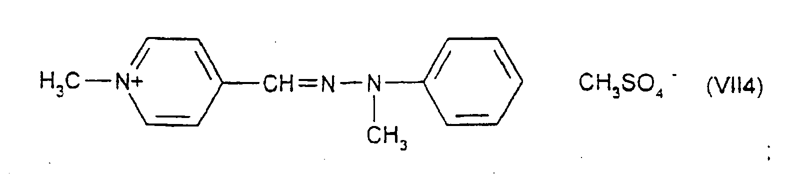 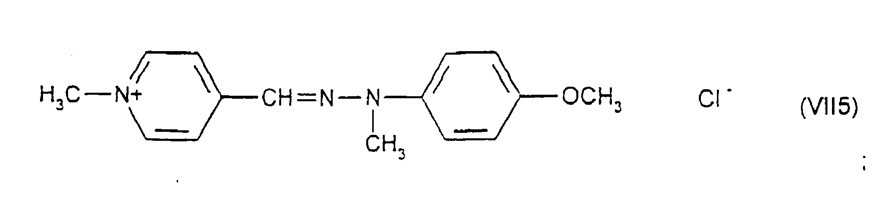 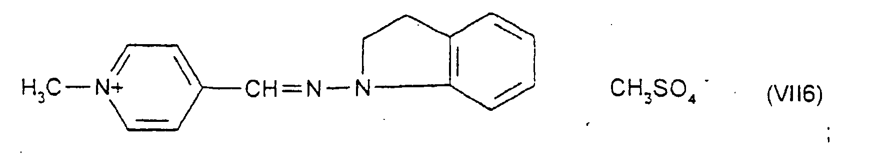 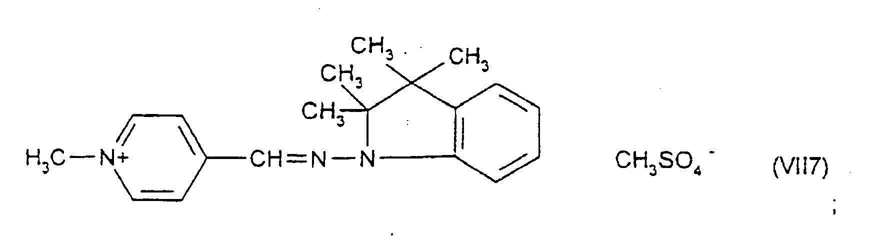 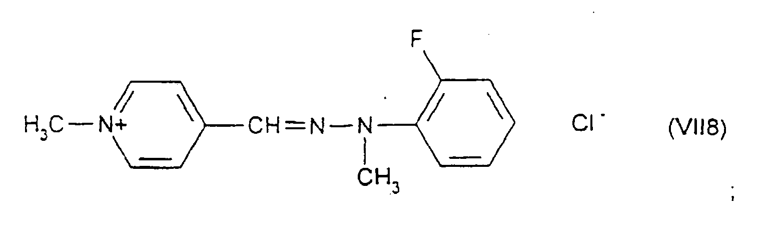 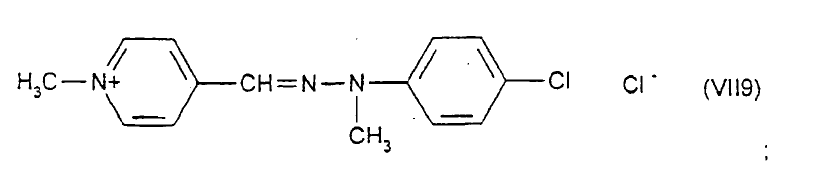 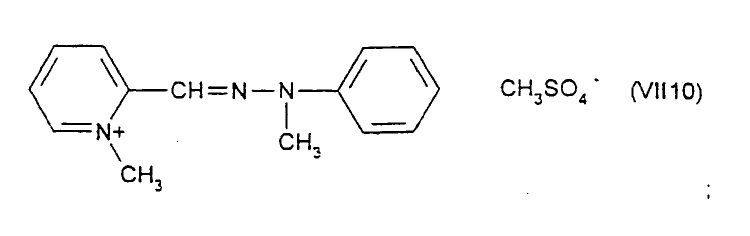 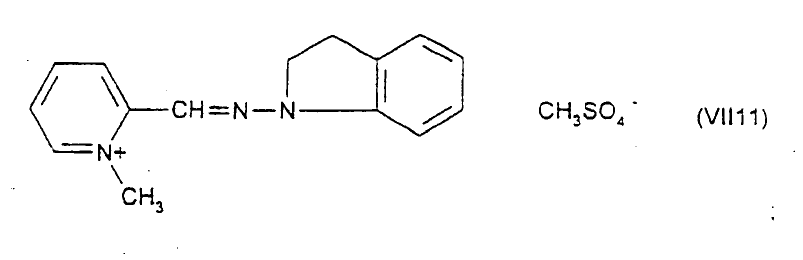 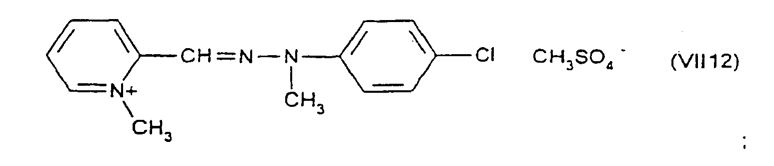 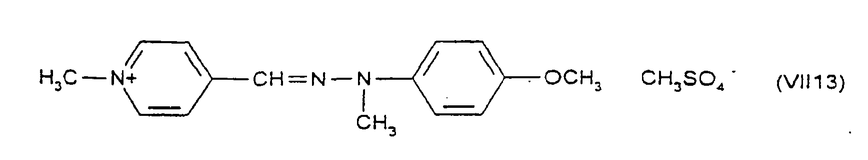 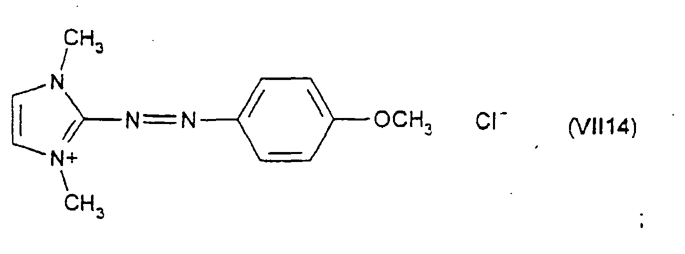 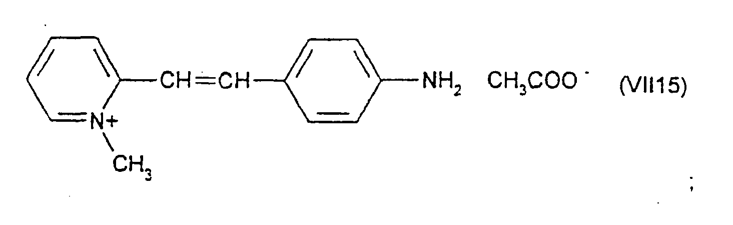 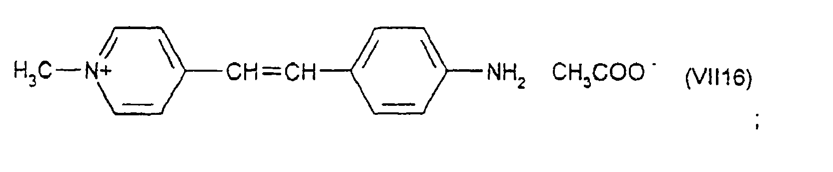 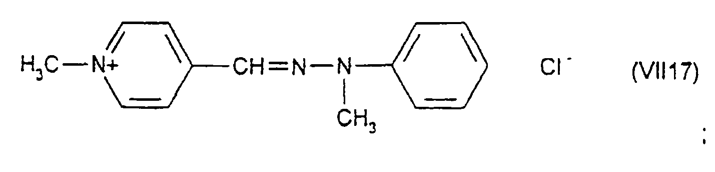 and

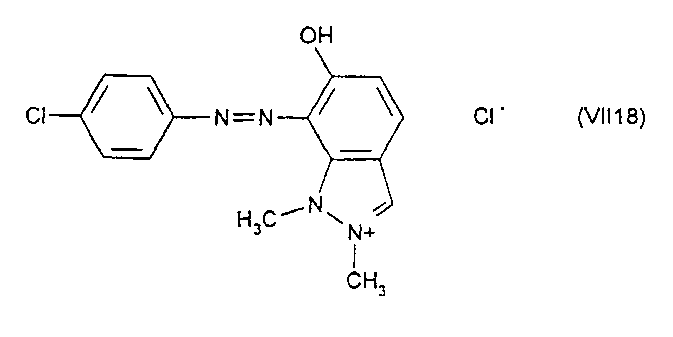

26. Composition according to any one of the preceding claims, **characterized in that** the cationic direct dyes of formula (VII') are chosen from the compounds corresponding to the following structures (VII'1) to (VII' 3) : 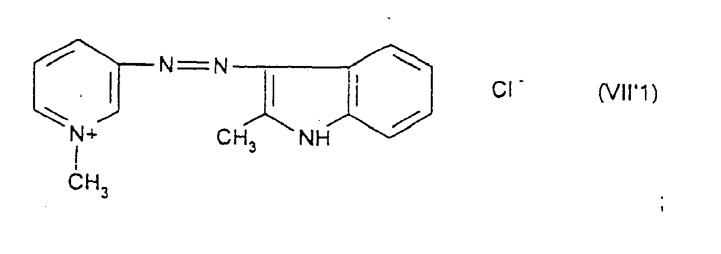 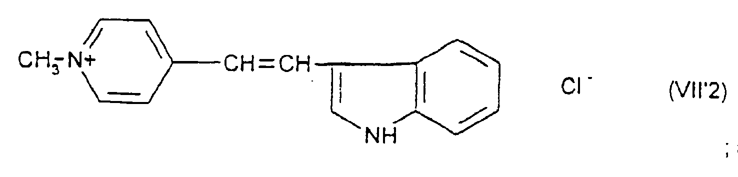 and

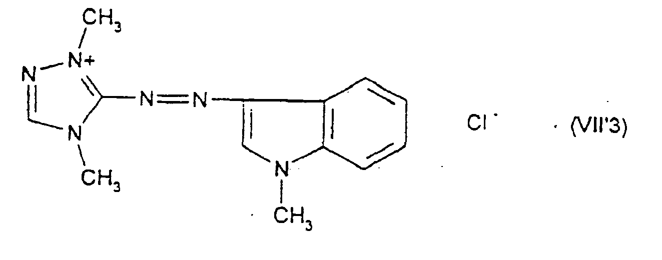

27. Composition according to any one of the preceding claims, **characterized in that** the cationic direct dye(s) represent from 0.001 to 10% by weight of the total weight of the ready-to-use dyeing composition.

28. Composition according to Claim 27, **characterized in that** the cationic direct dye(s) represent from 0.05 to 5% by weight of the total weight of the ready-to-use dyeing composition.

29. Composition according to any one of the preceding claims, **characterized in that** the addition salts with an acid are chosen from hydrochlorides, hydrobromides, sulphates and tartrates, lactates and acetates.

30. Composition according to any one of the preceding claims, **characterized in that** the medium appropriate for dyeing consists of water or of a mixture of water and at least one organic solvent.

31. Composition according to any one of the preceding claims, **characterized in that** it has a pH of between 4 and 11.

32. Method of dyeing keratinous fibres, and in particular human keratinous fibres such as hair, **characterized in that** at least one ready-to-use dyeing composition as defined in any one of the preceding claims is applied to the said fibres for a sufficient time to develop the desired colour.

33. Method according to Claim 32, **characterized in that** it comprises a preliminary step consisting in storing in a separate form, on the one hand, a composition (A) comprising, in a medium appropriate for dyeing, at least one oxidation dye, at least one cationic direct dye and, on the other hand, a composition (B) containing, in a medium appropriate for dyeing, at least one enzyme of the laccase type, and then in mixing them at the time of use before applying this mixture to the keratinous fibres.

34. Multicompartment device or dyeing "kit", **characterized in that** it comprises a first compartment containing the composition (A) as defined in Claim 33 and a second compartment containing the composition (B) as defined in Claim 33.

## Patentansprüche

1. Gebrauchsfertige Zusammensetzung zum oxidativen Färben von Keratinfasern und insbesondere menschlichen Keratinfasern, wie Haaren, **dadurch gekennzeichnet, dass** sie in einem zum Färben geeigneten Medium enthält:
- mindestens einen Oxidationsfarbstoff,
- mindestens einen kationischen Direktfarbstoff, der ausgewählt ist unter:
(a) den Verbindungen der folgenden Formel (V): 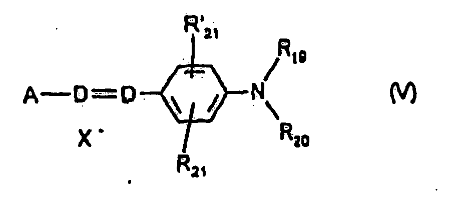 worin bedeuten:
D ein Stickstoffatom oder die Gruppe -CH,
die Gruppen R₁₉ und R₂₀, die identisch oder voneinander verschieden sind, ein Wasserstoffatom; eine C₁₋₄-Alkylgruppe, die mit -CN, -OH oder -NH₂ substituiert sein kann oder mit einem Kohlenstoffatom des Benzolrings einen gegebenenfalls sauerstoff- oder stickstoffhaltigen Heterocyclus bildet, der mit einer oder mehreren C₁₋₄-Alkylgruppen substituiert sein kann; eine 4'-Aminophenylgruppe,
die Gruppen R₂₁ und R'₂₁, die identisch oder voneinander verschieden sind, ein Wasserstoffatom oder ein Halogenatom, das unter Chlor, Brom, Iod und Fluor ausgewählt ist, Cyano, C₁₋₄-Alkoxy oder Acetyloxy,
X- ein Anion, das vorzugsweise unter Chlorid, Methylsulfat und Acetat ausgewählt ist,
A eine Gruppe, die unter den folgenden Strukturen A1 bis A 19 ausgewählt ist: 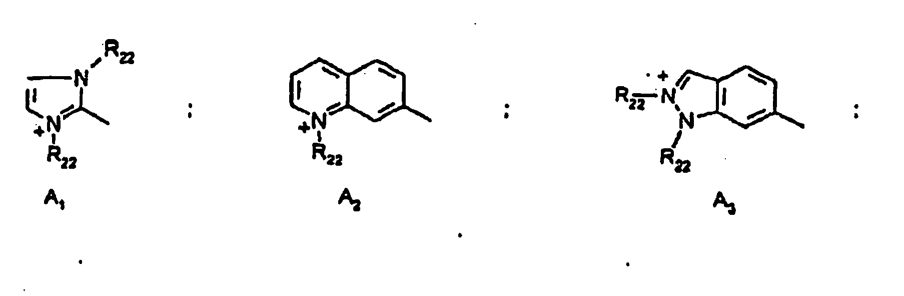 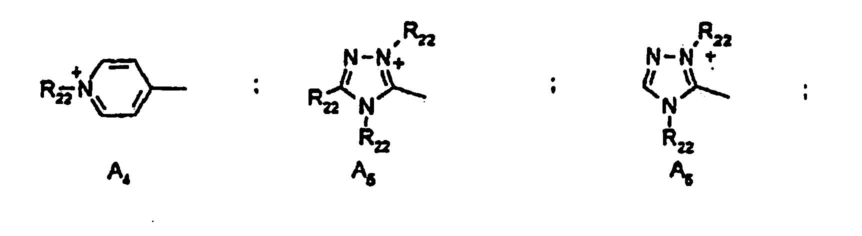 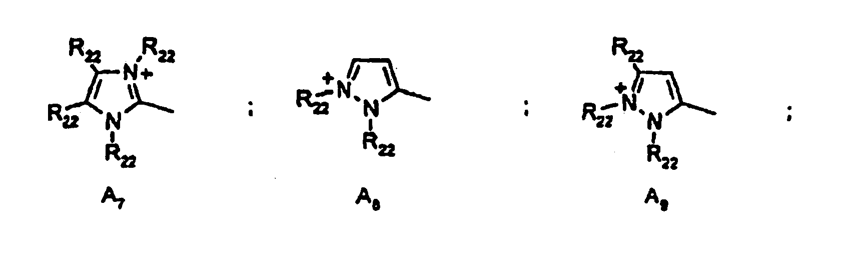 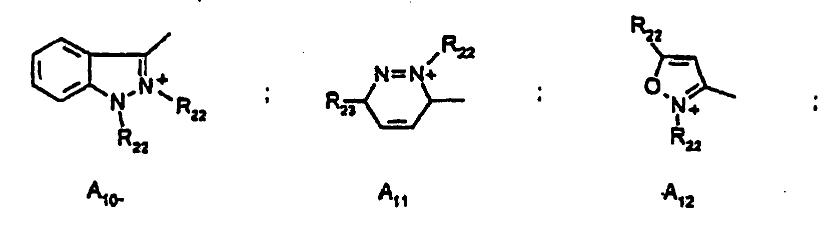 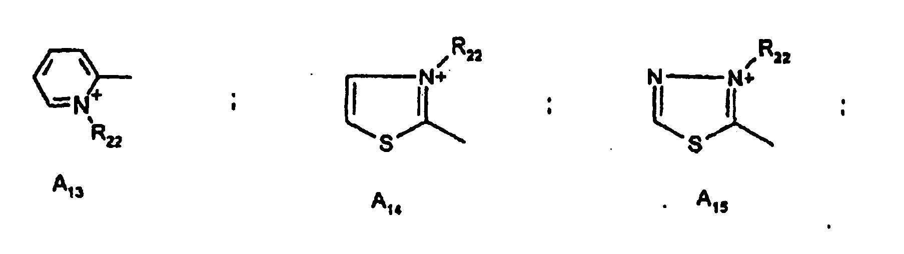 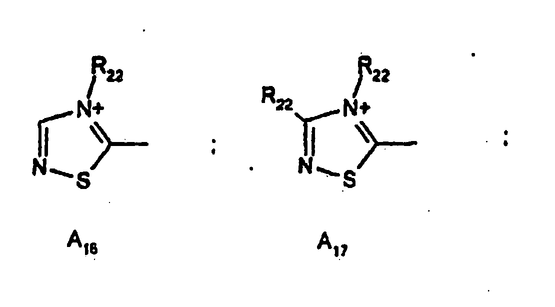 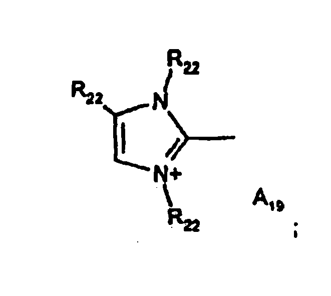
worin R₂₂ eine C₁₋₄-Alkylgruppe, die mit Hydroxy substituiert sein kann, und R₂₃ eine C₁₋₄-Alkoxygruppe bedeutet;
(b) den Verbindungen der folgenden Formel (VI): 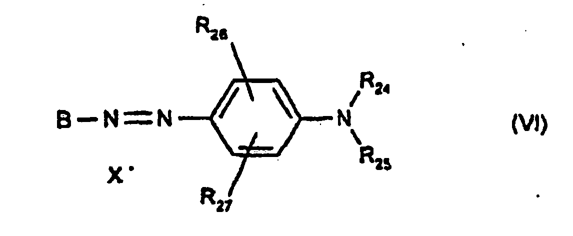 worin bedeuten:
R₂₄ ein Wasserstoffatom oder eine C₁₋₄-Alkylgruppe,
R₂₅ ein Wasserstoffatom, eine Alkylgruppe, die mit einer Gruppe -CN oder einer Aminogruppe substituiert sein kann, 4'-Aminophenyl, oder R₂₅ bildet mit R₂₄ einen gegebenenfalls sauerstoff- und/oder stickstoffhaltigen Heterocyclus, der mit C₁₋₄-Alkyl substituiert sein kann,
die Gruppen R₂₆ und R₂₇, die identisch oder voneinander verschieden sind, ein Wasserstoffatom oder ein Halogenatom, wie Chlor, Iod und Fluor, C₁₋₄-Alkyl, C₁₋₄-Alkoxy oder -CN,
X- ein Anion, das vorzugsweise unter Chlorid, Methylsulfat und Acetat ausgewählt ist,
B eine Gruppe, die unter den folgenden Strukturen B1 bis B6 ausgewählt ist: 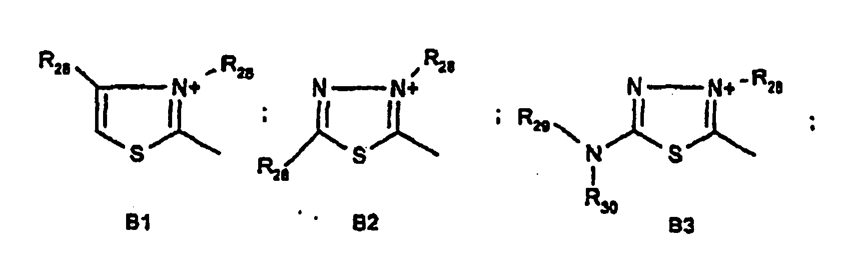 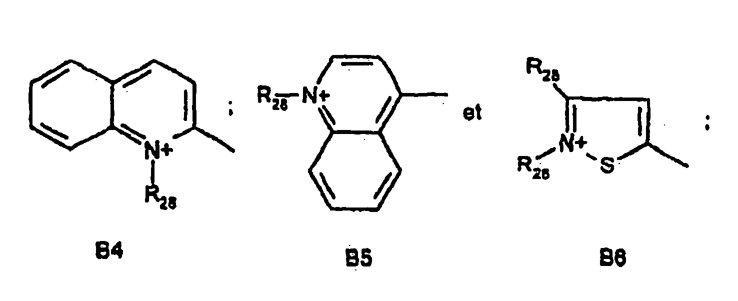
worin R₂₈ eine C₁₋₄-Alkylgruppe und R₂₉ und R₃₀, die identisch oder voneinander verschieden sind, ein Wasserstoffatom oder eine C₁₋₄-Alkylgruppe bedeuten; und
(c) den Verbindungen der folgenden Formel (VII) und (VII'): 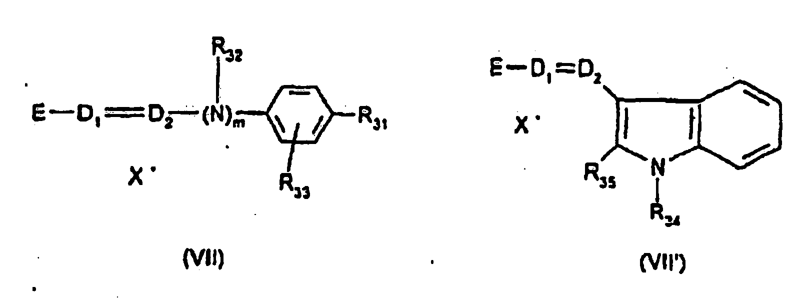 worin bedeuten:
R₃₁ ein Wasserstoffatom, eine C₁₋₄-Alkoxygruppe, ein Halogenatom, wie Brom, Chlor, Iod und Fluor, oder eine Aminogruppe,
R₃₂ ein Wasserstoffatom oder eine C₁₋₄-Alkylgruppe oder R₃₂ bildet mit einem Kohlenstoffatom des Benzolrings einen gegebenenfalls sauerstoffhaltigen und/oder mit einer oder mehreren C₁₋₄-Alkylgruppen substituierten Heterocyclus,
R₃₃ ein Wasserstoffatom oder ein Halogenatom, wie Brom, Chlor, Iod und Fluor,
die Gruppen R₃₄ und R₃₅, die identisch oder voneinander verschieden sind, ein Wasserstoffatom oder eine C₁₋₄-Alkylgruppe,
D₁ und D₂, die identisch oder voneinander verschieden sind, ein Stickstoffatom oder die Gruppe -CH,
m = 0 oder 1,
mit der Maßgabe, dass D₁ und D₂ gleichzeitig -CH bedeuten und m = 0, wenn R₃₁ eine unsubstituierte Aminogruppe ist,
X- ein Anion, das vorzugsweise unter Chlorid, Methylsulfat und Acetat ausgewählt ist,
E eine Gruppe, die unter den folgenden Strukturen E1 bis E8 ausgewählt ist: 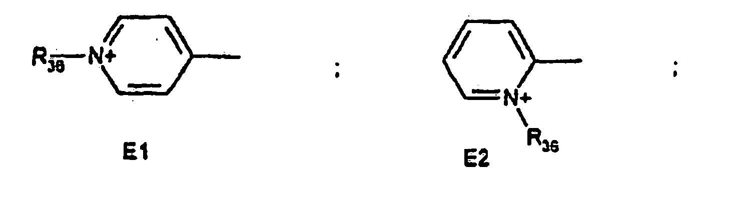 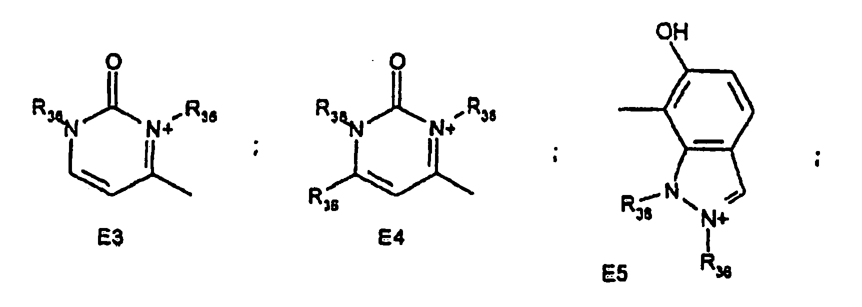 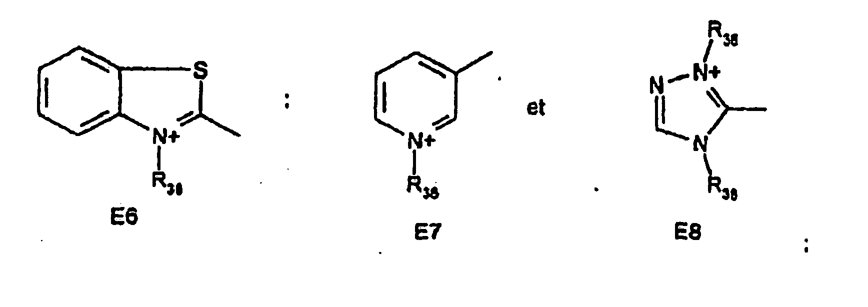
worin R₃₆ eine C₁₋₄-Alkylgruppe bedeutet,
wenn m = 0 und D₁ Stickstoff bedeutet, kann E auch eine Gruppe der folgenden Struktur E9 sein: 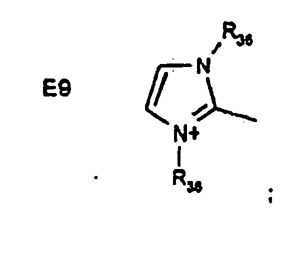 worin R₃₆ eine C₁₋₄-Alkylgruppe bedeutet, und
- mindestens ein Enzym vom Laccase-Typ.

2. Zusammensetzung nach Anspruch 1, **dadurch gekennzeichnet, dass** die Laccase(n) unter den Laccasen pflanzlicher Herkunft, den Laccasen tierischer Herkunft, den Laccasen, die von Pilzen gebildet werden, den Laccasen bakterieller Herkunft oder den biotechnologisch hergestellten Laccasen ausgewählt sind.

3. Zusammensetzung nach einem der Ansprüche 1 oder 2, wobei die Laccasen unter den Laccasen ausgewählt sind, die von Pflanzen gebildet werden, die Chloropyll synthetisieren.

4. Zusammensetzung nach Anspruch 3, wobei die Laccasen unter den Laccasen ausgewählt sind, die aus Anacardiaceae oder Podocarpaceae, Rosmarinus off., Solanum tuberosum, Iris sp., Coffea sp., Daucus carrota, Vinca minor, Persea americana, Catharenthus roseus, Musa sp., Malus pumila, Gingko biloba, Monotropa hypopithys (Fichtenspargel), Aesculus sp., Acer pseudoplatanus, Prunus persica oder Pistacia palaestina extrahiert wurden.

5. Zusammensetzung nach Anspruch 2, wobei die Laccasen unter den Laccasen ausgewählt sind, die von Pyricularia oryzae, Polyporus versicolor, Rhizoctonia praticola, Rhus vernicifera, Scytalidium, Polyporus pinsitus, Myceliophtora thermophila, Rhizoctonia solani, Tramates versicolor, Fomes fomentarius, Chaetomium thermophile, Neurospora crassa, Coriolus versicol, Botrytis cinerea, Rigidoporus lignosus, Phellinus noxius, Pleurotus ostreatus, Aspergillus nidulans, Podospora anserina, Agaricus bisporus, Ganoderma lucidum, Glomerella cingulata, Lactarius piperatus, Russula delica, Heterobasidion annosum, Thelephora terrestris, Cladosporium cladosporioides, Cerrena unicolor, Coriolus hirsutus, Ceriporiopsis subvermispora, Coprinus cinereus, Panaeolus papilionaceus, Panaeolus sphinctrinus, Schizophyllum commune, Dichomitus squalens und deren Varianten stammen.

6. Zusammensetzung nach einem der Ansprüche 1 bis 5, **dadurch gekennzeichnet, dass** die Laccase(n) in Mengenanteilen von 0,5 bis 2000 lacu oder 1000 bis 4.10⁷ Einheiten u oder 20 bis 2.10⁶ Einheiten ulac pro 100 g Zusammensetzung vorliegen.

7. Zusammensetzung nach einem der vorhergehenden Ansprüche, **dadurch gekennzeichnet, dass** die Oxidationsfarbstoffe unter den Oxidationsbasen und/oder den Kupplern ausgewählt sind.

8. Zusammensetzung nach Anspruch 7, **dadurch gekennzeichnet, dass** die Oxidationsbasen unter den p-Phenylendiaminen, Doppelbasen, p-Aminophenolen, o-Amino-phenolen und den heterocyclischen Oxidationsbasen ausgewählt sind.

9. Zusammensetzung nach Anspruch 8, **dadurch gekennzeichnet, dass** die p-Phenylendiamine unter den Verbindungen der folgenden Formel (I) und deren Additionssalzen mit einer Säure ausgewählt sind: 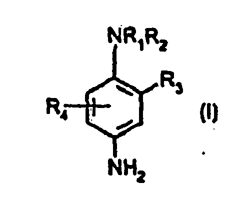 worin bedeuten:
- R₁ ein Wasserstoffatom, C₁₋₄-Alkyl, C₁₋₄-Monohydroxyalkyl, C₂₋₄-Polyhydroxyalkyl, C₁₋₄-Alkoxy-C₁₋₄-alkyl, eine mit einer stickstoffhaltigen Gruppe substituierte C₁₋₄-Alkylgruppe, Phenyl oder 4'-Aminophenyl,
- R₂ Wasserstoff, C₁₋₄-Alkyl, C₁₋₄-Monohydroxyalkyl, C₂₋₄-Polyhydroxyalkyl, C₁₋₄-Alkoxy-C₁₋₄-alkyl oder eine mit einer stickstoffhaltigen Gruppe substituierte C₁₋₄-Alkylgruppe,
- R₃ Wasserstoff, Halogen, wie Chlor, Brom, Iod oder Fluor, C₁₋₄-Alkyl, C₁₋₄-Monohydroxyalkyl, C₁₋₄-Hydroxyalkoxy, C₁₋₄-Acetylaminoalkoxy, C₁₋₄-Mesylaminoalkoxy oder C₁₋₄-Carbamoylaminoalkoxy, und
- R₄ Wasserstoff, Halogen oder C₁₋₄-Alkyl.

10. Zusammensetzung nach Anspruch 9, **dadurch gekennzeichnet, dass** die p-Phenylendiamine der Formel (I) unter p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-N,N-diethyl-3-methyl-anilin, N,N-Bis(β-hydroxyethyl)-p-phenylendiamin, 4-N,N-bis(β-Hydroxyethyl)amino-2-methyl-anilin, 4-N,N-bis(β-Hydroxyethyl)amino-2-chlor-anilin, 2-β-Hydroxyethyl-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl, β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-β-Hydroxyethyloxy-p-phenylendiamin, 2-β-Acetylaminoethyloxy-p-phenylendiamin, N-(β-Methoxyethyl)-p-phenylendiamin und deren Additionssalzen mit einer Säure ausgewählt sind.

11. Zusammensetzung nach Anspruch 8, **dadurch gekennzeichnet, dass** die Doppelbasen unter den Verbindungen der folgenden Formel (II) und deren Additionssalzen mit einer Säure ausgewählt sind: 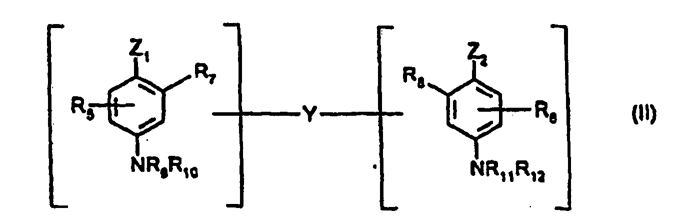 worin bedeuten:
- Z₁ und Z₂, die identisch oder voneinander verschieden sind, eine Gruppe Hydroxy oder -NH₂, die mit C₁₋₄-Alkyl oder einer Verbindungsgruppe Y substituiert sein kann,
- die Verbindungsgruppe Y eine geradkettige oder verzweigte Alkylengruppe mit 1 bis 14 Kohlenstoffatomen, die durch eine oder mehrere stickstoffhaltige Gruppen und/oder ein oder mehrere Heteroatome, wie Sauerstoff, Schwefel oder Stickstoff, unterbrochen oder abgeschlossen werden kann und gegebenenfalls mit einer oder mehreren Hydroxy- oder C₁₋₄-Alkoxygruppen substituiert sein kann,
- R₅ und R₆ Wasserstoff, Halogen, C₁₋₄-Alkyl, C₁₋₄-Monohydroxyalkyl, C₂₋₄-Polyhydroxyalkyl, C₁₋₄-Aminoalkyl oder eine Verbindungsgruppe Y,
- R₇, R₈, R₉, R₁₀, R₁₁ und R₁₂, die identisch oder voneinander verschieden sind, ein Wasserstoffatom, eine Verbindungsgruppe Y oder C₁₋₄-Alkyl,
- mit der Maßgabe, dass die Verbindungen der Formel (II) nur eine Verbindungsgruppe Y pro Molekül aufweisen.

12. Zusammensetzung nach Anspruch 11, **dadurch gekennzeichnet, dass** die Doppelbasen der Formel (II) unter N,N'-Bis(β-hydroxyethyl)-N,N'-bis-(4'-aminophenyl)-1,3-diaminopropanol, N,N'-Bis(β-hydroxyethyl)-N,N'-bis(4'-aminophenyl)-ethylendiamin, N,N'-Bis(4-aminophenyl)-tetramethylendiamin, N,N'-Bis(β-hydroxyethyl)-N,N'-bis(4-aminophenyl)-tetramethylendiamin, N,N'-Bis(4-methylaminophenyl)-tetramethylendiamin, N,N'-Bis(ethyl)-N,N'-bis-(4'-amino, 3'-methylphenyl)-ethylendiamin, 1,8-Bis(2,5-diaminophenoxy)-3,5-dioxaoctan und deren Additionssalzen mit einer Säure ausgewählt sind.

13. Zusammensetzung nach Anspruch 8, **dadurch gekennzeichnet, dass** die p-Aminophenole unter den Verbindungen der folgenden Formel (III) und deren Additionssalzen mit einer Säure ausgewählt sind: 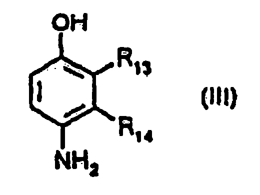 worin bedeuten:
- R₁₃ Wasserstoff, Halogen, C₁₋₄-Alkyl, C₁₋₄-Monohydroxyalkyl, C₁₋₄-Alkoxy-C₁₋₄-alkyl, C₁₋₄-Aminoalkyl oder C₁₋₄-Hydroxyalkyl-C₁₋₄-aminoalkyl,
- R₁₄ Wasserstoff, Halogen, C₁₋₄-Alkyl, C₁₋₄-Monohydroxyalkyl, C₂₋₄-Polyhydroxyalkyl, C₁₋₄-Aminoalkyl, C₁₋₄-Cyanoalkyl oder C₁₋₄-Alkoxy-C₁₋₄-alkyl,
mit der Maßgabe, dass mindestens eine der Gruppen R₁₃ oder R₁₄ Wasserstoff bedeutet.

14. Zusammensetzung nach Anspruch 13, **dadurch gekennzeichnet, dass** die p-Aminophenole der Formel (III) unter p-Aminophenol, 4-Amino-3-methyl-phenol, 4-Amino-3-fluor-phenol, 4-Amino-3-hydroxymethyl-phenol, 4-Amino-2-methyl-phenol, 4-Amino-2-hydroxymethyl-phenol, 4-Amino-2-methoxymethyl-phenol, 4-Amino-2-aminomethyl-phenol, 4-Amino-2-(β-hydroxyethylaminomethyl)-phenol, 4-Amino-2-fluor-phenol und deren Additionssalzen mit einer Säure ausgewählt sind.

15. Zusammensetzung nach Anspruch 8, **dadurch gekennzeichnet, dass** die o-Aminophenole unter 2-Aminophenol, 2-Amino-5-methylphenol, 2-Amino-6-methyl-phenol, 5-Acetamido-2-amino-phenol und deren Additionssalzen mit einer Säure ausgewählt sind.

16. Zusammensetzung nach Anspruch 8, **dadurch gekennzeichnet, dass** die heterocyclischen Oxidationsbasen unter den Pyridinderivaten, Pyrimidinderivaten, Pyrazolderivaten, Pyrazolo-pyrimidinderivaten und deren Additionssalzen mit einer Säure ausgewählt sind.

17. Zusammensetzung nach einem der vorhergehenden Ansprüche, **dadurch gekennzeichnet, dass** die Oxidationsbase(n) 0,0005 bis 12 Gew.-% des Gesamtgewichts der Färbemittelzusammensetzung ausmachen.

18. Zusammensetzung nach Anspruch 17, **dadurch gekennzeichnet, dass** die Oxidationsbase(n) 0,005 bis 6 Gew.-% des Gesamtgewichts der Färbemittelzusammensetzung ausmachen.

19. Zusammensetzung nach Anspruch 7, **dadurch gekennzeichnet, dass** der oder die Kuppler unter den m-Phenylendiaminen, m-Aminophenolen, m-Dihydroxybenzolen und heterocyclischen Kupplern und deren Additionssalzen mit einer Säure ausgewählt sind.

20. Zusammensetzung nach Anspruch 19, **dadurch gekennzeichnet, dass** die Kuppler unter 2-Methyl-5-amino-phenol, 5-N-(β-Hydroxyethyl)-amino-2-methyl-phenol, 3-Amino-phenol, 1,3-Dihydroxy-benzol, 1,3-Dihydroxy-2-methyl-benzol, 4-Chlor-1,3-dihydroxy-benzol, 2,4-Diamino-1-(β-hydroxyethyloxy)-benzol, 2-Amino-4-(β-hydroxyethylamino)-1-methoxy-benzol, 1,3-Diaminobenzol, 1,3-Bis(2,4-diaminophenoxy)-propan, Sesamol, α-Naphthol, 6-Hydroxyindol, 4-Hydroxy-indol, 4-Hydroxy-N-methyl-indol, 6-Hydroxyindolin, 2,6-Dihydroxy-4-methyl-pyridin, 1-H-3-Methyl-pyrazol-5-on, 1-Phenyl-3-methyl-pyrazol-5-on, 2,6-Dimethyl-pyrazolo-[1,5-b]-1,2,4-triazol, 2,6-Dimethyl-[3,2-c]-1,2,4-triazol, 6-Methyl-pyrazolo-[1,5-a]-benzimidazol und den Additionssalzen dieser Verbindungen mit einer Säure ausgewählt sind.

21. Zusammensetzung nach einem der vorhergehenden Ansprüche, **dadurch gekennzeichnet, dass** der oder die Kuppler 0,0001 bis 10 Gew.-% des Gesamtgewichts der Färbemittelzusammensetzung ausmachen.

22. Zusammensetzung nach Anspruch 21, **dadurch gekennzeichnet, dass** der oder die Kuppler 0,005 bis 5 Gew.-% des Gesamtgewichts der Färbemittelzusammensetzung ausmachen.

23. Zusammensetzung nach einem der vorhergehenden Ansprüche, **dadurch gekennzeichnet, dass** die kationischen Direktfarbstoffe der Formel (V) unter den Verbindungen der folgenden Strukturen (V1) bis (V52) ausgewählt sind:

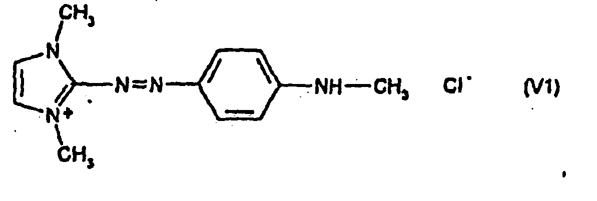

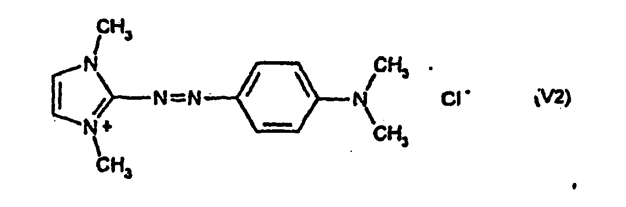

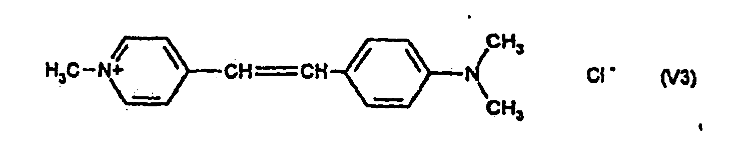

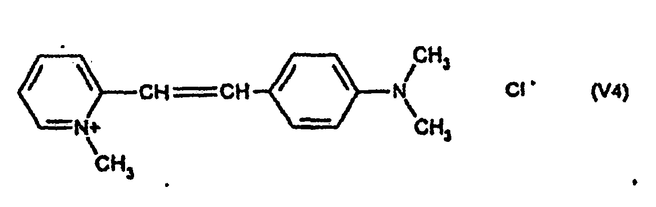

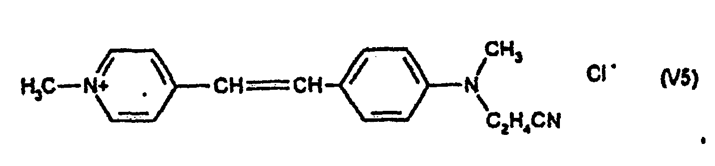

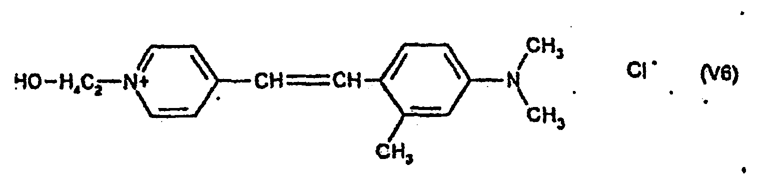

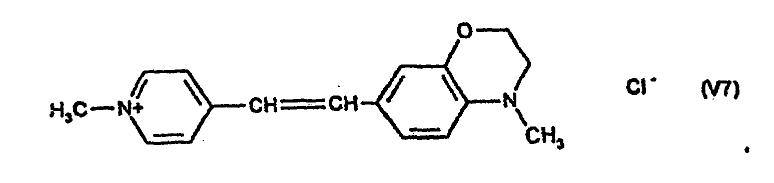

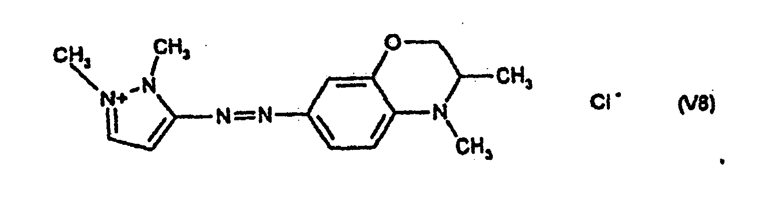

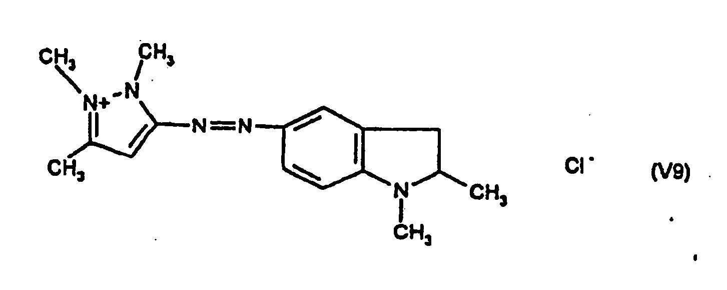

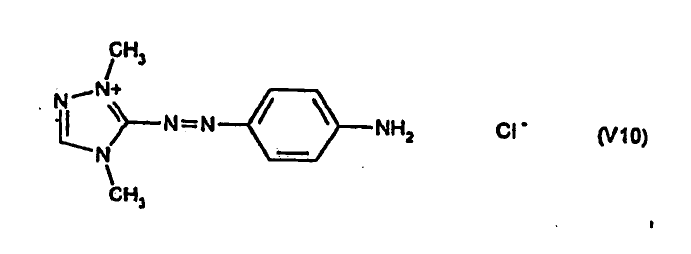

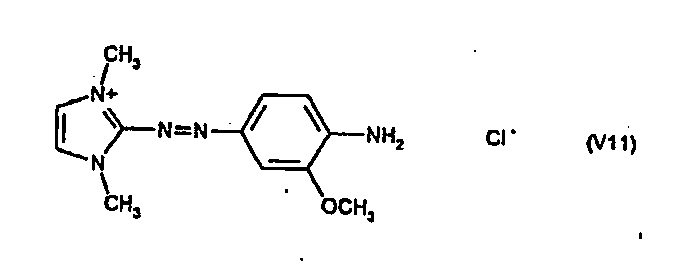

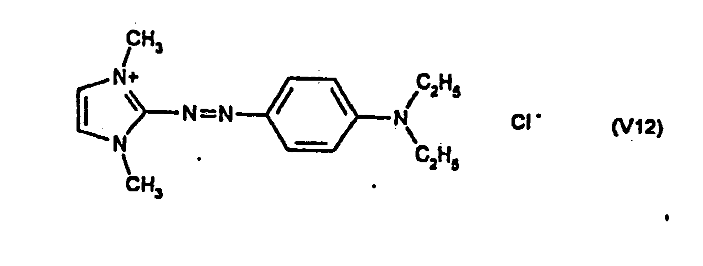

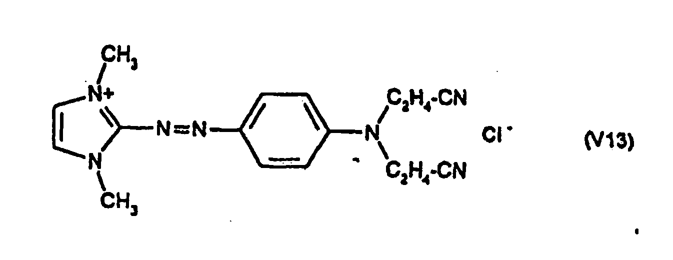

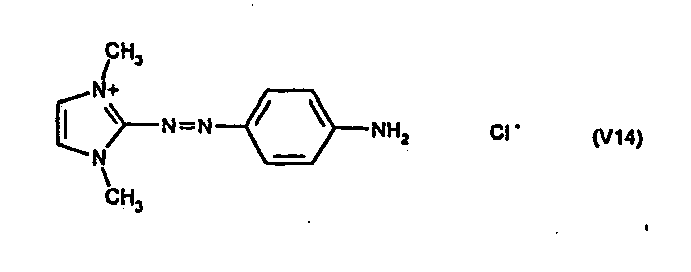

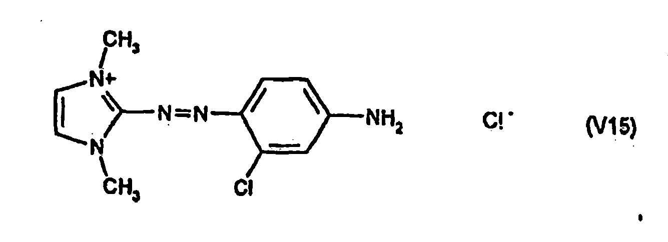

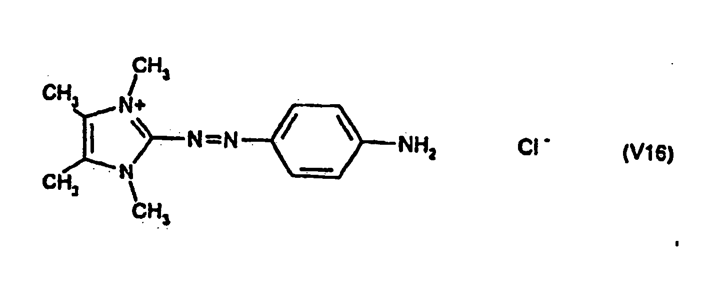

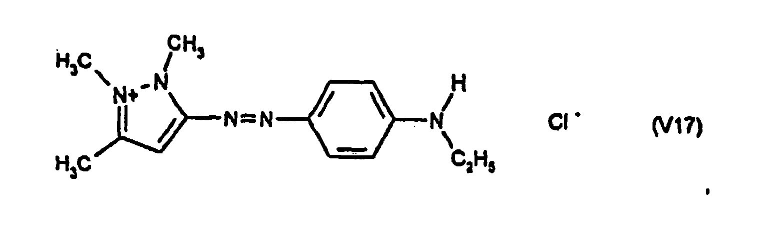

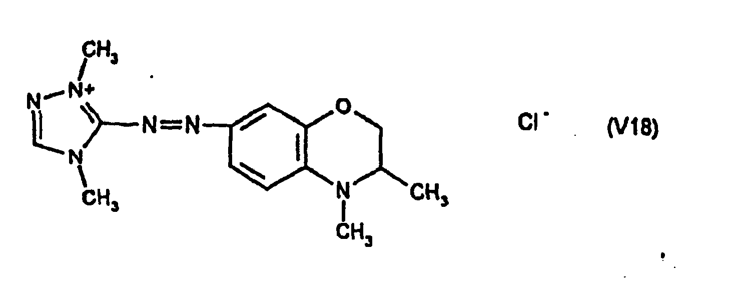

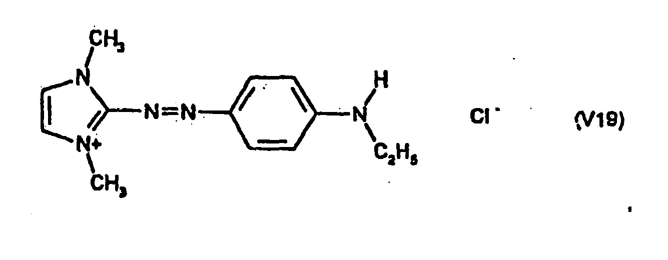

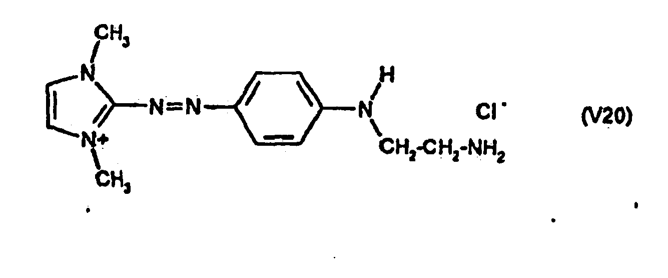

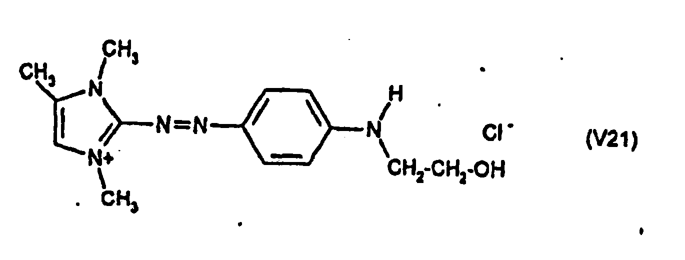

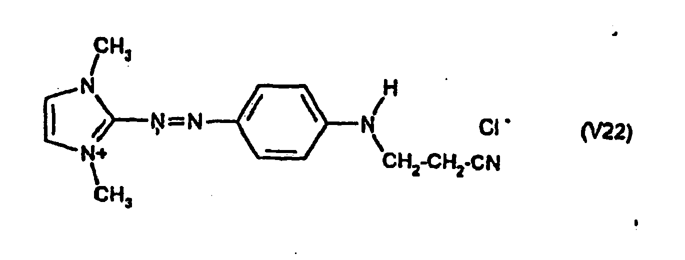

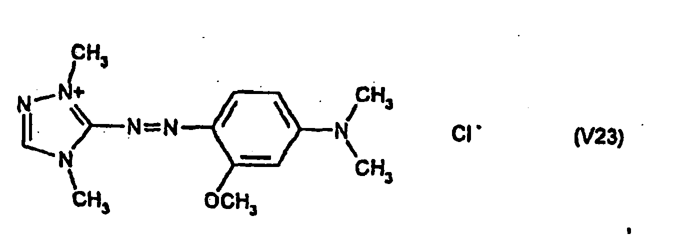

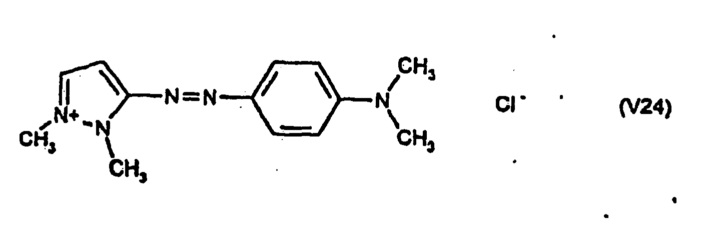

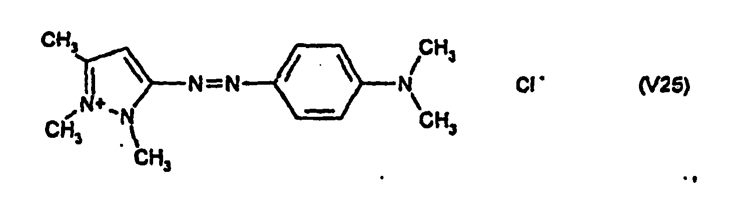

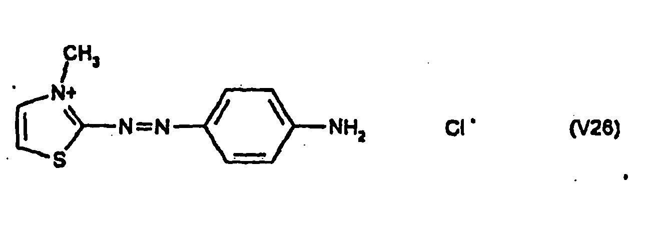

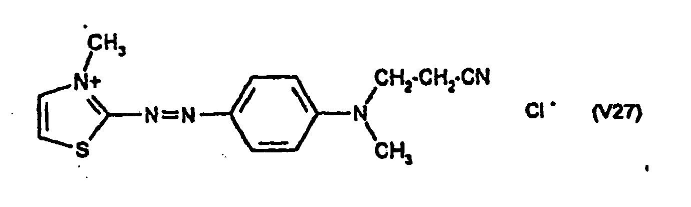

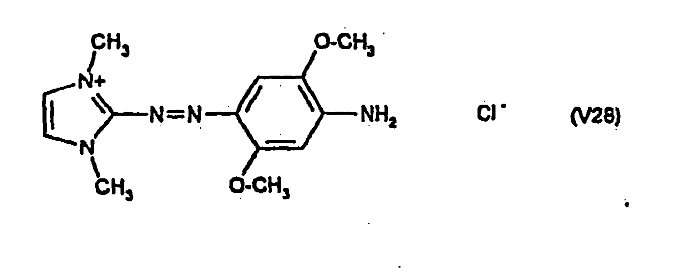

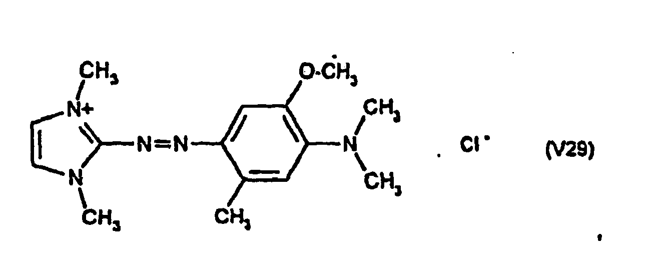

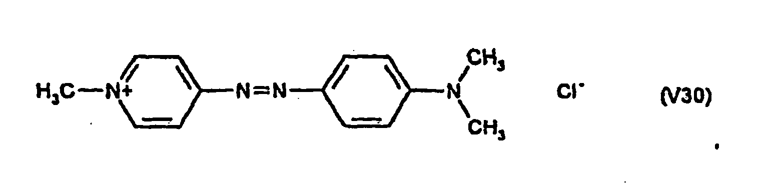

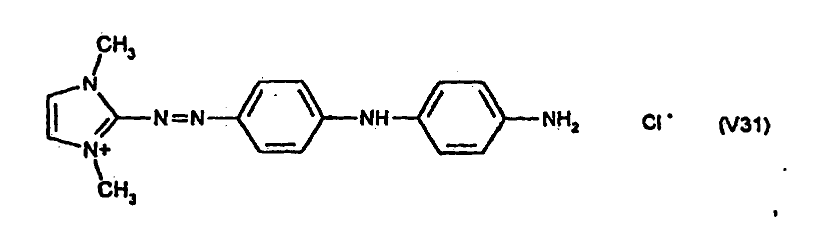

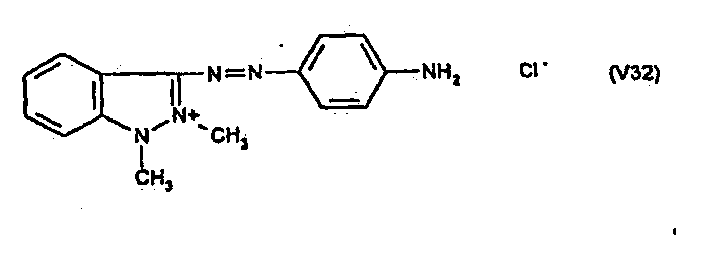

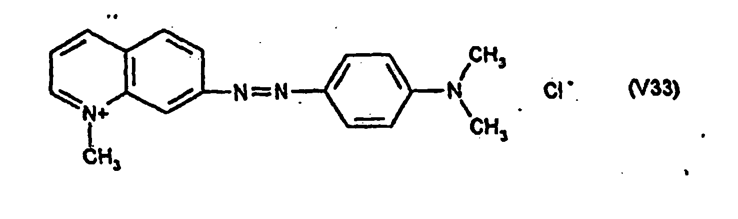

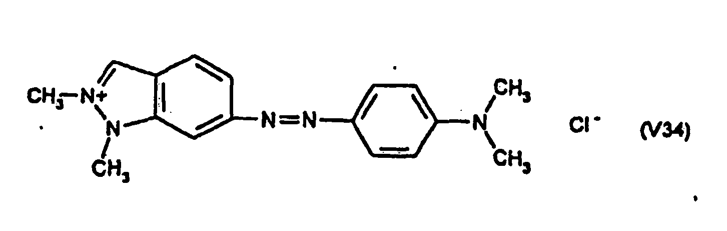

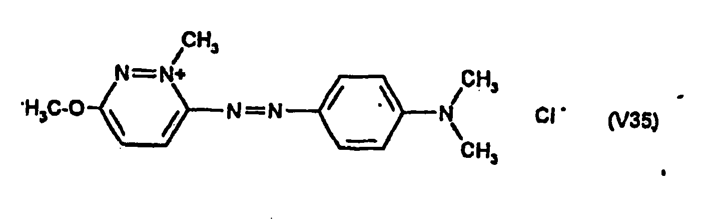

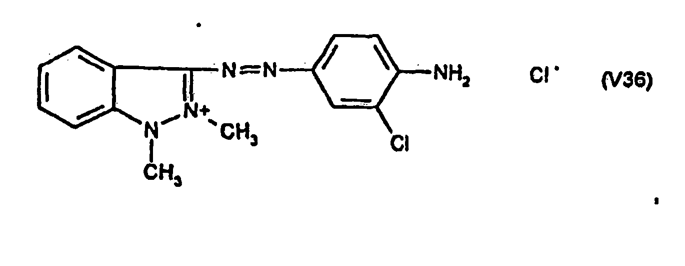

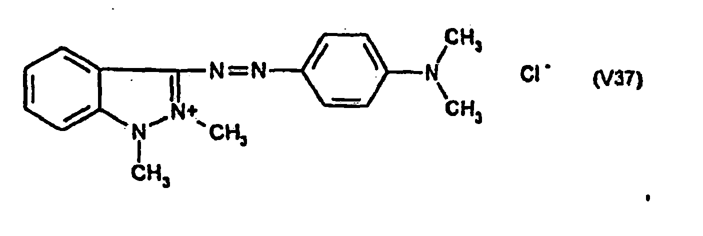

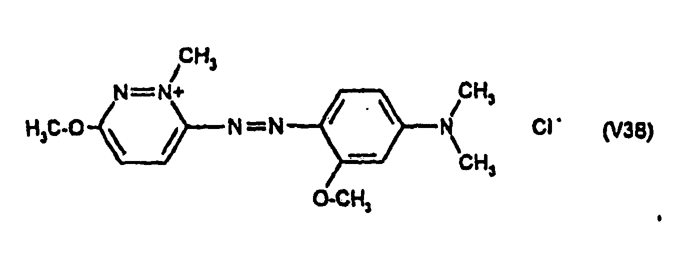

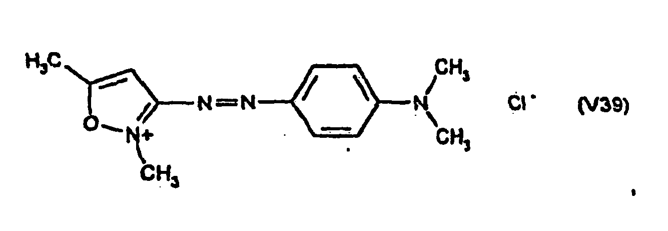

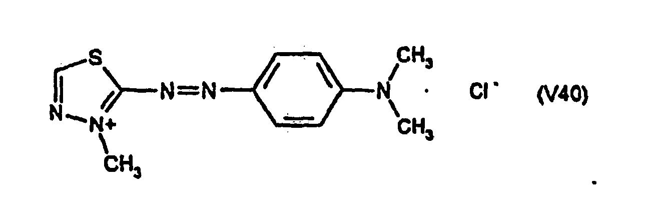

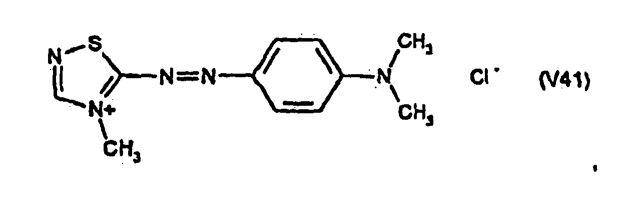

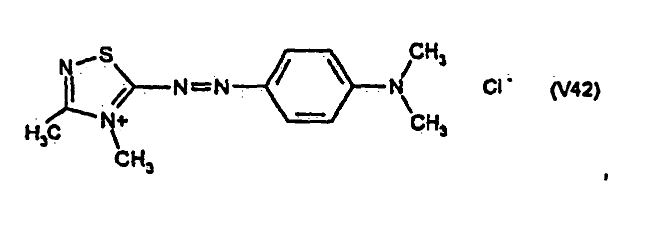

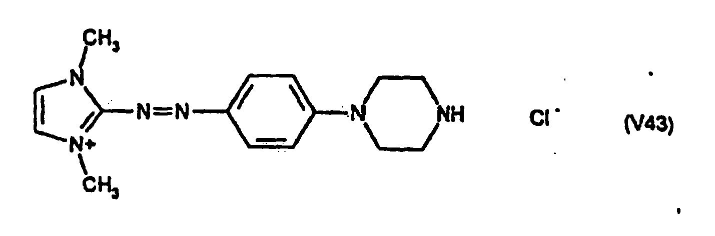

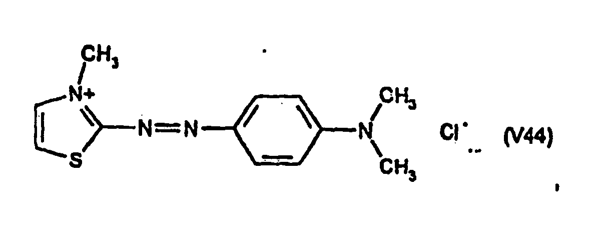

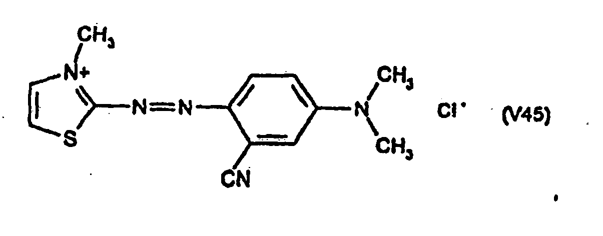

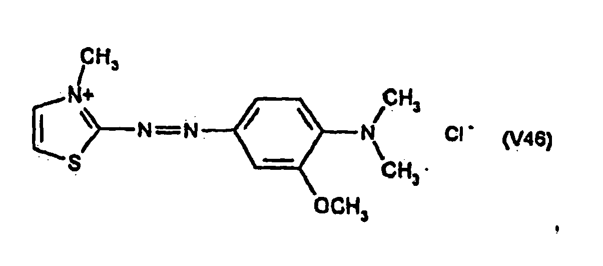

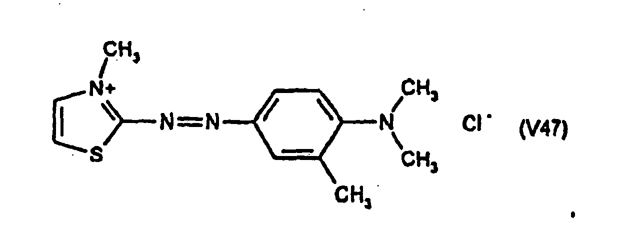

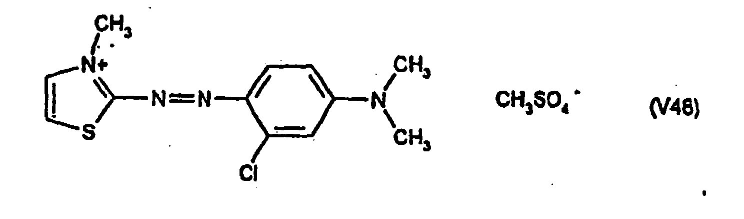

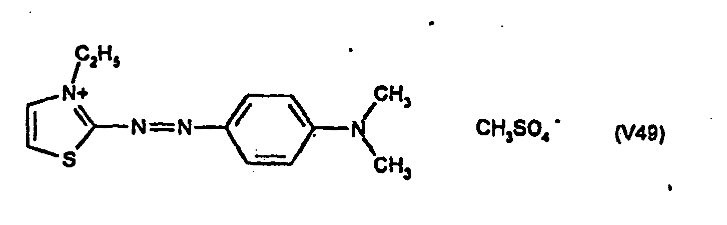

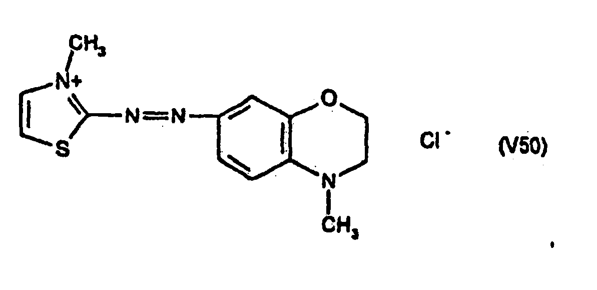

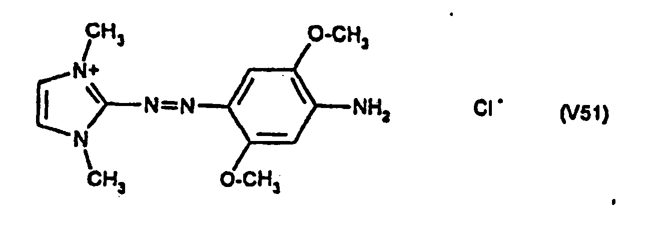

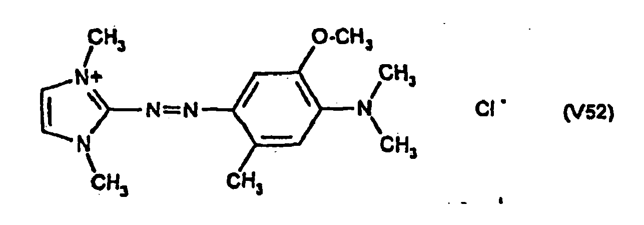

24. Zusammensetzung nach einem der vorhergehenden Ansprüche, **dadurch gekennzeichnet, dass** die kationischen Direktfarbstoffe der Formel (VI) unter den Verbindungen der folgenden Strukturen (VI1) bis (VI12) ausgewählt sind: 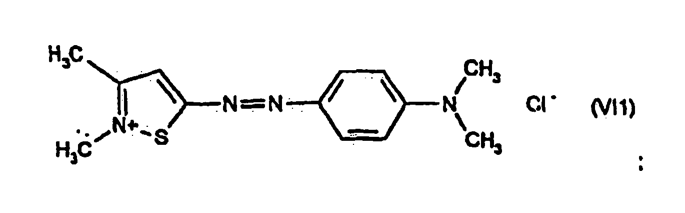 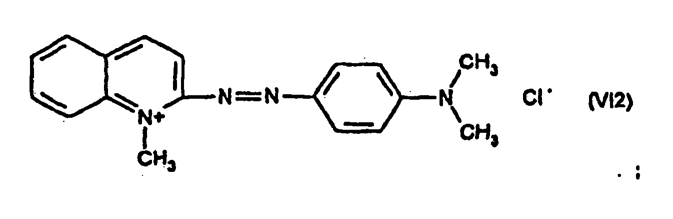 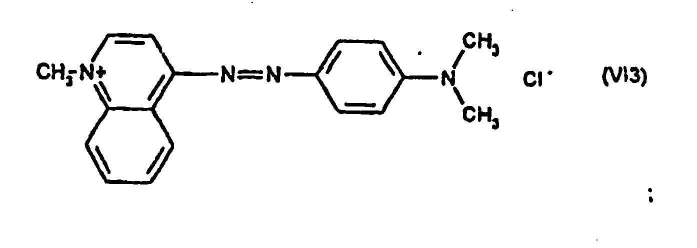 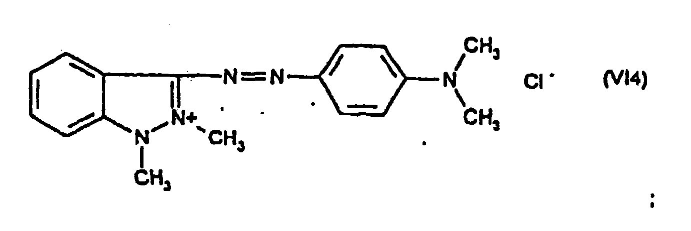 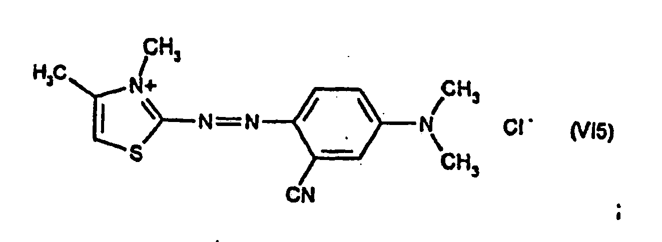 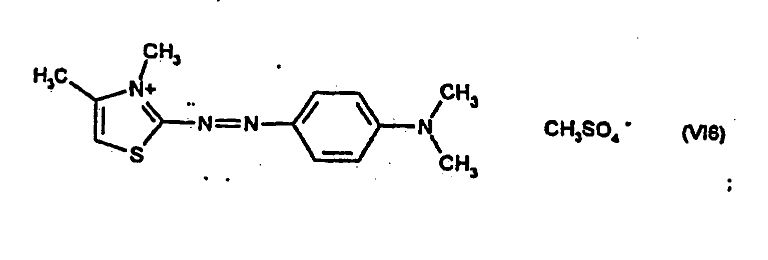 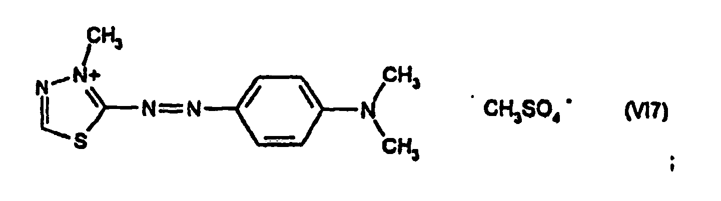 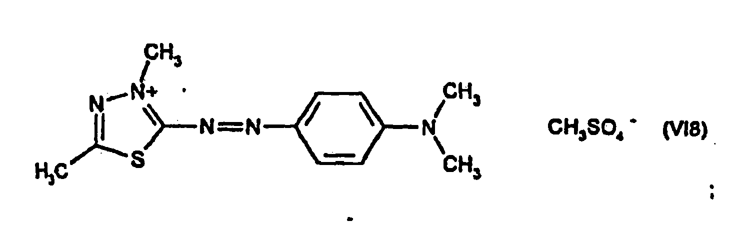 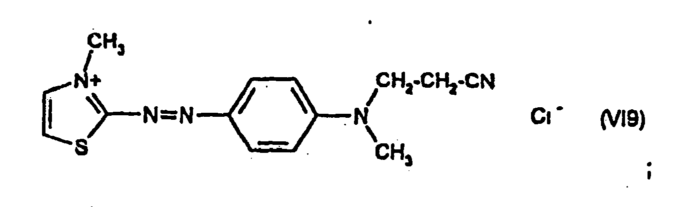 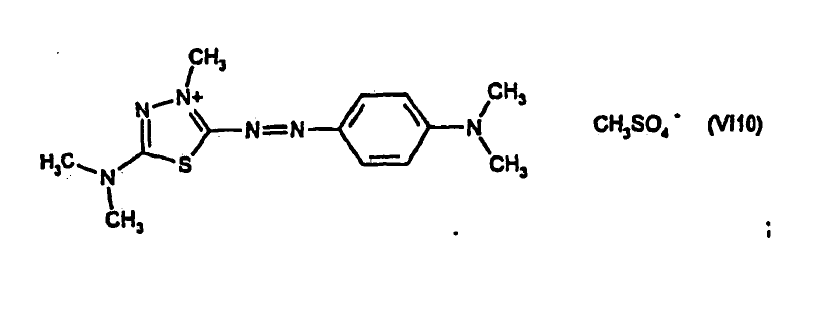 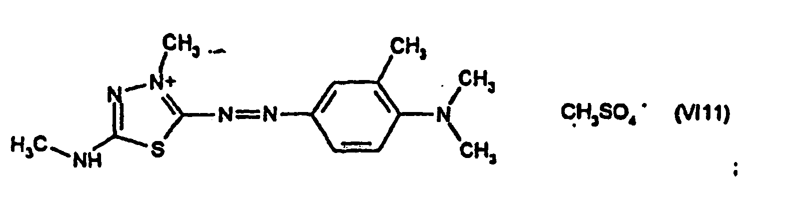 und

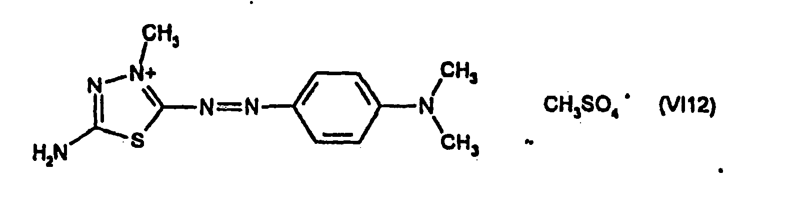

25. Zusammensetzung nach einem der vorhergehenden Ansprüche, **dadurch gekennzeichnet, dass** die kationischen Direktfarbstoffe der Formel (VII) unter den Verbindungen der folgenden Strukturen (VII1) bis (VII18) ausgewählt sind:

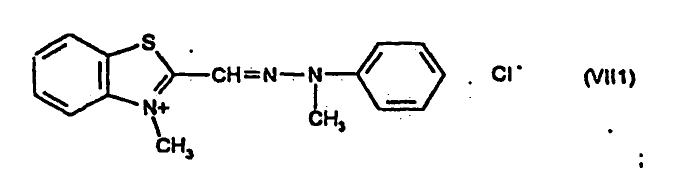

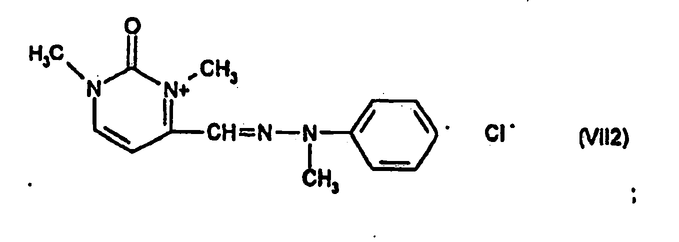

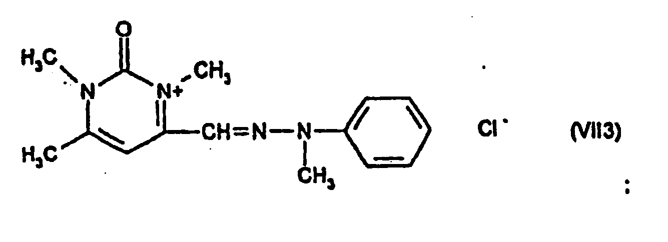

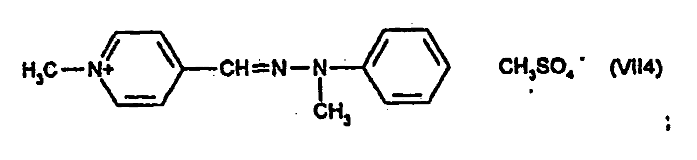

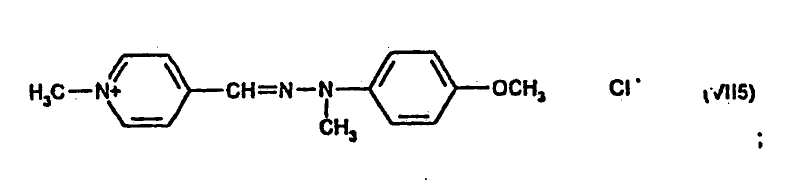

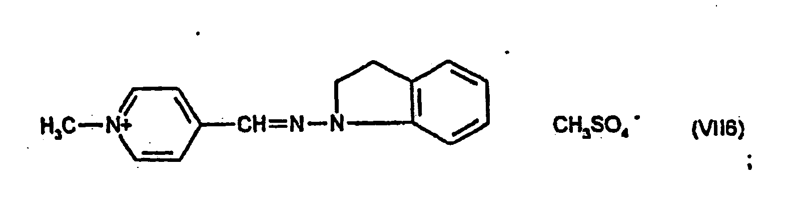

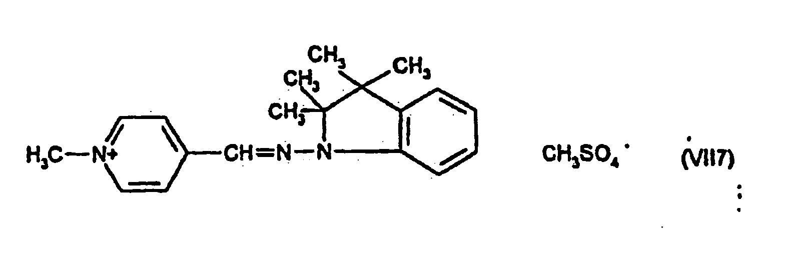

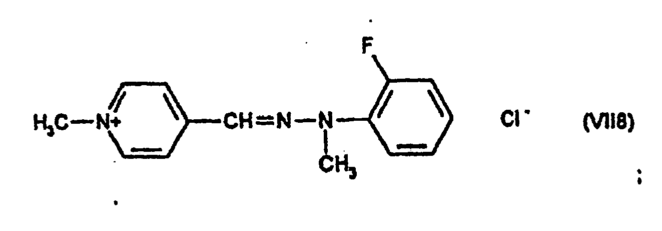

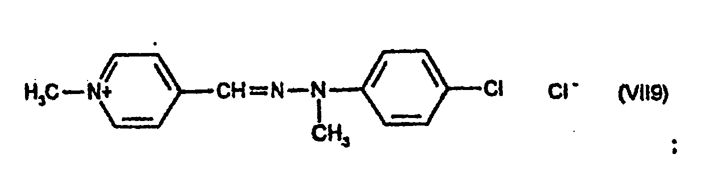

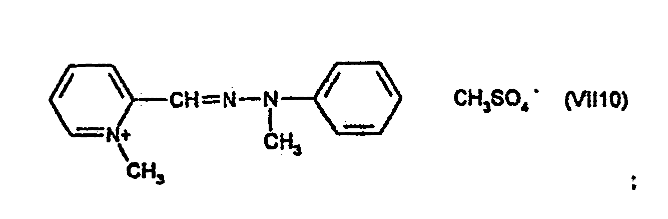

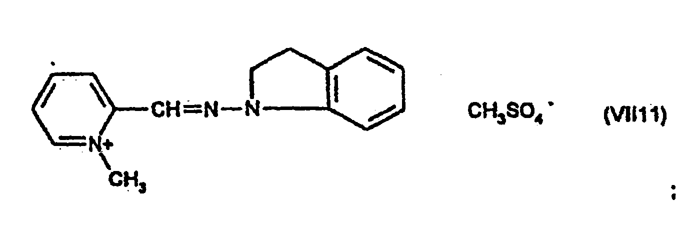

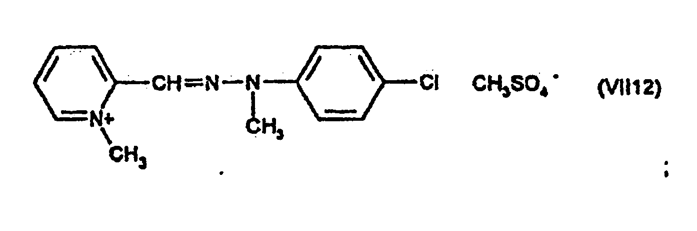

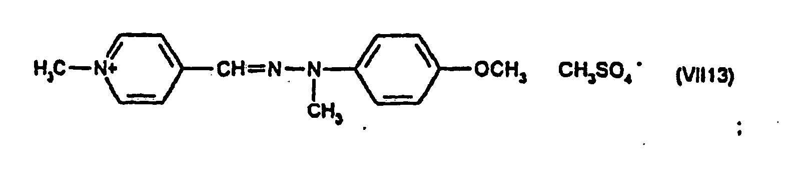

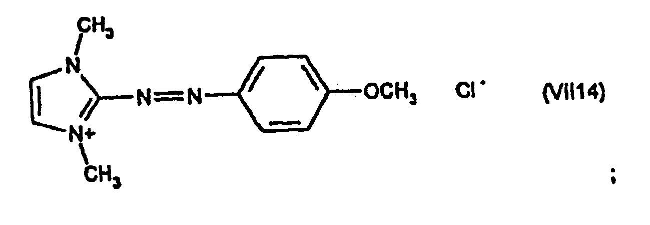

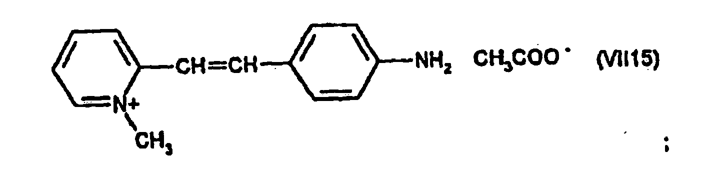

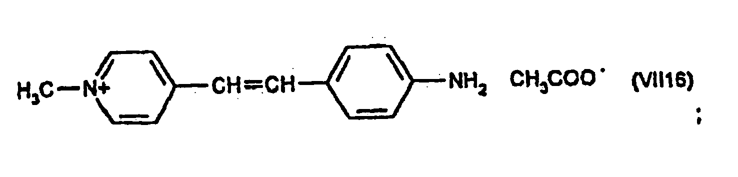

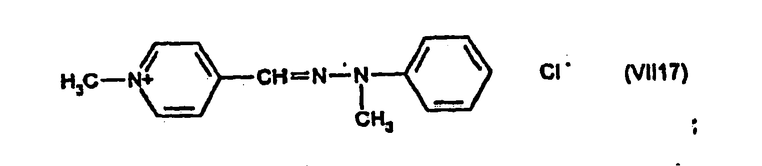

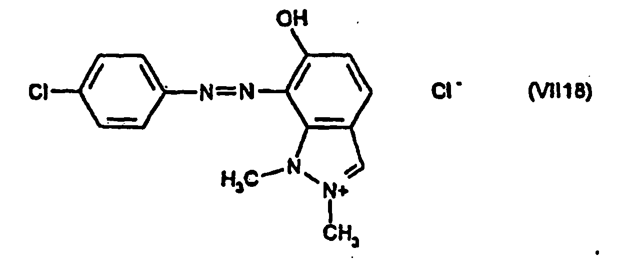

26. Zusammensetzung nach einem der vorhergehenden Ansprüche, **dadurch gekennzeichnet, dass** die kationischen Direktfarbstoffe der Formel (VII') unter den Verbindungen der folgenden Strukturen (VII'1) bis (VII'3) ausgewählt sind:

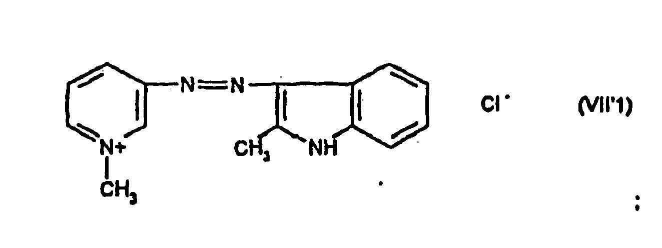

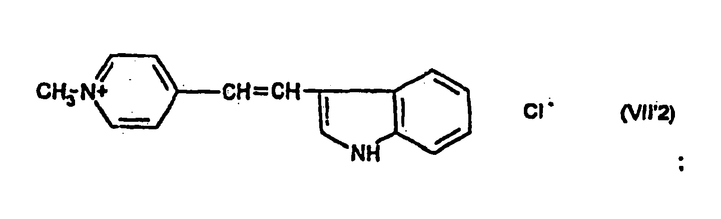

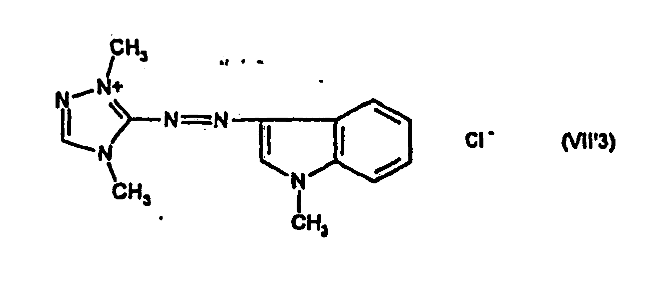

27. Zusammensetzung nach einem der vorhergehenden Ansprüche, **dadurch gekennzeichnet, dass** der oder die kationischen Direktfarbstoffe 0,001 bis 10 Gew.-% des Gesamtgewichts der gebrauchsfertigen Färbemittelzusammensetzung ausmachen.

28. Zusammensetzung nach Anspruch 27, **dadurch gekennzeichnet, dass** der oder die kationischen Direktfarbstoffe 0,05 bis 5 Gew.-% des Gesamtgewichts der gebrauchsfertigen Färbemittelzusammensetzung ausmachen.

29. Zusammensetzung nach einem der vorhergehenden Ansprüche, **dadurch gekennzeichnet, dass** die Additionssalze mit einer Säure unter den Hydrochloriden, Hydrobromiden, Sulfaten, Tartraten, Lactaten und Acetaten ausgewählt sind.

30. Zusammensetzung nach einem der vorhergehenden Ansprüche, **dadurch gekennzeichnet, dass** das zum Färben geeignete Medium aus Wasser oder einem Gemisch von Wasser und mindestens einem organischen Lösungsmittel besteht.

31. Zusammensetzung nach einem der vorhergehenden Ansprüche, **dadurch gekennzeichnet, dass** sie einen pH-Wert im Bereich von 4 bis 11 aufweist.

32. Verfahren zum Färben von Keratinfasern und insbesondere menschlichen Keratinfasern, wie dem Haar, **dadurch gekennzeichnet, dass** auf die Fasern mindestens eine gebrauchsfertige Färbemittelzusammensetzung nach einem der vorhergehenden Ansprüche während einer Zeitspanne, die zur Bildung einer Färbung ausreichend ist, aufgebracht wird.

33. Verfahren nach Anspruch 32, **dadurch gekennzeichnet, dass** es einen vorbereitenden Schritt umfasst, der darin besteht, einerseits eine Zusammensetzung (A), die in einem zum Färben geeigneten Medium mindestens einen Oxidationsfarbstoff und mindestens einen kationischen Direktfarbstoff enthält, und andererseits eine Zusammensetzung (B) getrennt voneinander aufzubewahren, die in einem zum Färben geeigneten Medium mindestens ein Enzym vom Laccase-Typ enthält, und diese bei der Anwendung zu vermischen, bevor das Gemisch auf die Keratinfasern aufgebracht wird.

34. Vorrichtung mit mehreren Abteilungen oder "Kit" zum Färben, **dadurch gekennzeichnet, dass** sie eine erste Abteilung mit der in Anspruch 33 definierten Zusammensetzung (A) und eine zweite Abteilung mit der in Anspruch 33 definierten oxidierenden Zusammensetzung (B) umfasst.
